(19) Europäisches Patentamt
European Patent Office
Office européen des brevets

(11) EP 4 269 394 A1

(12) EUROPEAN PATENT APPLICATION
published in accordance with Art. 153(4) EPC

(43) Date of publication:
**01.11.2023 Bulletin 2023/44**

(21) Application number: **22746162.1**

(22) Date of filing: **21.01.2022**

(51) International Patent Classification (IPC):
***C07D 235/30*** *(2006.01)* ***A61K 31/496*** *(2006.01)*
***A61K 31/501*** *(2006.01)* ***A61K 31/506*** *(2006.01)*
***A61K 31/4355*** *(2006.01)* ***A61P 35/00*** *(2006.01)*
***C07D 235/32*** *(2006.01)* ***C07D 403/12*** *(2006.01)*
***C07D 417/12*** *(2006.01)* ***C07D 277/82*** *(2006.01)*

(52) Cooperative Patent Classification (CPC):
**A61K 31/4355; A61K 31/496; A61K 31/501; A61K 31/506; A61P 35/00; C07D 235/30; C07D 235/32; C07D 277/82; C07D 403/12; C07D 417/12; C07D 491/107; C07D 491/113; C07D 513/04**

(86) International application number:
**PCT/KR2022/001117**

(87) International publication number:
**WO 2022/164135 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.01.2021 KR 20210012749**

(71) Applicant: **Korea Research Institute of Chemical Technology**
**Daejeon 34114 (KR)**

(72) Inventors:
- **KIM, Kwang Rok**
  **Daejeon 34114 (KR)**
- **JEONG, Kwan Young**
  **Daejeon 34114 (KR)**
- **JUNG, Hee Jung**
  **Daejeon 34114 (KR)**
- **KIM, Doyoun**
  **Daejeon 34114 (KR)**
- **LEE, Junmi**
  **Daejeon 34114 (KR)**
- **RHEE, Sang Dal**
  **Daejeon 34114 (KR)**
- **LEE, Kyu Myung**
  **Daejeon 34114 (KR)**

(74) Representative: **Gassner, Birgitta et al**
**REDL Life Science Patent Attorneys**
**Donau-City-Straße 11**
**1220 Wien (AT)**

(54) **BENZOTHIAZOLE AND BENZIMIDAZOLE DERIVATIVES, PHARMACEUTICALLY ACCEPTABLE SALT THEREOF, PREPARATION METHOD THEREFOR, AND PHARMACEUTICAL COMPOSITION COMPRISING SAME AS ACTIVE INGREDIENT**

(57) The present invention relates to a benzothiazole or benzimidazole derivative, a pharmaceutically acceptable salt thereof, and a pharmaceutical composition comprising same as an active ingredient for prevention or treatment of SIRTUIN 7 protein-related diseases. With excellent inhibitory activity against SIRTUIN 7 protein the derivative can be used for preventing or treating SIRTUIN 7 protein-related diseases.

EP 4 269 394 A1

Fig. 1a

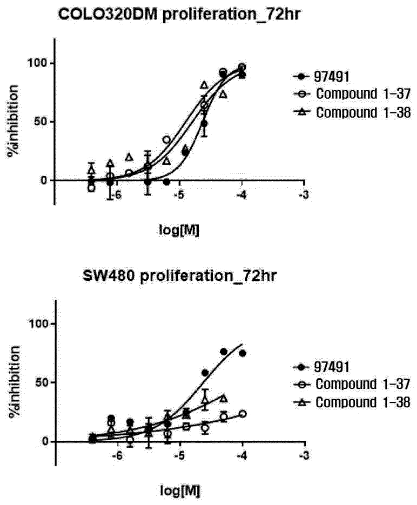


Fig. 1b

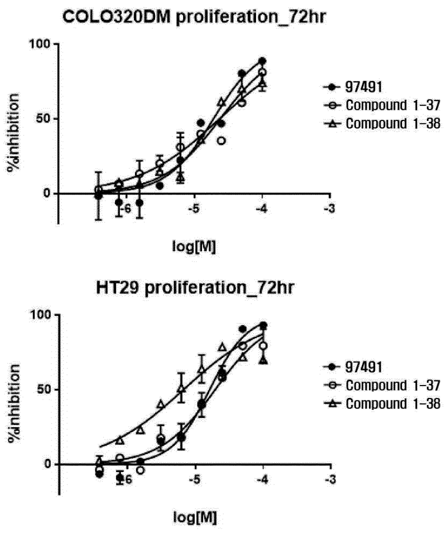


Fig. 1c

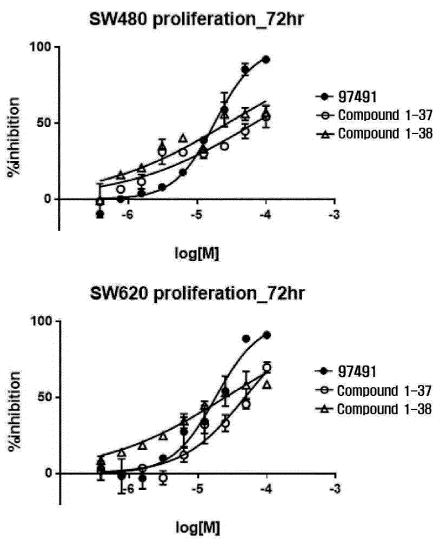

## Description

BACKGROUND

Technical Field

The present invention relates to benzothiazole and benzimidazole compounds, or pharmaceutically acceptable salts, and a pharmaceutical composition comprising the same as an active ingredient for prevention or treatment of SIRTUIN 7 (hereinafter referred to as 'SIRT7') protein-related diseases.

Background Art

A total of seven SIRTUIN proteins (hereinafter abbreviated as 'SIRT'), from SIRT1 to SIRT7, have been identified and reported to be involved in aging, metabolic diseases, and gene stability. SIRT7 was found to be present in the nucleolus and nucleoplasm (Kiran et al., FEBS J, 2013) and was reported to play an important role in cellular stress.

For example, it is observed when SIRT7 is overexpressed, HIF-1$\alpha$ expression decreases regardless of catalytic activity, and HIF-1$\alpha$ expression increases during siRNA treatment (Hubbi et al., J Biol Chem, 2013), therefore SIRT7 has been reported to be involved in hypoxia.

In addition, SIRT7 is related to hypoglycemic stress, for example, SIRT7 moves from the nucleolus to the nucleoplasm under hypoglycemic conditions or when treated with AICAR, an AMPK activator, which appears to inhibit the rDNA transcription process by reducing binding to PAF53 in the nucleolus, thereby enabling the system to respond to an energy depletion state. (Chen et al., MolCell, 2013).

In addition, it has been reported when the endoplasmic reticulum stress occurs, SIRT7 mRNA increases, which suppresses Myc activity in front of the promoter of the ribosomal protein gene (RPS20), which is an endoplasmic reticulum stress response protein, thereby reducing the transcription process of these genes, thereby reducing the endoplasmic reticulum stress. (Shin et al., Cell Rep, 2013).

As another example, SIRT7 is involved in aging, for example, reduced lifespan (Vakhrusheva et al., Circ Res, 2008) and age-related hearing loss (Ryu et al., Cell Metabolism, 2014) have been reported in SIRT7 knock-out mice, and the decreased expression of SIRT7 was observed in an aging mouse model (Lee et al., Proteomics, 2014) and hepatocytes of an aging mouse model (Ghiraldini et al., Mol BiolCell, 2013). Deacetylation of NPM1 by SIRT7 is expected to contribute to suppressing aging (Lee et al., Proteomics, 2014).

Also, SIRT7 is involved in DNA damage response. For example, SIRT7 knock-out MEFs are more sensitive to apoptosis by adriamycin or hydrogen peroxide as ac-p53 is higher than wild-type (Vakhrusheva etal., J Physiol Pharmacol, 2008; Vakhrusheva et al., Cir Res, 2008), and it was observed when SIRT7 was overexpressed, homologous recombination efficiency was increased after paraquat treatment. (Mao et al., Science, 2011). It was found when SIRT7 was overexpressed, apoptosis or cell senescence induced by doxorubicin was reduced by blocking p38 and JNK activity (Kiran et al., Exp Cell Res, 2014). Also, SIRT7 has been reported to be associated with cancer. For example, it has been reported that SIRT7 suppresses the expression of tumor suppressor genes by mediating the deacetylation of H3K18 (Barber et al., Nature, 2012), and increases the growth of liver cancer cells when the expression of SIRT7 regulated by mir-125a-5p and mir-125b is increased (Kim et al., Hepatology, 2013). In addition, it was observed that the expression level of SIRT7 was increased in thyroid cancer and breast cancer. (de Nigris et al., Br J Cancer, 2002; Frye, Br J Cancer, 2002; Ashraf et al., Br J Cancer, 2006).

Accordingly, it is expected that compounds with inhibitory activity against SIRT7 or pharmaceutically acceptable salts thereof can be used for the treatment or prevention of SIRT7-related diseases.

DETAILED DESCRIPTION OF THE INVENTION

Technical Problem

An object of the present invention is to provide a benzothiazole derivative or a benzimidazole derivative exhibiting inhibitory activity against SIRTUIN 7 protein (hereinafter referred to as 'SIRT7'), or a pharmaceutically acceptable salt thereof.

Another object of the present invention is to provide a pharmaceutical composition for prevention or treatment SIRT7-related diseases comprising a benzothiazole derivative or benzimidazole derivative, or a pharmaceutically acceptable salt thereof, exhibiting SIRT7 inhibition activity.

Another object of the present invention is to provide a pharmaceutical composition comprising at least one from the group consisting of a benzothiazole derivative or a benzimidazole derivative, or a pharmaceutically acceptable salt thereof, a carrier, an excipient, and a diluent thereof according to the present invention.

Technical Solution

The present invention provides a compound represented by Formula 1 or a pharmaceutically acceptable salt thereof.

Formula 1

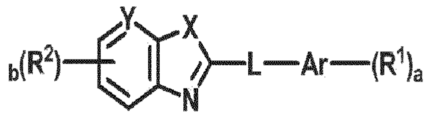

In another aspect, the present invention provides a pharmaceutical composition for preventing or treating SIRTUIN 7 protein-related diseases, comprising the compound represented by Formula 1 or a pharmaceutically acceptable salt thereof as an active ingredient.

Effects of the invention

The compound according to the present invention has an excellent effect of inhibiting SIRTUIN 7 activity, and has an effect of preventing or treating SIRTUIN 7 protein-related diseases.

Brief Description of Drawings

Fig. 1a shows the results of analyzing the inhibition effect of the cancer cell proliferation of COLO320DM and SW480 cells according to an embodiment of the present invention.

Fig. 1b shows the results of analyzing the inhibition effect of the cancer cell proliferation of KRAS wild-type COLO320DM and HT29 cells according to an embodiment of the present invention.

Fig. 1c shows the results of analyzing the inhibition effect of the cancer cell proliferation of KRAS mutant, SW480 and SW620 cells according to an embodiment of the present invention.

Fig. 2 shows the results of LDH cytotoxicity test of the compound according to an embodiment of the present invention. Fig. 2 (a) shows the LDH assay of the cancer cell line, Fig. 2 (b) shows the LDH assay of the normal lung fibroblast cell line.

Fig. 3 is a photograph of a mouse used for an in vivo test of a compound according to an embodiment of the present invention.

Fig. 4 shows the in vivo test results (tumor size and body weight) of compound 1-37 according to an embodiment of the present invention.

Fig. 5a shows the results of the first in vivo test of tumor size by concentration after injecting compound 1-37 into COLO320DM according to an embodiment of the present invention.

Fig. 5b is a mouse photograph of the results of the first in vivo test of tumor size by concentration after injecting compound 1-37 into COLO320DM according to an embodiment of the present invention.

Fig. 6a shows the results of the first in vivo test of tumor size by concentration after injecting compound 1-37 into SW480 according to an embodiment of the present invention.

Fig. 6b is a mouse photograph of the results of the first in vivo test of tumor size by concentration after injecting compound 1-37 into SW480 according to an embodiment of the present invention.

Fig. 7 shows the results of the first in vivo test of body weight by concentration after injecting compound 1-37 into

COLO320DM and SW480 according to an embodiment of the present invention.

Fig. 8a shows the results of the second in vivo test of tumor size by concentration after injecting compound 1-37 into COLO320DM according to an embodiment of the present invention.

Fig. 8b is a mouse photograph of the results of the second in vivo test of tumor size by concentration after injecting compound 1-37 into COLO320DM according to an embodiment of the present invention.

Fig. 9a shows the results of the second in vivo test of tumor size by concentration after injecting compound 1-37 into SW480 according to an embodiment of the present invention.

Fig. 9b is a mouse photograph of the results of the second in vivo test of tumor size by concentration after injecting compound 1-37 into SW480 according to an embodiment of the present invention.

Fig. 10 shows the results of the second in vivo test of body weight by concentration after injecting compound 1-37 into COLO320DM and SW480 according to an embodiment of the present invention.

Fig. 11a shows the results of analyzing the inhibition effect of the cancer cell proliferation after injecting compound 1-105 and 1-106 into COLO320DM according to an embodiment of the present invention.

Fig. 11b shows in vivo test results of the tumor size and weight after injecting compound 1-105 and 1-106 into COLO320DM according to an embodiment of the present invention.

Fig. 11c is a mouse photograph of in vivo test results of the tumor size after injecting compound 1-105 and 1-106 into COLO320DM according to an embodiment of the present invention.

Detailed Description

Hereinafter, preferred embodiments of the present invention will be described in detail. In describing the present invention, if it is determined that a detailed description of a related known configuration or function may obscure the gist of the present invention, the detailed description will be omitted.

As used in this specification and the appended claims, unless otherwise stated, the following terms have the following meanings:

As used herein, the term "halogen" is fluorine (F), bromine (Br), chlorine (Cl) or iodine (I) unless otherwise specified.

As used herein, the term "alkyl" or "alkyl group" refers to an aliphatic hydrocarbon radical, and refers to a radical of a saturated aliphatic functional group, comprising linear alkyl groups, branched chain alkyl groups, and cycloalkyl (cycloaliphatic) groups, alkyl-substituted cycloalkyl group, cycloalkyl-substituted alkyl group. For example, $C_1$~$C_6$ alkyl is an aliphatic hydrocarbon having 1 to 6 carbon atoms, and includes methyl, ethyl, propyl, n-butyl, n-pentyl, n-hexyl, isopropyl, isobutyl, sec-butyl, tert-butyl, neopentyl, isopentyl, etc.

As used herein, the term "alkenyl group" or "alkynyl group" refers to a group in which at least two carbon atoms have at least one carbon-carbon double bond or at least two carbon atoms have at least one carbon-carbon triple bond, and includes, but is not limited to, linear or branched chain groups.

As used herein, the term "alkoxyl group" or "alkoxy group" refers to a radical in which the hydrogen atom of a hydroxy group is substituted with an alkyl, unless otherwise defined. For example, $C_1$~$C_6$ alkoxy comprises all methoxy, ethoxy, propoxy, n-butoxy, n-pentyloxy, isopropoxy, sec-butoxy, tert-butoxy, neopentyloxy, isopentyloxy, and the like.

As used herein, the term "heterocycle" or "heterocyclic group" includes one or more heteroatoms, unless otherwise specified, and includes at least one of a single ring and multiple rings, and includes a heteroaliphatic ring and a heteroaromatic ring. It may also be formed by combining adjacent functional groups.

As used herein, the term "aryl group" or "arylene group" refers to a single-ring or multi-ring aromatic group, and includes an aromatic ring formed by bonding or participating in a reaction with adjacent substituents. For example, the aryl group may be a phenyl group, a biphenyl group, a fluorene group, or a spirofluorene group.

As used herein, the term "aliphatic ring" means an aliphatic hydrocarbon ring.

As used herein, the term "aromatic ring" refers to an aromatic system composed of hydrocarbons containing at least one ring, and examples thereof include benzene and naphthalene.

Also, the definitions described herein may be added to form chemically related combinations, such as "arylalkyl", "alkylcarbonyl", "arylcarbonyl", and the like. When the term "alkyl" is used as a suffix, as in another term, "phenylalkyl" or "hydroxyalkyl", it refers to an alkyl group substituted with a substituent selected from another specifically named group. Thus, for example, "phenylalkyl" means an alkyl group having a phenyl substituent and thus includes benzyl, phenylethyl

and biphenyl. "Alkylaminoalkyl" means an alkyl group with an alkylamino substituent.

Hereinafter, a compound according to an aspect of the present invention and a pharmaceutical composition containing the same will be described.

The present invention provides a compound represented by Formula 1, a stereoisomer thereof, tautomer, derivative, hydrate, solvate or pharmaceutically acceptable salt thereof;

Formula 1

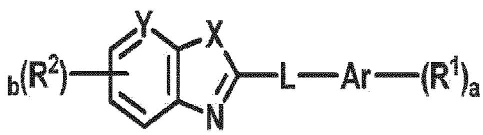

Formula 2-1

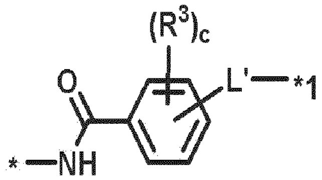

Formula 2-2

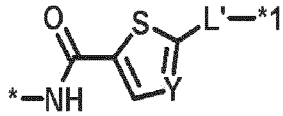

wherein:

1) X is S or N($R_1$),

2) wherein $R_1$ is hydrogen; $C_1$~$C_5$ alkyl group; or a $C_7$~ $C_{20}$ arylalkyl group substituted with fluorine:

3) Y is independently of each other N or CH,

4) $R^2$ are each the same or different, and each independently hydrogen; or halogen;

5) L is a single bond; $C_1$~$C_6$ alkylene group; $C_2$~$C_6$ alkenylene group; a substituent represented by Formula 2-1; or a substituent represented by Formula 2-2;

6) L' is a single bond; $C_1$~$C_{10}$ alkylene group; $C_2$~$C_{10}$ alkenylene group; and - ($C_nH_{2n}$)-O-($C_nH_{2n}$)- ;

7) * represents a position bonded to L, ∗1 represents a position bonded to Ar,

8) $R^3$ are each the same or different, and is each independently selected from the group consisting of hydrogen; halogen; and a $C_1$~$C_{10}$ alkyl group unsubstituted or substituted with fluorine,

9) Ar is a $C_6$~$C_{20}$ arylene group; or a $C_2$~$C_{20}$ heterocyclic group containing at least one heteroatom of N and 0;

10) $R^1$ is selected from the group consisting of hydrogen; halogen; a $C_1$~$C_{10}$ alkyl group unsubstituted or substituted with fluorine; -$SO_2$-R $^a$ ; -CO-NH-$R^b$; and -CO-$R^c$;

11) wherein $R^a$ is selected from the group consisting of a $C_1$~$C_{10}$ alkyl group; $C_3$~$C_{20}$ aliphatic ring group; $C_6$~$C_{20}$ aryl group unsubstituted or substituted with $CF_3$; and -N(R') (R") ;

12) wherein R' and R" are each independently a $C_1$~$C_{10}$ alkyl group;

13) wherein $R^b$ is selected from the group consisting of a $C_6$~$C_{20}$ aryl group; $C_3$~$C_{20}$ aliphatic ring group; fused ring group of $C_3$~$C_{20}$ aliphatic ring and $C_6$~$C_{20}$ aromatic ring; a $C_1$~$C_{20}$ alkyl group unsubstituted or substituted with halogen: and -($C_mH_{2m}$)-CO-O-($C_mH_{2m+1}$);

14) wherein $R^c$ is selected from the group consisting of a $C_1$~$C_{10}$ alkyl group; $C_6$~$C_{20}$ aryl group; and $C_1$~$C_{10}$ alkoxy group;

15) a is 0 or 1, b is an integer from 0 to 3, c is an integer from 0 to 4, n is independently an integer from 0 to 2, m is independently an integer from 1 to 6,

16) wherein, the alkyl group, the alkenyl group, the alkoxy group, the aryl group, the arylalkyl group, the alkylene group, the alkenylene group, the arylene group, the aliphatic ring group, the heterocyclic group, and the fused ring group may be further substituted with one or more substituents selected from the group consisting of halogen; $C_1$~$C_{10}$ alkyl group; $C_2$~$C_{10}$ alkenyl group; $C_2$~$C_{10}$ alkynyl group; $C_1$~$C_{10}$ alkoxy group; $C_1$~$C_{10}$ carbonyl group; and a $C_1$~$C_{10}$ alkyl group substituted with fluorine;

Also, the present invention provides a compound in which the compound represented by Formula 1 is represented by Formula 3-1 or Formula 3-2, a stereoisomer thereof, a tautomer, a derivative, a hydrate, a solvate, or a pharmaceutically acceptable salt thereof;

Formula 3-1

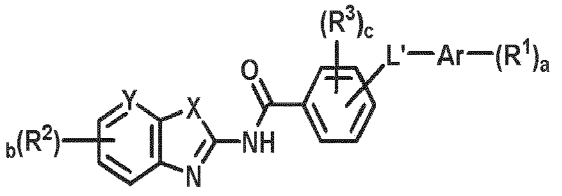

Formula 3-2

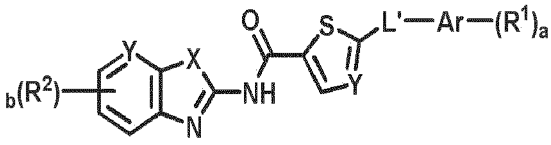

wherein, X, Y, $R^1$, $R^2$, $R^3$, L', Ar, a, b and c are the same as defined above.

Also, the present invention provides a compound in which the compound represented by Formula 1 is represented by Formula 4, a stereoisomer, a tautomer, a derivative, a hydrate, a solvate, or a pharmaceutically acceptable salt thereof;

Formula 4

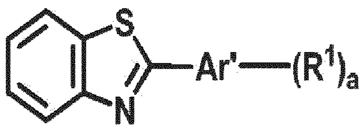

wherein,

1) $R^1$ and a are the same as defined above,

2) Ar' is a $C_2$-$C_{20}$ heterocyclic group comprising at least one N.

Also, the present invention provides a compound in which Ar is represented by any one of Formula Ar-1 to Formula Ar-11, a stereoisomer, a tautomer, a derivative, a hydrate, a solvate, or pharmaceutically acceptable salt thereof;

Formula Ar-1

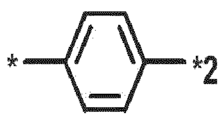

Formula Ar-2

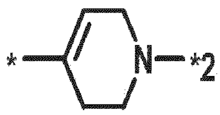

Formula Ar-3

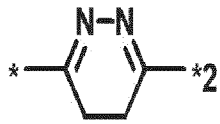

Formula Ar-4

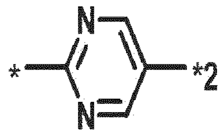

Formula Ar-5

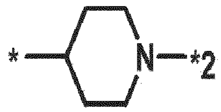

Formula Ar-6

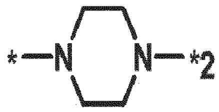

Formula Ar-7

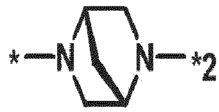

Formula Ar-8

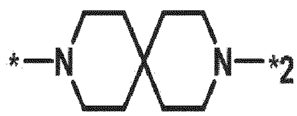

Formula Ar-9

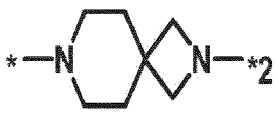

Formula Ar-10

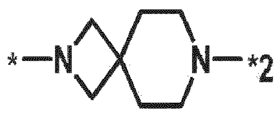

Formula Ar-11

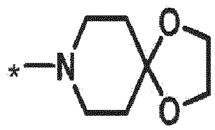

wherein,

1) * represents a position bonded to L,

2) *2 represents the position bonded to $R^1$.

Also, the present invention provides a compound in which the compound represented by Formula 1 is represented by any one of the following compounds, stereoisomers, tautomers, derivatives, hydrates, solvates, or pharmaceutically acceptable salts thereof;

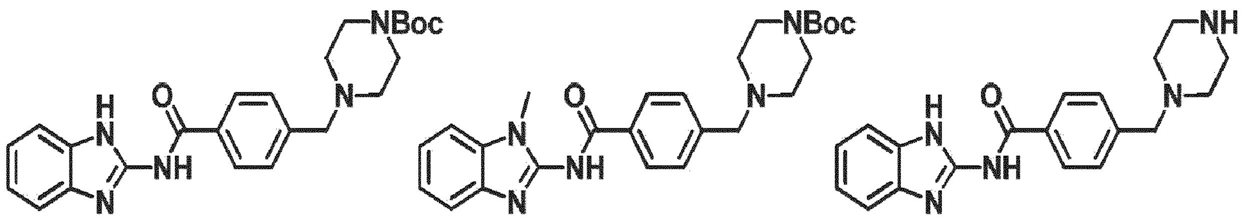

1-1 1-2 1-3

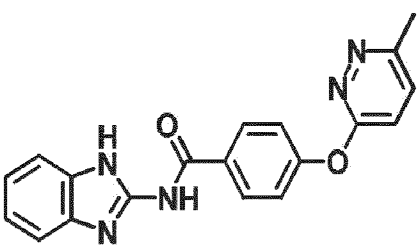

1-4

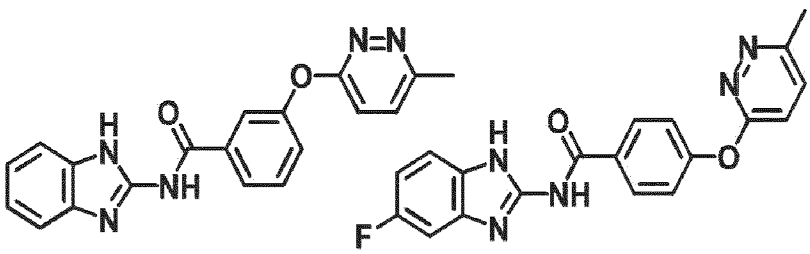

1-5 1-6

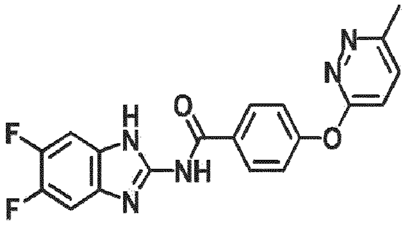

1-7

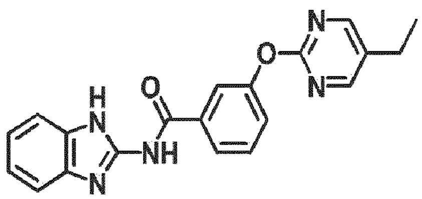

1-8

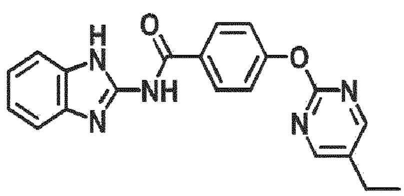

1-9

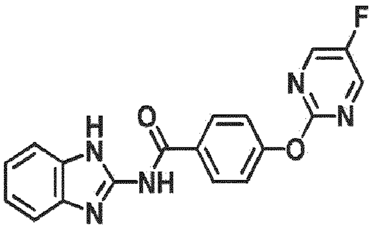

1-10

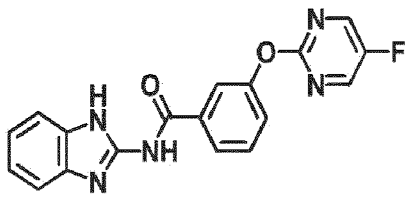

1-11

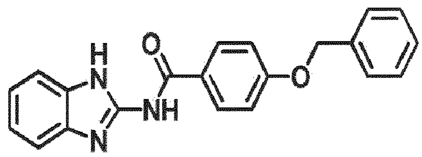

1-12

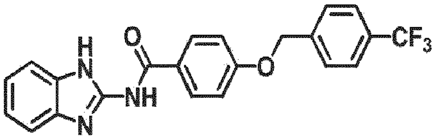

1-13

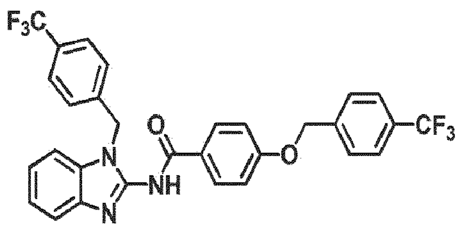

1-14

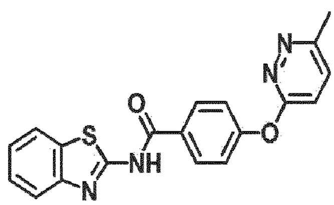

1-15

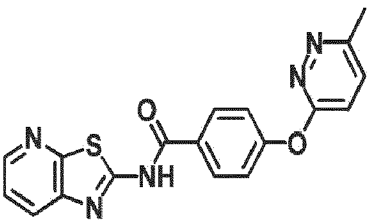

1-16

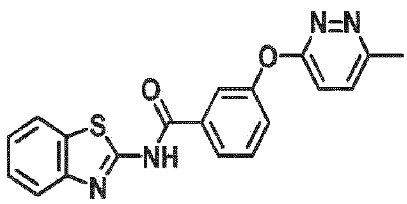

1-17

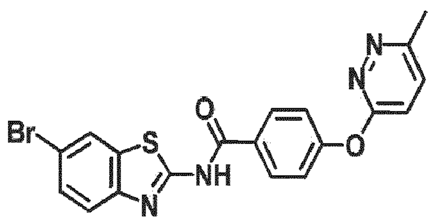

1-18

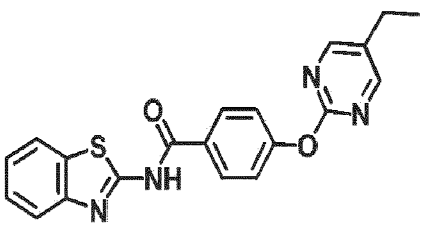

1-19

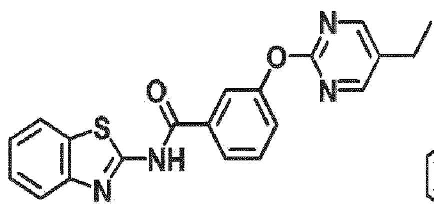

1-20

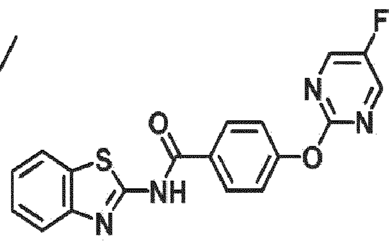

1-21

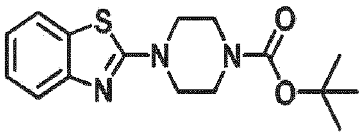

1-22

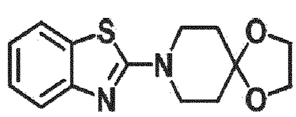

1-23

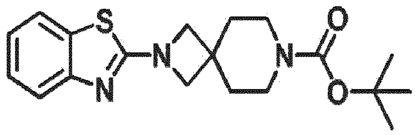

1-24

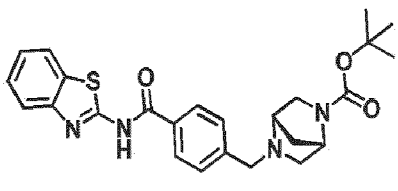

1-25

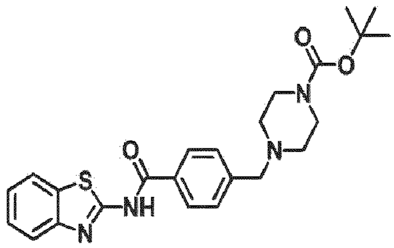

1-26

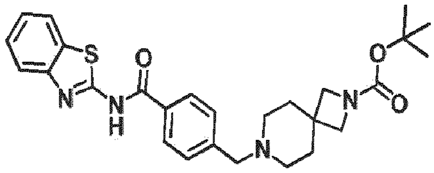

1-27

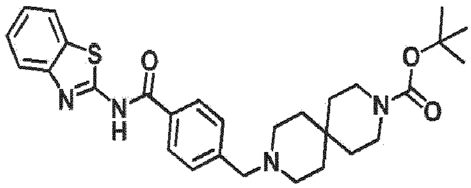

1-28

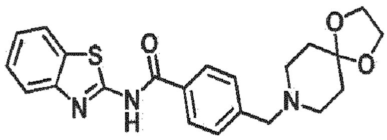

1-29

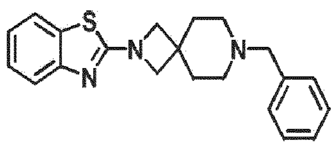

1-30

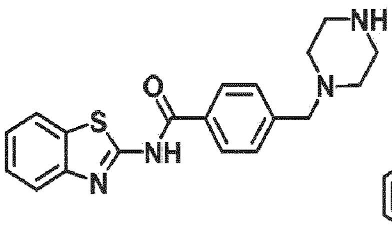

1-31

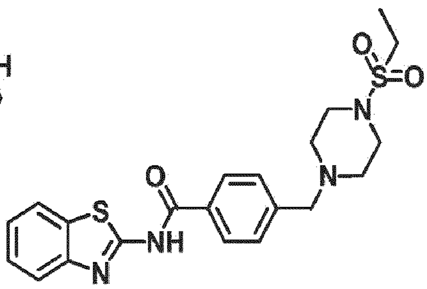

1-32

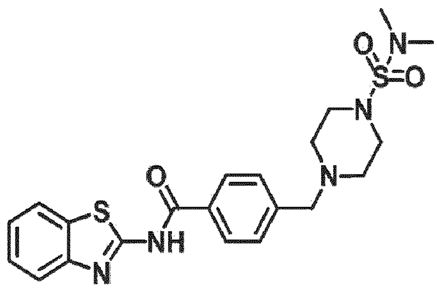

1-33

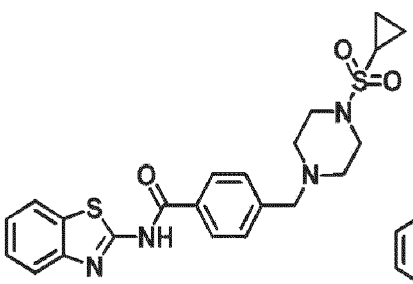

1-34

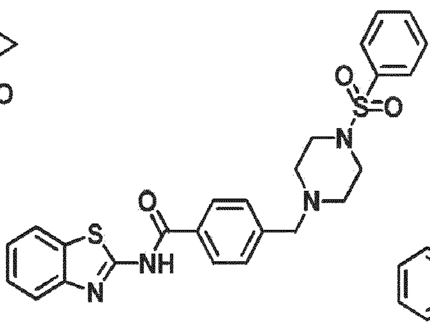

1-35

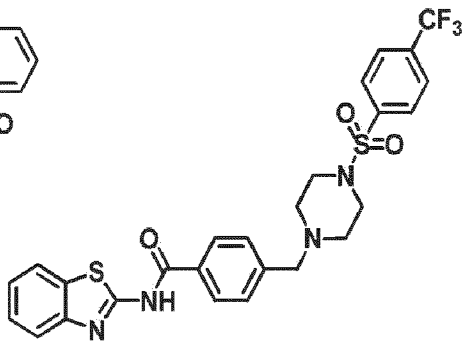

1-36

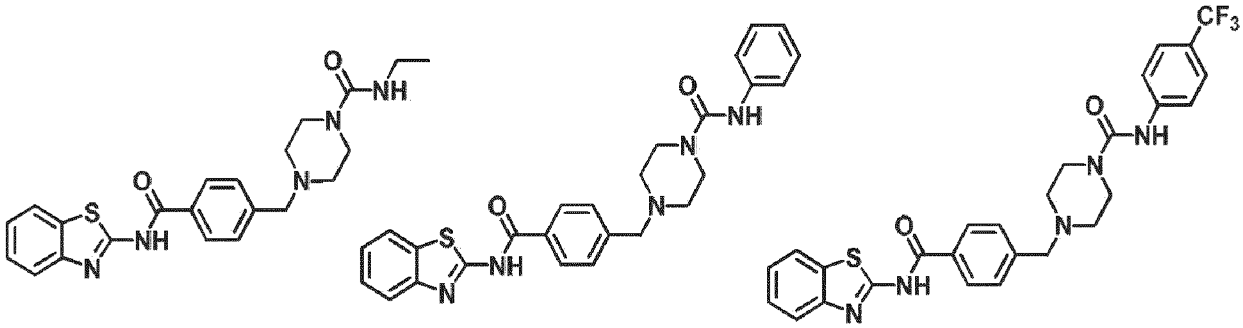

1-37 1-38 1-39

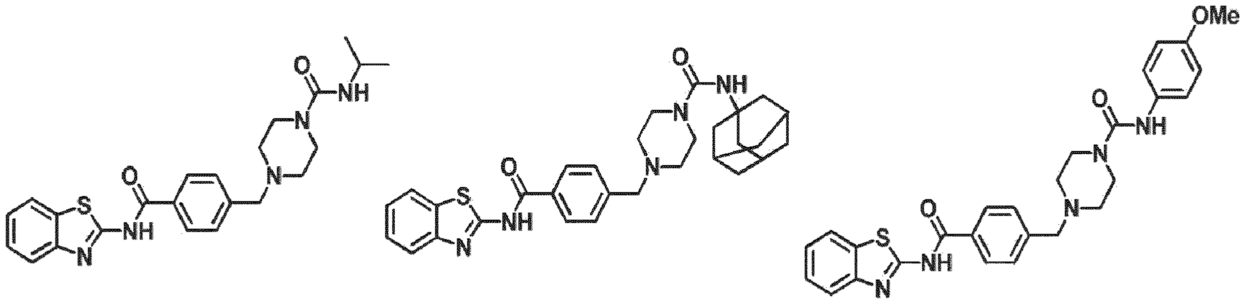

1-40 1-41 1-42

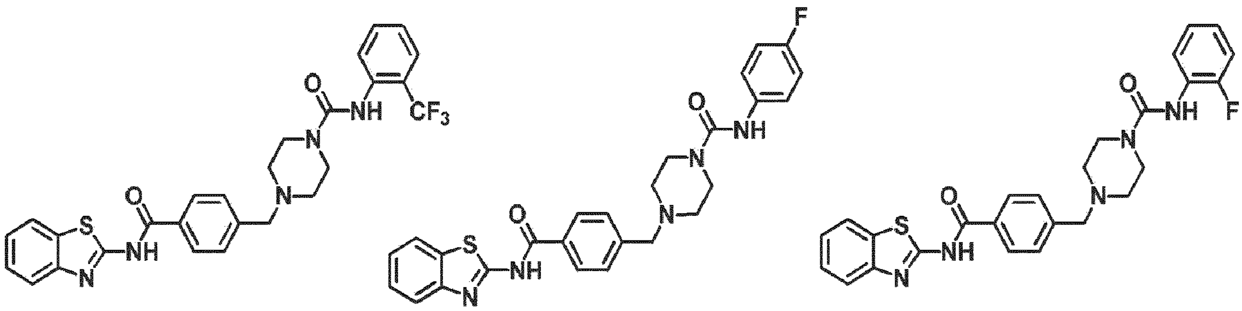

1-43 1-44 1-45

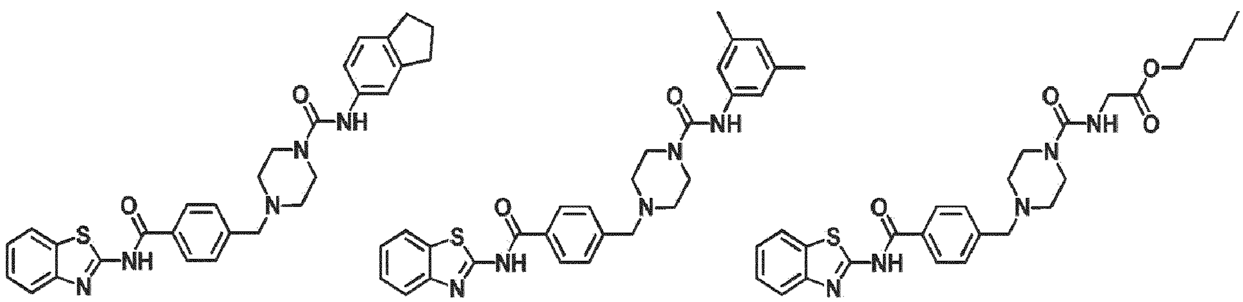

1-46 1-47 1-48

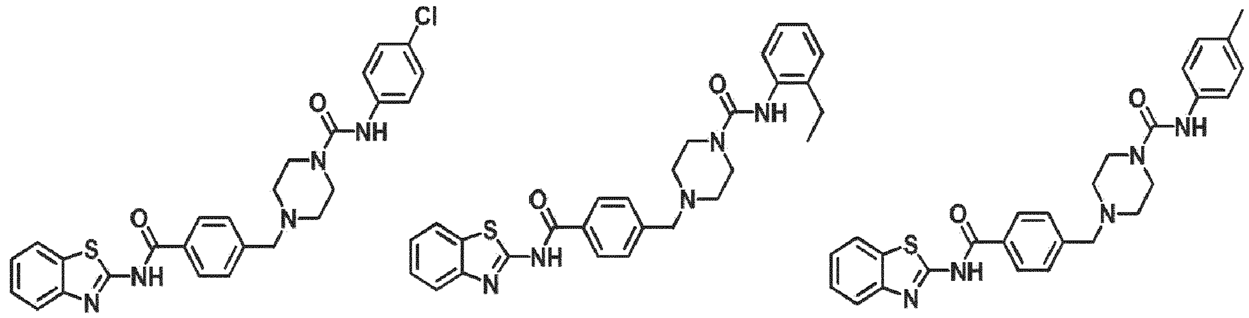

1-49 1-50 1-51

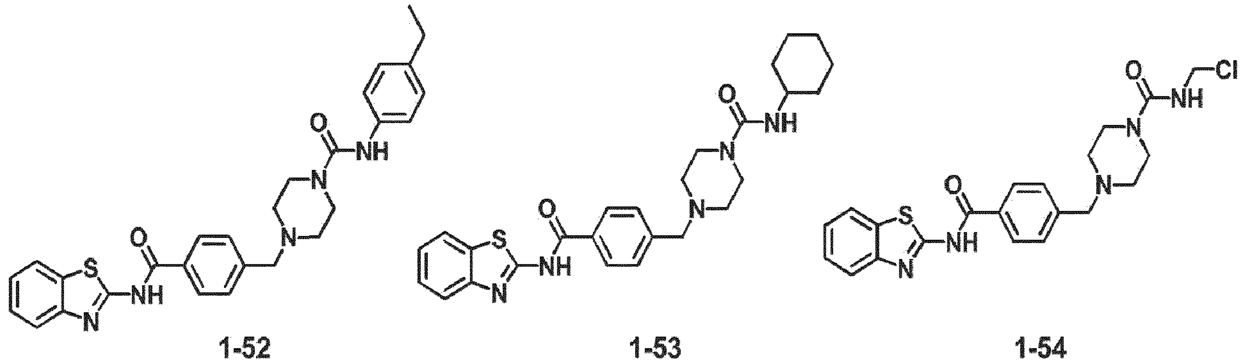

1-52 1-53 1-54

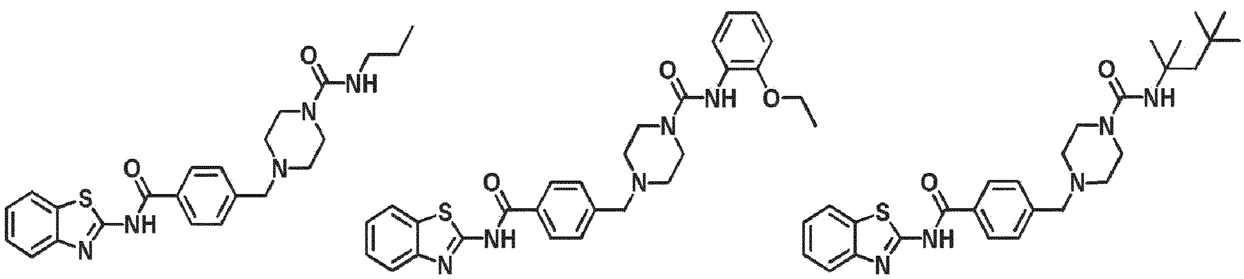

1-55 1-56 1-57

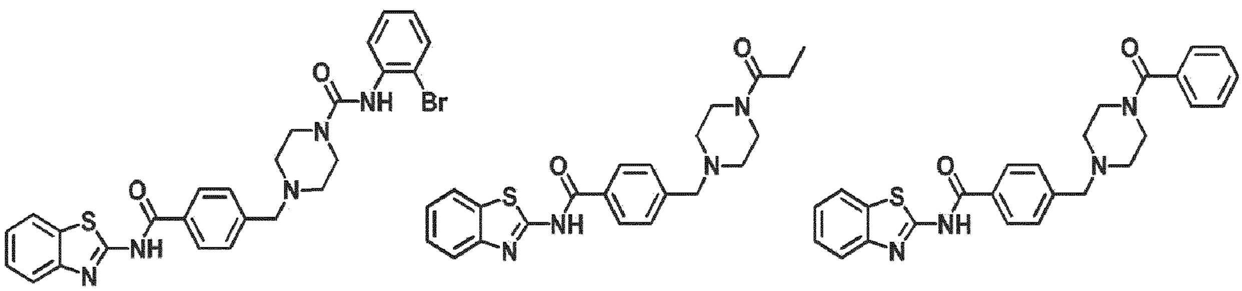

1-58 1-59 1-60

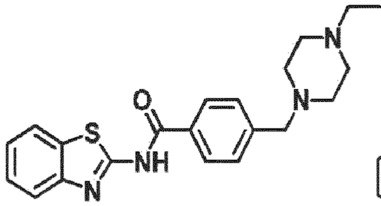

1-61

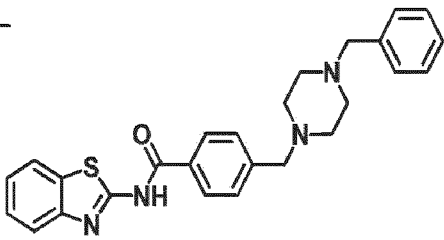

1-62

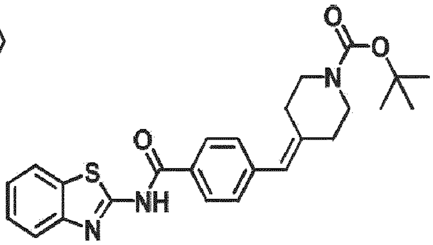

1-63

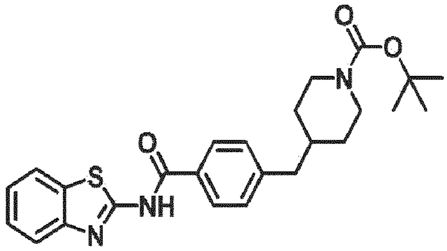

1-64

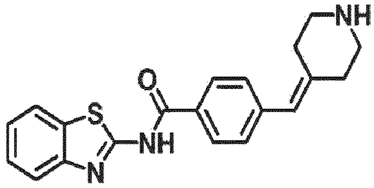

1-65

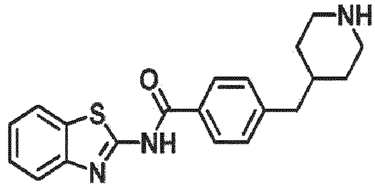

1-66

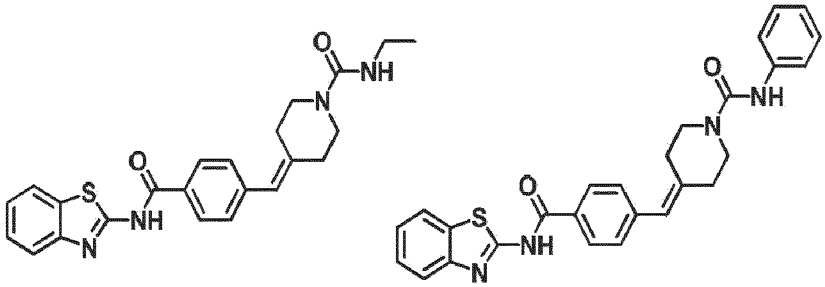

1-67

1-68

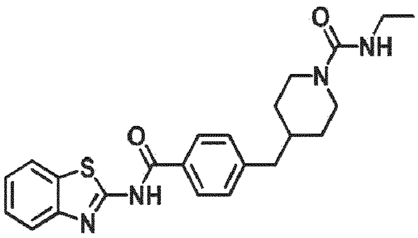

1-69

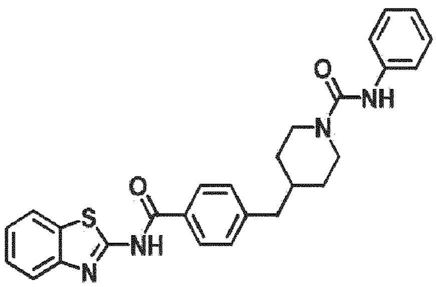

1-70

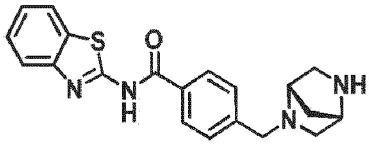

1-71

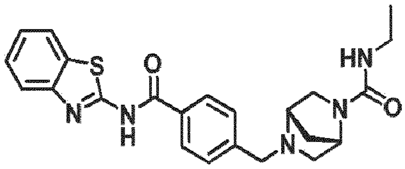

1-72

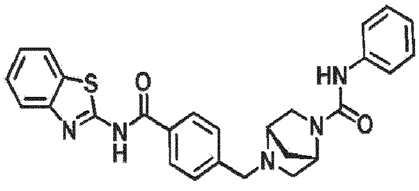

1-73

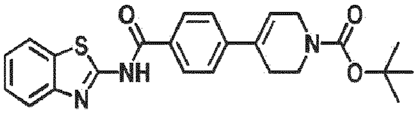

1-74

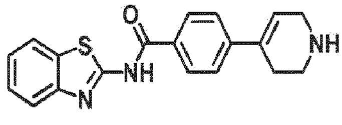

1-75

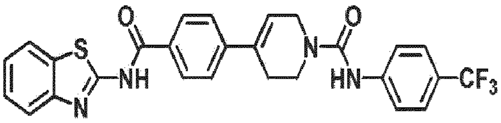

1-76

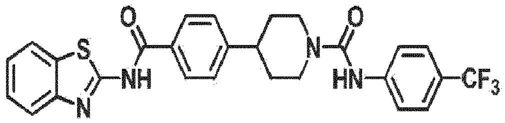

1-77

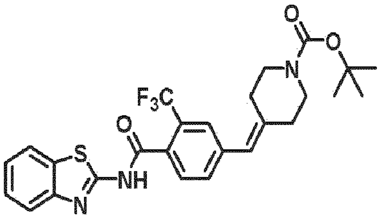

1-78

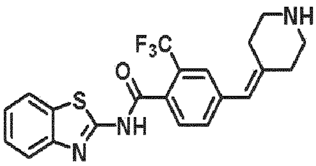

1-79

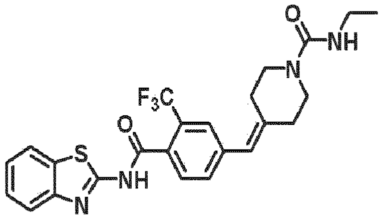

1-80

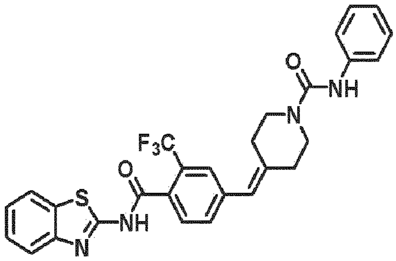

1-81

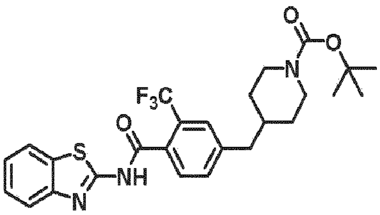

1-82

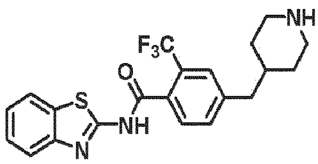

· TFA

1-83

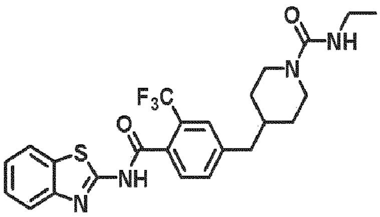

1-84

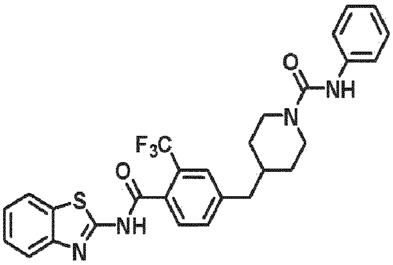

1-85

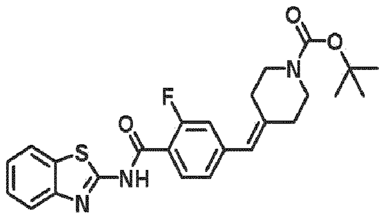

1-86

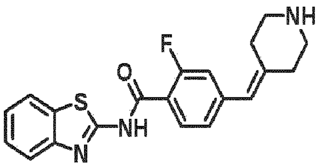

· TFA

1-87

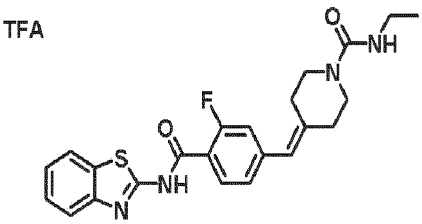

1-88

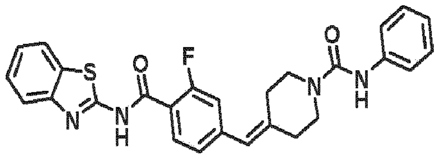

1-89

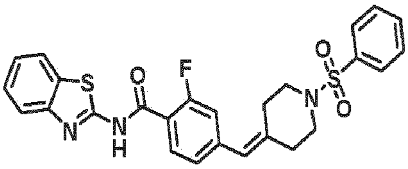

1-90

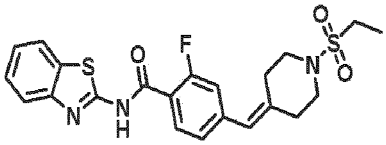

1-91

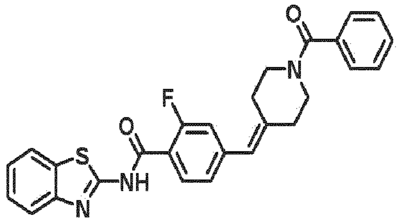

1-92

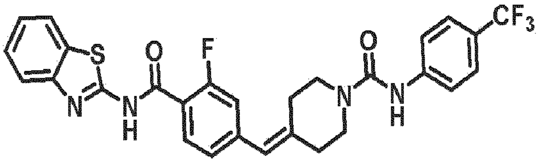

1-93

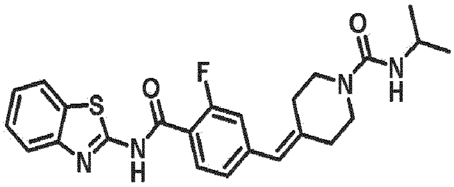

1-94

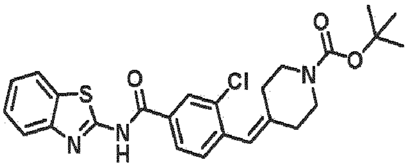

1-95

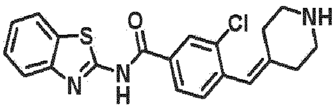

1-96

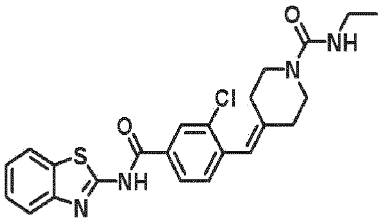

1-97

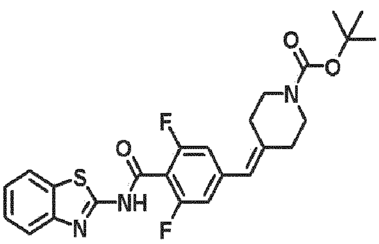

1-98

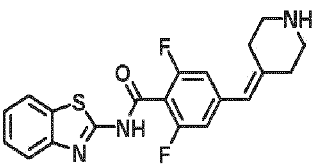

1-99

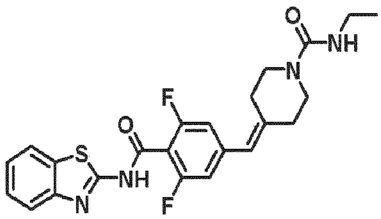

1-100

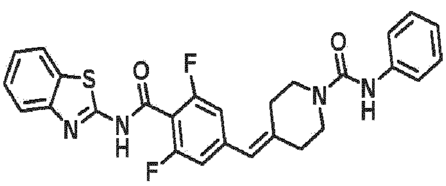

1-101

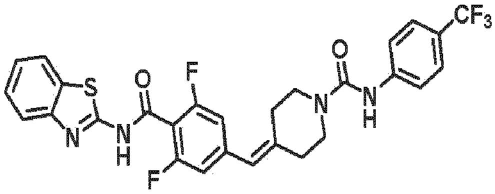

1-102

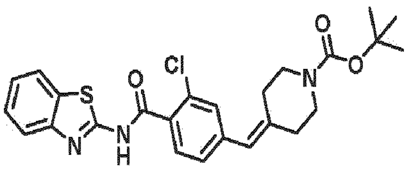

1-103

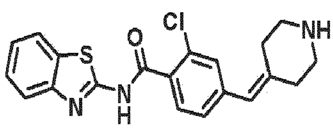

1-104

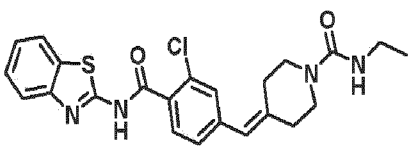

1-105

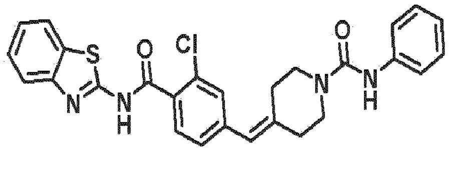

1-106

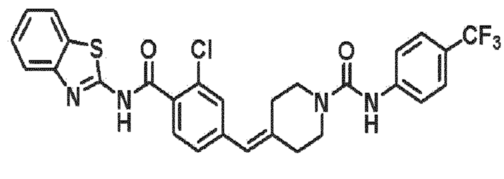

1-107

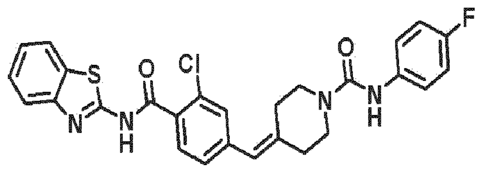

1-108

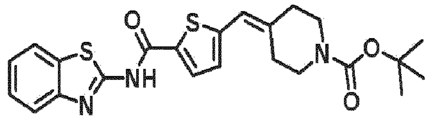

1-109

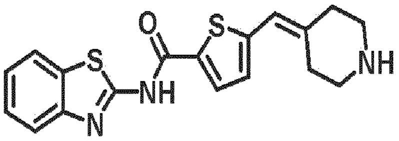

1-110

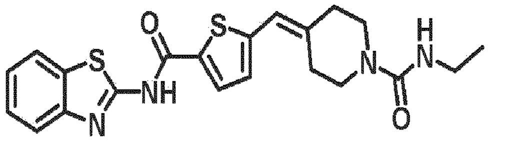

1-111

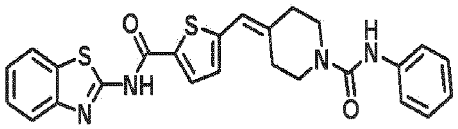

1-112

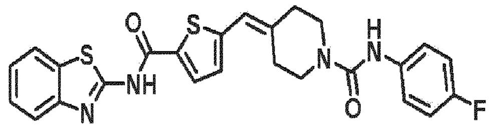

1-113

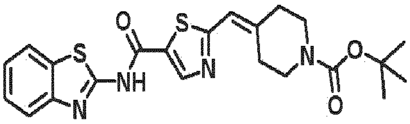

1-114

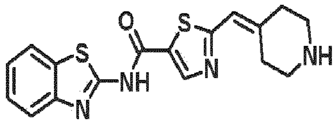

1-115

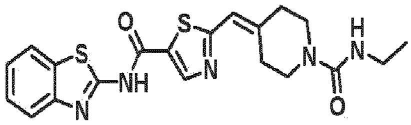

1-116

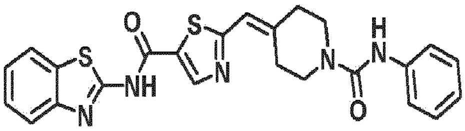

1-117

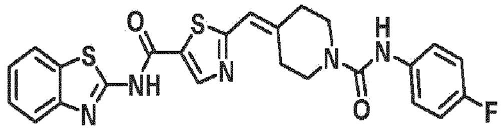

1-118

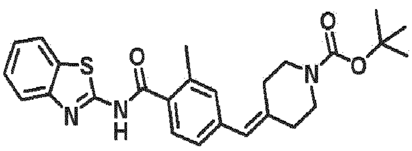

1-119

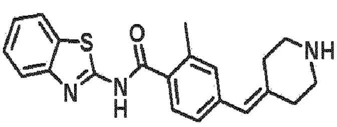

1-120

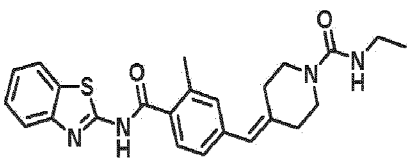

1-121

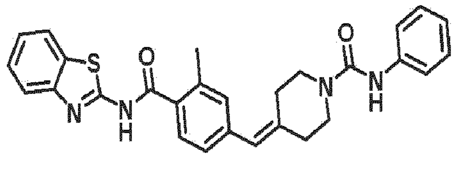

1-122

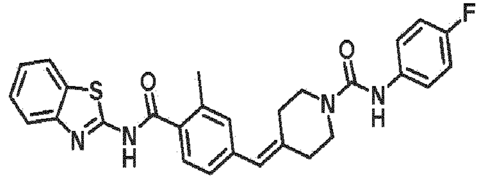

1-123

Also, the present invention provides the compound represented by Formula 1, stereoisomer, tautomer, derivative, hydrate, solvate or pharmaceutically acceptable salt thereof as an active ingredient and a pharmaceutical composition for preventing or treating of SIRTUIN 7 (hereinafter referred to as 'SIRT7') protein-related diseases comprising.

Wherein the SIRTUIN 7 protein-related disease may be selected from the group consisting of obesity, metabolic disorder, glucose resistance, insulin resistance, weight gain, fatty liver, liver fibrosis, hepatitis, liver cirrhosis, mitochondrial myopathy, brain disorder, diabetes, neurodegenerative disease, cardiovascular disease, eye disease, blood clotting disorder, hot flashes, lactic acidosis, MELAS Syndromes (mitochondrial encephalopathy, lactic acidosis, and stroke-like episodes) and cancer, but is not limited thereto.

Wherein the cancer may include gastric cancer, breast cancer, uterine cancer, colon cancer, colorectal cancer, pancreatic cancer, liver cancer, or prostate cancer, but is not limited thereto.

wherein the pharmaceutical composition may further comprise one or more pharmaceutically acceptable carriers.

Wherein the pharmaceutical composition has SIRTUIN 7 inhibitory activity.

Hereinafter, the present invention will be described in more detail through synthesis examples and examples. However, these examples are only for illustrating the present invention, and the scope of the present invention is not limited by these examples.

**Synthesis example 1: Synthesis of 4-((4-(tert-butoxycarbonyl)piperazin-1-yl)methyl)benzoic acid(compound 1)**

Compound 1

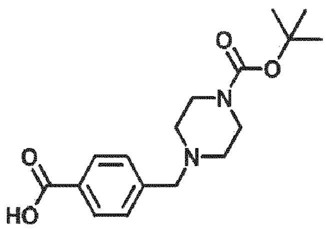

**Step 1:** After dissolving methyl 4-formylbenzoate (5.00 g, 30.46 mmol) and N-Boc-piperazine (5.11 g, 27.41 mmol) in DCM (100 mL), sodium triacetoxyborohydride (9.68 g, 45.69 mmol) was added and stirred at room temperature for 12 hours. The reaction product was diluted with EtOAc, washed with purified water, and the organic layer was extracted. The separated organic layer was dehydrated with anhydrous $Na_2SO_4$, concentrated under reduced pressure and separated by column chromatography to obtain 10.24 g of the compound, tert-butyl 4-(4-(methoxycarbonyl)benzyl)piperazine-1-carboxylate. $^1$H NMR (300 MHz, $CDCl_3$): δ 7.99 (d, 2H, J = 8.3 Hz), 7.40 (d, 2H, J = 8.2 Hz), 3.91 (s, 3H), 3.55 (s, 2H), 3.43 (t, 4H, J = 5.0 Hz), 2.39 (t, 4H, J = 5.0 Hz), 1.45 (s, 9H).

**Step 2:** tert-butyl 4-(4-(methoxycarbonyl)benzyl)piperazine-1-carboxylate (10.00 g, 29.90 mmol) and LiOH-$H_2O$ (1.88 g, 44.85 mmol) obtained from step 1 were added to 300 mL of THF/MeOH/$H_2O$ (ratio = 3:1:1) and stirred at room temperature for 4 hours. EtOAc was added to the reactant and ammonium chloride was added to pH 4.0-5.0. The water layer was extracted twice with ethyl acetate and dried with anhydrous sodium sulfate. The solvent was concentrated to obtain 9.6 of the title compound 4-((4-(tert-butoxycarbonyl)piperazin-1-yl)methyl)benzoic acid.

$^1$H NMR (300 MHz, $CDCl_3$): δ 8.05 (d, J = 8.0 Hz, 211), 7.43 (d, J = 8.0 Hz, 2H), 3.61 (s, 2H), 3.46 (t, J = 5.1 Hz, 4H), 2.44 (t, J = 4.8 Hz, 4H), 1.45 (s, 9H).

**Synthesis Example 2: Synthesis of Compound 2-1 to Compound 2-6**

**Synthesis example 2-1: Synthesis of 4-((6-methylpyridazin-3-yl)oxy)benzoic acid (compound 2-1)**

Compound 2-1

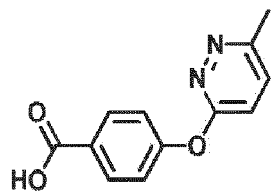

**Step 1:** Put 3-chloro-6-methylpyridazine (510 mg, 3.94 mmol) in methyl 4-hydroxybenzoate (500 mg, 3.28 mmol), then tripotassium phosphate (1.32 g, 6.27 mmol) and Palladium acetate (96 mg, 0.32 mmol) were added. After adding Xphos (204 mg, 0.43 mmol) to the flask, add Toluene as the reaction solvent. The reaction mixture was stirred at 100° C. for 24 hours, and the reaction product was confirmed by TLC. The reaction solution was diluted with ethyl acetate and washed with purified water. The organic layer was dehydrated with anhydrous $Na_2SO_4$, concentrated under reduced pressure and separated by column chromatography to obtain 480 mg of methyl 4-((6-methylpyridazin-3-yl)oxy)benzoate.
$^1$H NMR (500 MHz, DMSO): δ 8.24 (d, 2H, J= 8.61 Hz), 8.04 (d, 1H, J= 7.85 Hz), 7.80 (d, 1H, J= 8.10 Hz), 7.73 (d, 2H, J= 9.12 Hz), 3.98 (s, 3H), 2.59 (s, 3H).
**Step 2:** After dissolving methyl 4-((6-methylpyridazin-3-yl)oxy)benzoate (280 mg, 1.15 mmol) in THF/MeOH/$H_2O$=3:1:1 solution, Lithium hydroxide monohydrate (51 mg, 1.22 mmol) was added and stirred at room temperature for 4 hours. The reaction solution was concentrated under reduced pressure, diluted with purified water, and adjusted to pH 2-3 using 1N HCl. The precipitated crystals were stirred for 10 minutes or longer and filtered to obtain 210 mg of the title compound.
$^1$H NMR (500 MHz, DMSO): δ 12.75 (bs, 1H), 8.27 (d, 2H, J= 8.61 Hz), 8.05 (d, 1H, J= 7.85 Hz), 7.89 (d, 1H, J= 8.10 Hz), 7.81 (d, 2H, J= 9.12 Hz), 2.59 (s, 3H).

**Synthesis example 2-2: Synthesis of 3-((6-methylpyridazin-3-yl)oxy)benzoic acid (compound 2-2)**

Compound 2-2

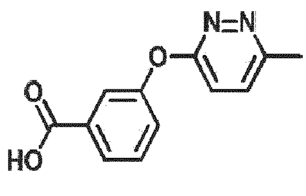

250 mg of the title compound was obtained by reacting Methyl 3-hydroxybenzoate (500 mg, 3.28 mmol) and 3-chloro-6-methylpyridazine (510 mg, 3.94 mmol) in the same manner as in Synthesis Example 2-1.
$^1$H NMR (300 MHz, DMSO): δ 12.72 (bs, 1H), 8.04 (d, 1H, J= 7.70 Hz), 7.92 (s, 1H), 7.71 (d, 1H, J= 9.05 Hz), 7.64 (d, 1H, J= 7.70 Hz), 7.16 (m, 2H), 2.59 (s, 3H).

**Synthesis example 2-3: Synthesis of 4-((5-ethylpyrimidin-2-yl)oxy)benzoic acid (compound 2-3)**

Compound 2-3

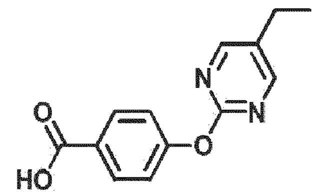

**Step 1:** After dissolving methyl 4-hydroxybenzoate (500 mg, 3.28 mmol) and 2-chloro-5-ethylpyrimidine (516 mg, 3.61 mmol) in anhydrous acetonitrile (40 mL), potassium carbonate (543 mg, 3.94 mmol) was added and reacted at 90 ° C overnight. After confirming the reaction product by TLC, the reaction solution was diluted with ethyl acetate and washed with purified water. The organic layer was dehydrated with anhydrous $Na_2SO_4$, concentrated under reduced pressure and separated by column chromatography to obtain 510 mg of methyl 4-((5-ethylpyrimidin-2-yl)oxy)benzoate.

$^1H$ NMR (500 MHz, DMSO): δ 8.58 (s, 2H), 8.04 (d, 2H, J= 7.81 Hz), 7.80 (d, 2H, J= 7.81 Hz), 3.94 (s, 3H), 2.63 (q, 2H, J= 7.55 Hz), 1.22 (t, 3H, J= 7.55 Hz).

**Step 2:** 200 mg of the title compound was obtained by reacting methyl 4-((5-ethylpyrimidin-2-yl)oxy)benzoate (280 mg, 1.15 mmol) obtained from step 1 in the same manner as in step 2 of synthesis example 2-1.

$^1H$ NMR (500 MHz, DMSO): δ 12.75 (s, 1H), 8.58 (s, 2H), 8.07 (d, 2H, J= 7.81 Hz), 7.88 (d, 2H, J= 7.81 Hz), 2.64 (q, 2H, J= 7.55 Hz), 1.25 (t, 3H, J= 7.55 Hz).

**Synthesis example 2-4: Synthesis of 3-((5-ethylpyrimidin-2-yl)oxy)benzoic acid (compound 2-4)**

Compound 2-4

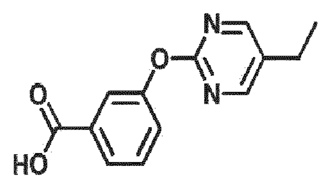

Methyl 3-hydroxybenzoate (500 mg, 3.28 mmol) and 2-chloro-5-ethylpyrimidine (516 mg, 3.61 mmol) were reacted in the same manner as in Synthesis Example 2-3 to obtain 340 mg of the title compound.

$^1H$ NMR (500 MHz, DMSO): δ 12.73 (s, 1H), 8.57 (s, 2H), 8.05 (t, 2H, J= 7.94 Hz), 7.97 (m, 1H), 7.99 (d, 1H, J= 7.94 Hz), 2.62 (q, 2H, J= 7.54 Hz), 1.21 (t, 3H, J= 7.94 Hz).

**Synthesis example 2-5: Synthesis of 4-((5-fluoropyrimidin-2-yl)oxy)benzoic acid (compound 2-5)**

Compound 2-5

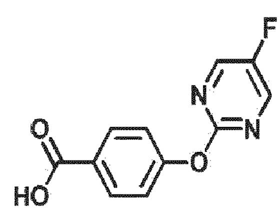

Methyl 4-hydroxybenzoate (1.00 g, 6.57 mmol) and 2-chloro-5-fluoropyrimidine (960 mg, 7.23 mmol) were

reacted in the same manner as in Synthesis Example 2-3 to obtain 1.15 g of the title compound.

$^1H$ NMR (500 MHz, DMSO): δ 12.76 (s, 1H), 8.79 (s, 2H), 8.24 (d, 2H, J= 8.45 Hz), 7.37 (d, 2H, J= 8.45 Hz).

**Synthesis example 2-6: Synthesis of 3-((5-fluoropyrimidin-2-yl)oxy)benzoic acid (compound 2-6)**

Compound 2-6

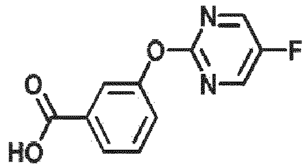

Methyl 3-hydroxybenzoate (1.00 g, 6.57 mmol) and 2-chloro-5-fluoropyrimidine (960 mg, 7.23 mmol) were reacted in the same manner as in Synthesis Example 2-3 to obtain 974 mg of the title compound.

$^1H$ NMR (500 MHz, DMSO): δ 12.74 (s, 1H), 8.79 (s, 2H), 8.23 (d, 1H, J= 8.45 Hz), 8.07 (d, 1H, J= 8.45 Hz), 7.59 (s, 1H), 7.38 (m, 1H).

**Synthesis example of 3: Synthesis of 4-(benzyloxy)benzoic acid (compound 3)**

Compound 3

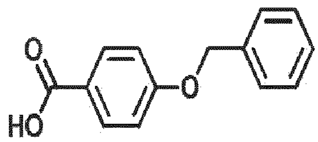

**Step 1:** Put Methyl 4-hydroxybenzoate (5.00 g, 32.86 mmol) and benzyl bromide (6.18 g, 36.15 mmol), potassium carbonate (9.08 g, 65.73 mmol) in anhydrous acetonitrile (60 mL) and stir at 60°C for 2 hours. After confirming the reaction product by TLC, the reaction solution was diluted with ethyl acetate and washed with purified water. The organic layer was dehydrated with anhydrous $Na_2SO_4$, concentrated under reduced pressure and separated by column chromatography to obtain 7.93 g of methyl 4-(benzyloxy)benzoate.

$^1H$ NMR (400 MHz, $CDCl_3$): δ 8.07 - 7.79 (m, 2H), 7.46 - 7.31 (m, 5H), 7.04 - 6.95 (m, 2H), 5.12 (s, 2H), 3.88 (s, 3H).

**Step 2:** After dissolving methyl 4-(benzyloxy)benzoate (2.6 g, 10.73 mmol) obtained from step 1 in THF/MeOH/$H_2O$=3:1:1 solution, add Lithium hydroxide monohydrate (1.35 g, 32.20 mmol) and stirring at room temperature for 4 hours, the reaction solution was concentrated under reduced pressure, diluted in purified water, and adjusted to pH 2-3 using 1N HCl. The precipitated crystals were stirred for 10 minutes or longer and filtered to obtain 2.4 g of the title compound.

$^1H$ NMR (400 MHz, DMSO): δ 8.06 (d, J = 8.5 Hz, 2H), 7.51 - 7.29 (m, 5H), 7.02 (d, J = 8.5 Hz, 2H), 5.14 (s, 2H).

**Synthesis example 4: Synthesis of N-(benzo[d]thiazol-2-yl)-4-formylbenzamide (compound 4)**

Compound 4

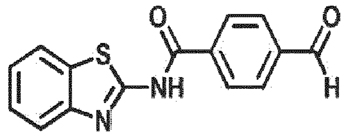

After adding 2-aminobenzothiazole (2.00 g, 13.32 mmol), 4-carboxybenzaldehyde (2.20 g, 14.65 mmol), HBTU (7.58 g, 19.97 mmol), HOBT (2.70 g, 19.97 mmol), NMM (2.69 g, 26.63 mmol) to anhydrous DMF (65 mL), the mixture was stirred overnight at room temperature. Ethyl acetate and water were added to the reactant, and the organic layer was dried with anhydrous sodium sulfate. After concentration, 2.5 g of the title compound was obtained by recrystallization with EA.

$^1$H NMR (300 MHz, DMSO): δ 13.16 (s, 1H), 10.14 (s, 1H), 8.32 (d, J = 8.1 Hz, 2H), 8.09 (d, J = 8.3 Hz, 2H), 8.05 (d, J = 7.8 Hz, 1H), 7.80 (d, J = 8.0 Hz, 1H), 7.53 - 7.44 (m, 1H), 7.41 - 7.32 (m, 1H).

**Synthesis example 5: Synthesis of N-(benzo[d]thiazol-2-yl)-4-bromobenzamide (compound 5)**

Compound 5

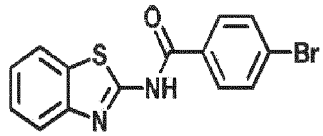

After adding 2-aminobenzothiazole (1.00 g, 6.66 mmol), 4-bromobenzoic acid (1.47 g, 7.32 mmol), HBTU (3.79 g, 9.99 mmol), DIPEA (2.32 mL, 13.32 mmol) to anhydrous DCM (30 mL), the mixture was stirred overnight at room temperature. After confirming the reaction product by TLC, the reaction solution was diluted with ethyl acetate and washed with purified water. The organic layer was dried with anhydrous sodium sulfate. The organic layer was dehydrated with anhydrous $Na_2SO_4$, concentrated under reduced pressure, and separated by column chromatography to obtain 2.00 g of the title compound.

$^1$H NMR (500 MHz, DMSO): δ 13.00 (s, 1H), 8.10 - 8.07 (m, 2H), 8.03 (dd, J = 8.0, 1.3 Hz, 1H), 7.81 - 7.78 (m, 3H), 7.48 (ddd, J = 8.2, 7.2, 1.3 Hz, 1H), 7.36 (ddd, J = 8.2, 7.2, 1.1 Hz, 1H).

**Synthesis example 6: Synthesis of N-(benzo[d]thiazol-2-yl)-4-bromo-2-(trifluoromethyl)benzamide (compound 6)**

Compound 6

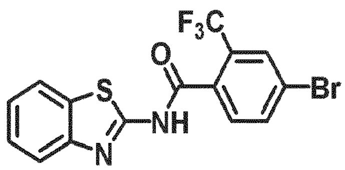

After adding 2-aminobenzothiazole (2.80 g, 18.64 mmol), 4-bromo-2-(trifluoromethyl)benzoic acid (5.15 g, 20.51 mmol), HBTU (7.78 g, 20.51 mmol), DIPEA (6.50 mL, 37.28 mmol)to anhydrous DCM (30 mL), the mixture was stirred for 4 hours at room temperature. After confirming the reaction product by TLC, the reaction solution was diluted with ethyl acetate and washed with purified water. The organic layer was dehydrated with anhydrous $Na_2SO_4$, concentrated under reduced pressure and separated by column chromatography to obtain 4.80 g of the title compound.

$^1H$ NMR (300 MHz, $CDCl_3$) δ 11.90 (s, 1H), 7.89 - 7.81 (m, 1H), 7.79 (d, J = 1.9 Hz, 1H), 7.58 (dd, J = 8.3, 1.9 Hz, 1H), 7.42 (d, J = 8.2 Hz, 1H), 7.39 - 7.21 (m, 2H), 7.03 - 6.94 (m, 1H).

**Synthesis example 7: Synthesis of N-(benzo[d]thiazol-2-yl)-4-bromo-2-fluorobenzamide (compound 7)**

Compound 7

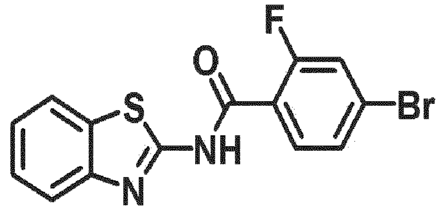

2-Aminobenzothiazole (2.80 g, 18.64 mmol) and 4-bromo-2-fluorobenzoic acid (4.49 g, 20.51 mmol) were reacted in the same manner as in Synthesis Example 6 to obtain 5.40 g of the title compound.
$^1H$ NMR (400 MHz, $CDCl_3$) δ 9.99 (s, 1H), 8.14 (t, J = 8.4 Hz, 1H), 7.89 (dt, J = 7.9, 1.0 Hz, 1H), 7.83 (dd, J = 8.1, 1.0 Hz, 1H), 7.56 (dd, J = 8.5, 1.8 Hz, 1H), 7.53 - 7.44 (m, 2H), 7.38 (ddd, J = 8.2, 7.2, 1.2 Hz, 1H).

**Synthesis example 8: Synthesis of N-(benzo[d]thiazol-2-yl)-4-bromo-3-chlorobenzamide (compound 8)**

Compound 8

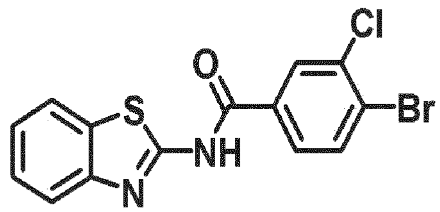

2-Aminobenzothiazole (3.00 g, 19.97 mmol) and 4-bromo-3-chlorobenzoic acid (5.17 g, 21.97 mmol) were reacted in the same manner as in Synthesis Example 6 to obtain 6.60 g of the title compound.
$^1H$ NMR (300 MHz, DMSO-$d_6$) δ 13.11 (s, 1H), 8.37 (t, J = 1.2 Hz, 1H), 8.07 - 7.95 (m, 3H), 7.78 (d, J = 8.0 Hz, 1H), 7.54 - 7.42 (m, 1H), 7.41 - 7.29 (m, 1H).

**Synthesis example 9: Synthesis of N-(benzo[d]thiazol-2-yl)-4-bromo-2,6-difluorobenzamide (compound 9)**

Compound 9

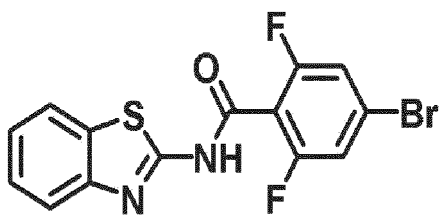

2-Aminobenzothiazole (2.90 g, 19.31 mmol) and 4-bromo-2,6-difluorobenzoic acid (5.03 g, 21.24 mmol) were reacted in the same manner as in Synthesis Example 6 to obtain 5.40 g of the title compound.

$^1$H NMR (300 MHz, DMSO-$d_6$) δ 13.29 (s, 1H), 8.11 - 8.01 (m, 1H), 7.87 - 7.77 (m, 1H), 7.79 - 7.68 (m, 2H), 7.56 - 7.44 (m, 1H), 7.44 - 7.32 (m, 1H).

**Synthesis example 10: Synthesis of N-(benzo[d]thiazol-2-yl)-4-bromo-2-chlorobenzamide (compound 10)**

Compound 10

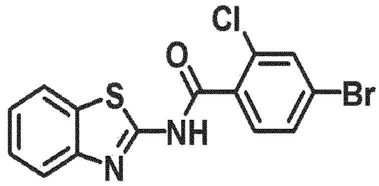

2-Aminobenzothiazole (1.50 g, 9.99 mmol) and 4-bromo-2chlorobenzoic acid (2.59 g, 10.99 mmol) were reacted in the same manner as in Synthesis Example 6 to obtain 2.50 g of the title compound.

$^1$H NMR (300 MHz, DMSO-$d_6$) δ 13.02 (s, 1H), 8.05 (d, J = 7.9, 1.2 Hz, 1H), 7.94 (d, J = 1.7 Hz, 1H), 7.80 (d, J = 8.0 Hz, 1H), 7.76 - 7.64 (m, 2H), 7.54 - 7.43 (m, 1H), 7.41 - 7.31 (m, 1H).

**Synthesis example 11: Synthesis of N-(benzo[d]thiazol-2-yl)-5-bromothiophene-2-carboxamide (compound 11)**

Compound 11

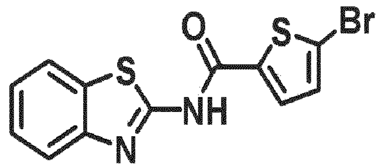

2-Aminobenzothiazole (2.00 g, 13.32 mmol) and 5-bromothiophene-2-carboxylic acid (3.03 g, 14.65 mmol) were reacted in the same manner as in Synthesis Example 6 to obtain 3.40 g of the title compound.

$^1$H NMR (300 MHz, DMSO-$d_6$) δ 13.15 (s, 1H), 8.16 - 7.94 (m, 2H), 7.75 (d, J = 8.0 Hz, 1H), 7.53 - 7.40 (m, 2H), 7.40 - 7.28 (m, 1H).

**Synthesis example 12: Synthesis of N-(benzo[d]thiazol-2-yl)-2-bromothiazole-5-carboxamide (compound 12)**

Compound 12

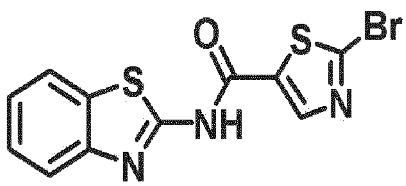

2-Aminobenzothiazole (2.00 g, 13.32 mmol) and 2-bromothiazole-5-carboxylic acid (3.05 g, 14.65 mmol) were reacted in the same manner as in Synthesis Example 6 to obtain 2.50 g of the title compound.

$^1H$ NMR (300 MHz, DMSO-$d_6$) δ 13.41 (s, 1H), 8.55 (s, 1H), 7.99 (d, J = 7.9 Hz, 1H), 7.71 (d, J = 8.1 Hz, 1H), 7.48 (t, J = 7.6 Hz, 1H), 7.35 (t, J = 7.6 Hz, 1H).

**Synthesis example 13: Synthesis of N-(benzo[d]thiazol-2-yl)-4-bromo-2-methylbenzamide (compound 13)**

Compound 13

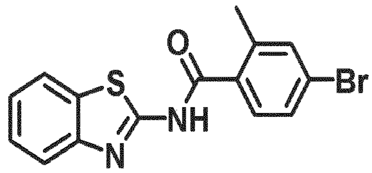

2-Aminobenzothiazole (0.70 g, 4.66 mmol) and 4-bromo-2-methylbenzoic acid (1.10 g, 5.13 mmol) were reacted in the same manner as in Synthesis Example 6 to obtain 1.30 g of the title compound.
$^1H$ NMR (300 MHz, DMSO-$d_6$) δ 12.83 (s, 1H), 8.03 (d, J = 7.9 Hz, 1H), 7.79 (d, J = 8.0 Hz, 1H), 7.66 - 7.51 (m, 3H), 7.51 - 7.41 (m, 1H), 7.35 (t, J = 7.6 Hz, 1H), 2.44 (s, 3H).

**Example 1: tert-butyl 4-(4-((1H-benzo[d]imidazol-2-yl)carbamoyl)benzyl) piperazine-1-carboxylate (compound 1-1)**

After adding compound 1 (500 mg, 1.56 mmol), 2-aminobenzimidazole (229 mg, 1.72 mmol), HBTU (888 mg, 2.34 mmol), HOBT (316 mg, 2.34 mmol), NMM (316 mg, 3.12 mmol) to anhydrous DMF (3 mL), the mixture was stirred overnight at room temperature. The reaction product was diluted with EtOAc and washed with purified water. The separated organic layer was dehydrated with anhydrous $Na_2SO_4$, concentrated under reduced pressure and separated by column chromatography to obtain 550 mg of the title compound.
$^1H$ NMR (300 MHz, $CDCl_3$): δ 8.15 (d, J = 8.0 Hz, 2H), 7.47 (d, J = 8.1 Hz, 2H), 7.19 - 7.12 (m, 4H), 3.63 (s, 2H), 3.47 (t, J = 5.0 Hz, 4H), 2.45 (t, J = 5.0 Hz, 4H), 1.49 (s, 9H).

**Example 2: tert-butyl 4-(4-((1-methyl-1H-benzo[d]imidazol-2-yl)carbamoyl) benzyl)piperazine-1-carboxylate (compound 1-2)**

After dissolving tert-butyl 4-(4-((1H-benzo[d]imidazol-2-yl)carbamoyl)benzyl)piperazine-1-carboxylate (50 mg, 0.11 mmol) obtained in Example 1 in anhydrous dichloromethane, NaH (5 mg, 0.23 mg) and iodomethane (17 mg, 0.13 mmol) were added at 0° C and stirred for 2 hours. The reaction product was diluted with EtOAc and washed with purified water. The separated organic layer was dehydrated with anhydrous $Na_2SO_4$, concentrated under reduced pressure and separated by column chromatography to obtain 32 mg of the title compound.
$^1H$ NMR (300 MHz, $CDCl_3$): δ 8.16 (d, J = 8.0 Hz, 2H), 7.45 (d, J = 8.1 Hz, 2H), 7.20 - 7.11 (m, 4H), 3.62 (s, 2H), 3.47 (t, J = 5.0 Hz, 4H), 3.44 (s, 3H), 2.44 (t, J = 5.0 Hz, 4H), 1.50 (s, 9H).

**Example 3: N-(1H-benzo[d]imidazol-2-yl)-4-(piperazin-1-ylmethyl)benzamide (compound 1-3)**

After tert-butyl 4-(4-((1H-benzo[d]imidazol-2-yl)carbamoyl)benzyl) piperazine-1-carboxylate (0.50 g, 1.15 mmol) obtained in Example 1 was dissolved in anhydrous DCM (5 mL), TFA (2 mL) was added, and the mixture was stirred at room temperature for 2 hours. After completion of the reaction, the solvent was concentrated under reduced pressure and washed twice with ether to obtain 500 mg of the title compound.
$^1H$ NMR (300 MHz, Acetone-d6): δ 8.34 (d, J = 8.1 Hz, 2H), 7.86 - 7.74 (m, 4H), 7.56 - 7.53 (m, 2H), 4.24 (s, 2H), 3.70 (t, J = 5.2 Hz, 4H), 3.35 (t, J = 5.2 Hz, 4H).

**Example 4: N-(1H-benzo[d]imidazol-2-yl)-4-((6-methylpyridazin-3-yl)oxy) benzamide (compound 1-4)**

After adding compound 2-1 (100 mg, 0.43 mmol), 1H-benzo[d]imidazol-2-amine (69 mg, 0.52 mmol), HBTU (189 mg, 0.50 mmol), N-methylmorpholine (50 mg, 0.50 mmol) and HOBt (62 mg, 0.46 mmol) to anhydrous DMF (3 mL), the mixture was stirred for 16 hours. The reaction product was diluted with EtOAc and washed with purified water. The separated organic layer was dehydrated with anhydrous $Na_2SO_4$, concentrated under reduced pressure and separated by column chromatography to obtain 86 mg of the title compound.

$^1$H NMR (500 MHz, DMSO): δ 12.29 (s, 2H), 8.23 (d, 2H, J= 8.94 Hz), 7.71 (d, 1H, J= 8.94 Hz), 7.48 (m, 3H), 7.33 (d, 2H, J= 8.47 Hz), 7.15 (dd, 2H, J= 3.29, 5.65 Hz), 2.59 (s, 3H).

**Example 5: N-(1H-benzo[d]imidazol-2-yl)-3-((6-methylpyridazin-3-yl)oxy) benzamide (compound 1-5)**

Compound 2-2 (100 mg, 0.43 mmol) was reacted in the same manner as in Example 4 to obtain 79 mg of the title compound.

$^1$H NMR (300 MHz, DMSO): δ 12.34 (s, 2H), 8.04 (d, 1H, J= 7.70 Hz), 7.92 (s, 1H), 7.71 (d, 1H, J= 9.05 Hz), 7.61 (t, 1H, J= 7.90 Hz), 7.47 (m, 4H), 7.16 (m, 2H), 2.59 (s, 3H).

**Example 6: N-(5-fluoro-1H-benzo[d]imidazol-2-yl)-4-((6-methylpyridazin-3-yl)oxy)benzamide (compound 1-6)**

5-Fluoro-1H-benzo[d]imidazol-2-amine (72 mg, 0.48 mmol) was reacted in the same manner as in Example 4 to obtain 41 mg of the title compound.

$^1$H NMR (400 MHz, DMSO): δ 12.29 (s, 2H), 8.21 (d, J = 8.4 Hz, 2H), 7.71 (d, J = 9.1 Hz, 1H), 7.48 (d, J = 8.8 Hz, 2H), 7.34 (d, J = 8.4 Hz, 2H), 7.26 (dd, J = 9.5, 2.5 Hz, 1H), 7.03 - 6.92 (m, 1H), 2.59 (s, 3H).

**Example 7: N-(5,6-difluoro-1H-benzo[d]imidazol-2-yl)-4-((6-methylpyridazin-3-yl)oxy)benzamide (compound 1-7)**

5,6-difluoro-1H-benzo[d]imidazol-2-amine (67 mg, 0.40 mmol) was reacted in the same manner as in Example 4 to obtain 37 mg of the title compound.

$^1$H NMR (400 MHz, DMSO): δ 12.33 (s, 1H), 8.20 (d, J = 8.5 Hz, 2H), 7.72 (d, J = 9.0 Hz, 1H), 7.55 - 7.43 (m, 3H), 7.35 (d, J = 8.5 Hz, 2H), 2.59 (s, 3H).

**Example 8: \N-(1H-benzo[d]imidazol-2-yl)-3-((5-ethylpyrimidin-2-yl)oxy) benzamide (compound 1-8)**

Compound 2-4 (105 mg, 0.43 mmol) was reacted in the same manner as in Example 4 to obtain 135 mg of the title compound.

$^1$H NMR (500 MHz, DMSO): δ 12.69 (s, 2H), 8.56 (s, 2H), 8.05 (d, 1H, J= 8.09 Hz), 7.93 (m, 1H), 7.59 (t, 1H, J= 8.09 Hz), 7.44 (m, 4H), 7.17 (m, 2H), 2.62 (q, 2H, J= 7.58 Hz), 1.21 (t, 3H, J= 7.58 Hz).

**Example 9: N-(1H-benzo[d]imidazol-2-yl)-4-((5-ethylpyrimidin-2-yl)oxy) benzamide (compound 1-9)**

Compound 2-3 (105 mg, 0.43 mmol) was reacted in the same manner as in Example 4 to obtain 121 mg of the title compound.

$^1$H NMR (500 MHz, DMSO): δ 12.29 (s, 211), 8.59 (s, 2H), 8.22 (d, 1H, J= 8.69 Hz), 7.47 (m, 1H), 7.34 (d, 2H, J= 8.72 Hz), 7.15 (m, 1H), 2.62 (q, 2H, J= 7.60 Hz), 1.21 (t, 3H, J= 7.46 Hz).

**Example 10: N-(1H-benzo[d]imidazol-2-yl)-4-((5-fluoropyrimidin-2-yl)oxy) benzamide (compound 1-10)**

Compound 2-5 (100 mg, 0.42 mmol) was reacted in the same manner as in Example 4 to obtain 87 mg of the title compound.

$^1$H NMR (500 MHz, DMSO): δ 12.29 (s, 1H), 8.79 (s, 2H), 8.24 (d, 2H, J= 8.45 Hz), 7.48 (m, 2H), 7.37 (d, 2H, J= 8.45 Hz), 7.16 (m, 2H).

**Example 11: N-(1H-benzo[d]imidazol-2-yl)-3-((5-fluoropyrimidin-2-yl)oxy) benzamide (compound 1-11)**

Compound 2-6 (100 mg, 0.42 mmol) was reacted in the same manner as in Example 4 to obtain 75 mg of the title compound.

$^1$H NMR (500 MHz, DMSO): δ 12.33 (s, 2H), 8.78 (s, 2H), 8.06 (d, 1H, J= 7.82 Hz), 7.95 (m, 1H), 7.61 (t, 1H, J= 7.82 Hz), 7.46 (m, 3H), 7.17 (m, 2H).

**Example 12: N-(1H-benzo[d]imidazol-2-yl)-4-(benzyloxy)benzamide (compound 1-12)**

Compound 4 (1.89 g, 8.26 mmol) was reacted in the same manner as in Example 4 to obtain 675 mg of the title compound.

$^1$H NMR (400 MHz, DMSO): δ 12.12 (s, 2H), 8.13 (d, J = 8.6 Hz, 2H), 7.53 - 7.29 (m, 7H), 7.17 - 7.06 (m, 4H), 5.22 (s, 2H).

**Example 13: N-(1H-benzo[d]imidazol-2-yl)-4-((4-(trifluoromethyl)benzyl)oxy) benzamide (compound 1-13)**

N-(1H-benzo[d]imidazol-2-yl)-4-(benzyloxy)benzamide (50 mg, 0.20 mmol) obtained in Example 12 was dissolved in anhydrous acetonitrile (5 mL), 4-(trifluoromethyl)benzyl bromide (52 mg, 0.22mmol) and potassium carbonate (82 mg, 0.59 mmol) were added, and the mixture was stirred at room temperature for 4 hours. The reaction product was diluted with EtOAc and washed with purified water. The separated organic layer was dehydrated with anhydrous $Na_2SO_4$, concentrated under reduced pressure and separated by column chromatography to obtain 21 mg of the title compound.
$^1$H NMR (400 MHz, $CDCl_3$): δ 12.79 (s, 1H), 7.75 - 7.65 (m, 4H), 7.63 (d, J = 8.1 Hz, 2H), 7.58 - 7.50 (m, 1H), 7.45 - 7.38 (m, 1H), 7.26 - 7.13 (m, 2H), 7.08 (d, J = 8.4 Hz, 2H), 5.31 (s, 2H).

**Example 14: N-(1-(4-(trifluoromethyl)benzyl)-1H-benzo[d]imidazol-2-yl)-4-((4-(trifluoromethyl)benzyl)oxy)benzamide (compound 1-14)**

N-(1H-benzo[d]imidazol-2-yl)-4-(benzyloxy)benzamide (50 mg, 0.20 mmol) obtained in Example 12 was dissolved in anhydrous acetonitrile (5 mL), 4-(trifluoromethyl)benzyl bromide (104 mg, 0.44 mmol) and potassium carbonate (82 mg, 0.59 mmol) were added, and the mixture was stirred at 60 ° C for 12 hours. The reaction product was diluted with EtOAc and washed with purified water. The separated organic layer was dehydrated with anhydrous $Na_2SO_4$, concentrated under reduced pressure and separated by column chromatography to obtain 31 mg of the title compound.
$^1$H NMR (400 MHz, $CDCl_3$): δ 12.79 (s, 1H), 8.20 (d, J = 8.4 Hz, 2H), 7.78 (d, J = 8.0 Hz, 2H), 7.75 - 7.65 (m, 4H), 7.63 (d, J = 8.1 Hz, 2H), 7.58 - 7.50 (m, 1H), 7.45 - 7.38 (m, 1H), 7.26 - 7.13 (m, 2H), 7.08 (d, J = 8.4 Hz, 2H), 5.61 (s, 2H), 5.31 (s, 2H).

**Example 15: N-(benzo[d]thiazol-2-yl)-4-((6-methylpyridazin-3-yl)oxy) benzamide (compound 1-15)**

Compound 2-1 (100 mg, 0.43 mmol) and 2-aminobenzothiazole (71 mg, 0.47 mmol) were reacted in the same manner as in Example 4 to obtain 102 mg of the title compound.
$^1$H NMR (500 MHz, DMSO): δ 12.95 (s, 1H), 8.24 (d, 2H, J= 8.61 Hz), 8.04 (d, 1H, J= 7.85 Hz), 7.80 (d, 1H, J= 8.10 Hz), 7.73 (d, 2H, J= 9.12 Hz), 7.50 (m, 3H), 7.37 (m, 3H), 2.59 (s, 3H).

**Example 16: 4-((6-methylpyridazin-3-yl)oxy)-N-(thiazolo[5,4-b]pyridin-2-yl) benzamide (compound 1-16)**

Compound 2-1 (100 mg, 0.43 mmol) and thiazolo[5,4-b]pyridin-2-amine (71 mg, 0.47 mmol) were reacted in the same manner as in Example 4 to obtain 54 mg of the title compound.
$^1$H NMR (400 MHz, DMSO): δ 13.04 (s, 1H), 8.51 (d, J = 4.6 Hz, 1H), 8.24 (d, J = 8.4 Hz, 2H), 8.16 (d, J = 8.2 Hz, 1H), 7.72 (d, J = 8.9 Hz, 1H), 7.58 - 7.44 (m, 2H), 7.38 (d, J = 8.3 Hz, 2H), 2.60 (s, 3H).

**Example 17: N-(benzo[dlthiazol-2-yl)-3-((6-methylpyridazin-3-yl)oxy) benzamide (compound 1-17)**

compound 2-2 (100 mg, 0.43 mmol and 2-aminobenzothiazole (71 mg, 0.47 mmol) were reacted in the same manner as in Example 4 to obtain 91 mg of the title compound.
$^1$H NMR (500 MHz, DMSO): δ 12.97 (s, 1H), 8.05 (t, 2H, J= 6.33 Hz), 7.97 (s, 1H), 7.79 (d, 1H, J= 7.91 Hz), 7.72 (m, 2H), 7.53 (m, 3H), 7.36 (t, 1H, J= 7.72 Hz), 2.57 (s, 3H).

**Example 18: N-(6-bromobenzo[d]thiazol-2-yl)-4-((6-methylpyridazin-3-yl)oxy) benzamide (compound 1-18)**

compound 2-1 (100 mg, 0.43 mmol) and 2-amino-6-bromobenzothiazole (108 mg, 0.47 mmol) were reacted in the same manner as in Example 4 to obtain 121 mg of the title compound.
$^1$H NMR (500 MHz, DMSO): δ 13.08 (s, 1H), 8.24 (d, 2H, J= 8.90 Hz), 8.03 (d, 1H, J= 8.39 Hz), 7.99 (d, 111, J= 7.62 Hz), 7.73 (d, 111, J= 8.90 Hz), 7.52 (m, 2H), 7.38 (d, 2H, J= 8.83 Hz), 2.59 (s, 3H).

**Example 19: N-(benzo[d]thiazol-2-yl)-4-((5-ethylpyrimidin-2-yl)oxy) benzamide (compound 1-19)**

compound 2-3 (100 mg, 0.41 mmol) was reacted in the same manner as in Example 4 to obtain 70 mg of the title compound.
$^1$H NMR (500 MHz, DMSO): δ 12.93 (s, 1H), 8.58 (s, 2H), 8.23 (d, 2H, J= 8.74 Hz), 8.04 (d, 1H, J= 7.81 Hz),

7.80 (d, 1H, J= 7.81 Hz), 7.49 (t, 111, J= 7.15 Hz), 7.39 (d, 2H, J= 8.74 Hz), 7.37 (t, 1H, J= 7.55 Hz), 2.63 (q, 2H, J= 7.55 Hz), 1.22 (t, 3H, J= 7.55 Hz).

**Example 20: N-(benzo[d]thiazol-2-yl)-3-((5-ethylpyrimidin-2-yl)oxy) benzamide (compound 1-20)**

compound 2-4 (100 mg, 0.41 mmol) was reacted in the same manner as in Example 4 to obtain 70 mg of the title compound.

$^1$H NMR (500 MHz, DMSO): δ 12.95 (s, 1H), 8.57 (s, 2H), 8.05 (t, 2H, J= 7.94 Hz), 7.97 (m, 1H), 7.99 (d, 1H, J= 7.94 Hz), 7.52 (d, 1H, J= 7.94 Hz), 7.49 (t, 1H, J= 7.14 Hz), 7.36 (t, 1H, J= 7.14 Hz), 2.62 (q, 2H, J= 7.54 Hz), 1.21 (t, 3H, J= 7.94 Hz).

**Example 21: N-(benzo[dlthiazol-2-yl)-4-((5-fluoropyrimidin-2-yl)oxy) benzamide (compound 1-21)**

compound 2-5 (100 mg, 0.43 mmol) was reacted in the same manner as in Example 4 to obtain 53 mg of the title compound.

$^1$H NMR (500 MHz, DMSO): δ 12.93 (s, 1H), 8.80 (s, 2H), 8.24 (d, 2H, J= 8.63 Hz), 8.04 (d, 1H, J= 7.61 Hz), 7.81 (d, 1H, J= 8.12 Hz), 7.50 (t, 1H, J= 7.03 Hz), 7.43 (d, 2H, J= 8.68 Hz), 7.37 (t, 1H, J= 8.63 Hz).

**Example 22: tert-butyl 4-(benzo[d]thiazol-2-yl)piperazine-1-carboxylate (compound 1-22)**

2-Chlorobenzothiazole (500 mg, 2.95 mmol), tert-butyl piperazine-1-carboxylate (576.47 mg, 3.09 mmol), potassium carbonate (427.73 mg, 3.09 mmol) were added to anhydrous DMF (10 mL) and stirred at 50° C for 3 hours. The reaction product was diluted with EtOAc and washed with purified water. The separated organic layer was dehydrated with anhydrous $Na_2SO_4$, concentrated under reduced pressure, and separated by column chromatography to obtain 482 mg of the title compound.

$^1$H NMR (300 MHz, $CDCl_3$): δ 7.63 - 7.55 (m, 2H), 7.34 - 7.31 (m, 1H), 7.12 - 7.07 (m, 1H), 3.61 (m, 8H), 1.59 - 1.49 (s, 9H).

**Example 23: 8-(benzo[d]thiazol-2-yl)-1,4-dioxa-8-azaspiro[4.5]decane (compound 1-23)**

4-piperidine ethylene ketal (443.17 mg, 3.09 mmol) was reacted in the same manner as in Example 22 to obtain 709 mg of the title compound.

$^1$H NMR (300 MHz, $CDCl_3$): δ 7.59 - 7.52 (m, 2H), 7.30 - 7.25 (m, 1H), 7.08 - 7.03 (m, 1H), 4.00 (s, 4H), 3.75 (t, J = 6 Hz, 4H), 1.83 (t, J = 6 Hz, 4H).

**Example 24: tert-butyl 2-(benzo[d]thiazol-2-yl)-2,7-diazaspiro[3.5]nonane-7-carboxylate (compound 1-24)**

7-Boc-2,7-diazaspiro[3,5]nonane (700.45 mg, 3.09 mmol) was reacted in the same manner as in Example 22 to obtain 832 mg of the title compound.

$^1$H NMR (300 MHz, $CDCl_3$): δ 7.62 - 7.58 (m, 2H), 7.33 - 7.30 (m, 1H), 7.11 - 7.06 (m, 1H), 3.93 (s, 4H), 3.43 - 3.39 (m, 4H), 1.84 - 1.80 (m, 4H), 1.46 (s, 1H).

**Example 25: tert-butyl (1S,4S)-5-(4-(benzo[d]thiazol-2-ylcarbamoyl)benzyl)-2,5-diazabicyclo[2.2.1]heptane-2-carboxylate (compound 1-25)**

compound 4 (1.00 g, 3.54 mmol) and tert-butyl (1S,4S)-2,5-diazabicyclo[2.2.1]heptane-2-carboxylate (632.03 mg, 3.19 mmol) were dissolved in 1,2-dichloroethane (20 mL), sodium triacetoxyborohydride (1.13 g, 5.31 mmol) was added, followed by stirring overnight at room temperature. The pH was titrated to 8.0-9.0 with sodium carbonate solution, diluted with EtOAc, and washed with purified water. The separated organic layer was dehydrated with anhydrous $Na_2SO_4$, concentrated under reduced pressure and separated by column chromatography to obtain 1.32 g of the title compound.

$^1$H NMR (400 MHz, $CDCl_3$): δ 11.07 (s, 1H), 7.99 - 7.93 (m, 2H), 7.88 - 7.82 (m, 1H), 7.47 - 7.40 (m, 3H), 7.33 - 7.28 (m, 2H), 4.39 - 4.26 (m, 1H), 3.78 (d, J = 4.4 Hz, 2H), 3.61 - 3.40 (m, 2H), 3.20 - 3.14 (m, 1H), 2.90 - 2.81 (m, 1H), 2.70 - 2.48 (m, 1H), 1.86 (d, J = 9.9 Hz, 1H), 1.68 (d, J = 9.6 Hz, 1H), 1.47 (s, 9H).

**Example 26: tert-butyl 4-(4-(benzo[d]thiazol-2-ylcarbamoyl)benzyl) piperazine-1-carboxylate (compound 1-26)**

N-Boc-piperazine (658 mg, 3.54 mg) was reacted in the same manner as in Example 25 to obtain 1.35g of the title compound.

$^1$H NMR (500 MHz, $CDCl_3$): δ 11.12 (s, 1H), 7.95 (d, J = 8.2 Hz, 2H), 7.88 - 7.82 (m, 1H), 7.42 (d, J = 8.0 Hz, 2H), 7.43 - 7.37 (m, 1H), 7.33 - 7.27 (m, 2H), 3.55 (s, 2H), 3.42 (t, J = 5.0 Hz, 4H), 2.36 (t, J = 5.0 Hz, 4H), 1.46 (s, 9H).

**Example 27: tert-butyl 7-(4-(benzo[dlthiazol-2-ylcarbamoyl)benzyl)-2,7-diazaspiro[3.5]nonane-2-carboxylate (compound 1-27)**

Tert-butyl 2,7-diazaspiro[3.5]nonane-2-carboxylate (144 mg, 0.64 mmol) was reacted in the same manner as in Example 25 to obtain 220 mg of the title compound.

$^1$H NMR (300 MHz, $CDCl_3$): δ 7.95 (d, J = 7.9 Hz, 2H), 7.86 (d, J = 7.3 Hz, 1H), 7.50 (d, J = 7.5 Hz, 1H), 7.43 (d, J = 8.0 Hz, 2H), 7.36 - 7.28 (m, 2H), 3.61 (s, 4H), 3.50 (s, 2H), 2.86 - 2.71 (m, 2H), 1.76-1.69 (m, 6H), 1.44 (s, 9H).

**Example 28: tert-butyl 9-(4-(benzoLdlthiazol-2-ylcarbamoyl)benzyl)-3,9-diazaspiro[5.5]undecane-3-carboxylate (compound 1-28)**

tert-butyl 3,9-diazaspiro[5.5]undecane-3-carboxylate (162 mg, 0.64 mmol) was reacted in the same manner as in Example 25 to obtain 259 mg of the title compound.

$^1$H NMR (300 MHz, $CDCl_3$): δ 7.93 (d, J = 8.0 Hz, 2H), 7.86 (d, J = 7.4 Hz, 1H), 7.56 (d, J = 7.3 Hz, 1H), 7.45 (d, J = 8.0 Hz, 2H), 7.34 (tt, J = 7.7, 6.1 Hz, 2H), 3.55 (s, 2H), 3.36 (t, J = 5.8 Hz, 4H), 2.39 (t, J = 5.6 Hz, 4H), 1.52 (t, J = 5.6 Hz, 4H), 1.45 (m, 13H).

**Example 29: 4-((1,4-dioxa-8-azaspiro[4.5]decan-8-yl)methyl)-N-(benzo[d] thiazol-2-yl)benzamide (compound 1-29)**

1,4-dioxa-8-azaspiro[4.5]decane (91 mg, 0.64 mmol) was reacted in the same manner as in Example 25 to obtain 120 mg of the title compound.

$^1$H NMR (300 MHz, $CDCl_3$): δ 7.95 (d, J = 8.1 Hz, 2H), 7.89 - 7.82 (m, 1H), 7.46 - 7.41 (m, 3H), 7.32 - 7.28 (m, 2H), 3.96 (d, J = 2.3 Hz, 4H), 3.57 (s, 2H), 2.51 (t, J = 5.7 Hz, 4H), 1.74 (t, J = 5.7 Hz, 4H).

**Example 30: 2-(7-benzyl-2,7-diazaspiro[3.5]nonan-2-yl)benzo[d]thiazole (compound 1-30)**

2-(2,7-Diazaspiro[3.5]nonan-2-yl)benzo[d]thiazole (100 mg, 0.28 mmol) was dissolved in anhydrous acetonitrile (4 ml), potassium carbonate (124 mg, 0.90 mmol) and benzyl bromide (56 mg, 0.33 mmol) were added and reacted overnight. The reaction product was diluted with EtOAc and washed with purified water. The separated organic layer was dehydrated with anhydrous $Na_2SO_4$, concentrated under reduced pressure, and separated by column chromatography to obtain 24 mg of the title compound.

$^1$H NMR (300 MHz, acetone): δ 7.70 (d, J = 9 Hz, 1H), 7.47 (d, J = 9 Hz, 1H), 7.36 - 7.20 (m, 6H), 7.08 - 7.03 (m, 1H), 3.87 (s, 4H), 3.47 (s, 2H), 2.39 (s, 2H), 1.87 (t, J = 6 Hz, 4H).

**Example 31: N-(benzo[d]thiazol-2-yl)-4-(piperazin-1-ylmethyl)benzamide (compound 1-31)**

Tert-butyl 4-(4-(benzo[d]thiazol-2-ylcarbamoyl)benzyl)piperazine-1-carboxylate (2.0 g, 4.42 mmol) obtained in Example 26 was reacted in the same manner as in Example 3 to obtain 2.2 g of the title compound.

$^1$H NMR (300 MHz, Acetone-d6): δ 8.27 - 8.52 (m, 2H), 7.98 - 7.91 (m, 2H), 7.77 - 7.69 (m, 4H), 4.31 (s, 2H), 3.71 (s, 4H), 3.44 (s, 4H).

**Example 32: N-(benzo[d]thiazol-2-yl)-4-((4-(ethylsulfonyl)piperazin-1-yl) methyl)benzamide (compound 1-32)**

N-(benzo[d]thiazol-2-yl)-4-((4-(ethylsulfonyl)piperazin-1-yl)methyl)benzamide (100 mg, 0.28 mmol) obtained in Example 31 was dissolved in anhydrous dichloromethane (4 mL), ethanesulfonyl chloride (30 μL, 0.31 mmol) and trimethylamine (80 pL, 0.57 mmol) were added, and the mixture was stirred at room temperature for 3 hours. The reaction product was diluted with EtOAc and washed with purified water. The separated organic layer was dehydrated with anhydrous $Na_2SO_4$, concentrated under reduced pressure, and separated by column chromatography to obtain 88 mg of the title compound.

$^1$H NMR (500 MHz, $CDCl_3$): δ 10.56 (s, 1H), 7.90 (t, J = 8.1 Hz, 4H), 7.88 - 7.82 (m, 1H), 7.56 - 7.50 (m, 1H), 7.39 - 7.27 (m, 2H), 3.56 (s, 2H), 3.45 (qd, J = 7.1, 5.2 Hz, 2H), 3.07 (s, 4H), 2.54 (t, J = 5.0 Hz, 4H), 0.98 (t, J= 7.1 Hz, 3H).

**Example 33: N-(benzo[d]thiazol-2-yl)-4-((4-(N,N-dimethylsulfamoyl)piperazin -1-yl)methyl)benzamide (compound 1-33)**

N,N-Dimethylsulfamoyl chloride (34 μL , 0.31 mmol) was reacted in the same manner as in Example 32 to obtain 79 mg of the title compound.

$^1$H NMR (500 MHz, $CDCl_3$): δ 10.51 (s, 1H), 7.91 (t, J = 8.1 Hz, 4H), 7.88 - 7.80 (m, 1H), 7.56 - 7.54 (m, 1H), 7.39 - 7.29 (m, 2H), 3.56 (s, 2H), 3.07 (s, 4H), 2.66 (s, 6H), 2.54 (t, J = 5.0 Hz, 4H).

**Example 34: N-(benzo[d]thiazol-2-yl)-4-((4-(cyclopropylsulfonyl)piperazin-1-yl)methyl)benzamide (compound 1-34)**

Cyclopropylsulfonyl chloride (48 mg, 0.31 mmol) was reacted in the same manner as in Example 32 to obtain 87 mg of the title compound.

$^1$H NMR (500 MHz, $CDCl_3$): δ 10.83 (s, 1H), 7.96 (d, J = 8.3 Hz, 2H), 7.89 - 7.83 (m, 1H), 7.51 - 7.45 (m, 1H), 7.45 (d, J = 8.2 Hz, 2H), 7.35 - 7.29 (m, 2H), 3.60 (s, 2H), 3.33 (t, J = 4.9 Hz, 4H), 2.54 (t, J = 4.9 Hz, 4H), 2.32 - 2.22 (m, 1H), 1.20 - 1.15 (m, 2H), 1.03 - 0.97 (m, 3H).

**Example 35: N-(benzo[d]thiazol-2-yl)-4-((4-(phenylulfonyl)piperazin-1-yl) methyl)benzamide (compound 1-35)**

Benzenesulfonyl chloride (60 mg, 0.31 mmol) was reacted in the same manner as in Example 32 to obtain 85 mg of the title compound.

$^1$H NMR (500 MHz, $CDCl_3$): δ 10.81 (s, 1H), 7.91 (d, J = 8.3 Hz, 2H), 7.86 - 7.83 (m, 1H), 7.79 - 7.75 (m, 2H), 7.65 - 7.60 (m, 1H), 7.57 - 7.54 (m, 2H), 7.50 - 7.46 (m, 1H), 7.36 (d, J = 8.3 Hz, 2H), 7.35 - 7.28 (m, 2H), 3.54 (s, 2H), 3.04 (s, 4H), 2.52 (t, J = 4.9 Hz, 4H).

**Example 36: N-(benzo[dlthiazol-2-yl)-4-((4-((4-(trifluoromethyl)phenyl) sulfonyl)piperazin-1-yl)methyl)benzamide (compound 1-36)**

4-(Trifluoromethyl)benzenesulfonyl chloride (76 mg, 0.31 mmol) was reacted in the same manner as in Example 32 to obtain 115 mg of the title compound. $^1$H NMR (500 MHz, $CDCl_3$): δ 10.56 (s, 1H), 7.90 (t, J = 8.1 Hz, 4H), 7.88 - 7.82 (m, 1H), 7.82 (d, J = 8.2 Hz, 2H), 7.56 - 7.50 (m, 1H), 7.38 (d, J = 8.3 Hz, 2H), 7.39 - 7.27 (m, 2H), 3.56 (s, 2H), 3.07 (s, 4H), 2.54 (t, J = 5.0 Hz, 4H).

**Example 37: 4-(4-(benzo[d]thiazol-2-ylcarbamoyl)benzyl)-N-ethylpiperazine-1-carboxamide (compound 1-37)**

N-(Benzo[d]thiazol-2-yl)-4-((4-(ethylsulfonyl)piperazin-1-yl)methyl) benzamide (100 mg, 0.28 mmol) obtained in Example 31 was dissolved in anhydrous dichloromethane (4 mL), triethylamine (80 μL, 0.57 mmol) and ethyl isocyanate (24 pL, 0.31 mmol) were added, and the mixture was stirred at room temperature for 3 hours. The reaction product was diluted with EtOAc and washed with purified water. The separated organic layer was dehydrated with anhydrous $Na_2SO_4$, concentrated under reduced pressure, and separated by column chromatography to obtain 99 mg of the title compound.

$^1$H NMR (500 MHz, DMSO): δ 12.84 (s, 1H), 8.12 (d, J = 7.9 Hz, 2H), 8.02 (d, J = 7.8 Hz, 1H), 7.78 (d, J = 8.1 Hz, 1H), 7.51 - 7.45 (m, 3H), 7.38 - 7.30 (m, 1H), 3.57 (s, 2H), 3.28 (t, J = 4.9 Hz, 4H), 3.03 (qd, J = 7.1, 5.3 Hz, 2H), 2.33 (t, J = 5.0 Hz, 4H), 0.99 (t, J = 7.1 Hz, 3H).

**Example 38: 4-(4-(benzo[d]thiazol-2-ylcarbamoyl)benzyl)-N-phenylpiperazine-1-carboxamide (compound 1-38)**

Phenyl isocyanate (34 μL, 0.31 mmol) was reacted in the same manner as in Example 37 to obtain 110 mg of the title compound.

$^1$H NMR (500 MHz, DMSO): δ 12.86 (s, 1H), 8.50 (s, 1H), 8.14 (d, J = 8.4 Hz, 2H), 8.03 (dd, J = 7.9, 1.1 Hz, 1H), 7.80 (d, J = 8.0 Hz, 1H), 7.53 (d, J = 8.3 Hz, 2H), 7.52 - 7.42 (m, 3H), 7.35 (td, J = 7.6, 1.1 Hz, 1H), 7.26 - 7.18 (m, 2H), 6.93 (tt, J = 7.4, 1.2 Hz, 1H), 3.63 (s, 2H), 3.48 (t, J = 4.9 Hz, 4H), 2.43 (t, J = 5.0 Hz, 4H).

**Example 39: 4-(4-(benzo[d]thiazol-2-ylcarbamoyl)benzyl)-N-(4-(trifluoromethyl)phenyl)piperazine-1-carboxamide (compound 1-39)**

4-(Trifluoromethyl)phenyl isocyanate (44 pL, 0.31 mmol) was reacted in the same manner as in Example 37 to obtain 107 mg of the title compound.

$^1$H NMR (500 MHz, DMSO): δ 12.98 - 12.71 (m, 1H), 8.14 (d, J = 8.1 Hz, 2H), 8.03 (d, J = 7.9 Hz, 1H), 7.80

(d, J = 8.0 Hz, 1H), 7.69 (d, J = 8.5 Hz, 2H), 7.58 (d, J = 8.6 Hz, 2H), 7.53 (d, J = 8.1 Hz, 2H), 7.50 - 7.46 (m, 1H), 7.38 - 7.32 (m, 1H), 3.63 (s, 2H), 3.57 - 3.45 (m, 4H), 2.44 (t, J = 5.0 Hz, 4H).

**Example 40: 4-(4-(benzo[d]thiazol-2-ylcarbamoyl)benzyl)-N-isopropyl-piperazine-1-carboxamide (compound 1-40)**

Isopropyl isocyanate (21 mg, 0.25 mmol) was reacted in the same manner as in Example 37 to obtain 54 mg of the title compound.

$^1$H NMR (300 MHz, $CDCl_3$): δ 8.06 - 7.91 (m, 2H), 7.91 - 7.76 (m, 1H), 7.50 - 7.43 (m, 3H), 7.38 - 7.27 (m, 2H), 3.98 (h, J = 6.6 Hz, 1H), 3.57 (s, 2H), 3.35 (t, J = 5.0 Hz, 4H), 2.42 (t, J = 5.1 Hz, 4H), 1.16 (d, J = 6.5 Hz, 3H).

**Example 41: N-(adamantan-1-yl)-4-(4-benzo[d]thiazol-2-ylcarbamoyl)benzyl) piperazine-1-carboxamide (compound 1-41)**

1-Adamantyl isocyanate (44 mg, 0.25 mmol) was reacted in the same manner as in Example 37 to obtain 95 mg of the title compound.

$^1$H NMR (300 MHz, $CDCl_3$): δ 7.96 (d, J = 8.3 Hz, 2H), 7.91 - 7.78 (m, 1H), 7.58 - 7.48 (m, 1H), 7.46 (d, J = 8.1 Hz, 2H), 7.40 - 7.28 (m, 2H), 3.57 (s, 2H), 3.32 (t, J = 5.1 Hz, 4H), 2.42 (t, J = 5.1 Hz, 4H), 2.06 (d, J = 7.5 Hz, 3H), 2.01 - 1.87 (m, 6H), 1.68-1.66 (m, 6H).

**Example 42: 4-(4-(benzo[d]thiazol-2-ylcarbamoyl)benzyl)-N-(4-methoxyphenyl) piperazine-1-carboxamide (compound 1-42)**

4-Methoxyphenyl isocyanate (37 mg, 0.25 mmol) was reacted in the same manner as in Example 37 to obtain 61 mg of the title compound.

$^1$H NMR (300 MHz, $CDCl_3$): δ 10.63 (s, 1H), 7.97 (d, J = 7.8 Hz, 2H), 7.86 (d, J = 7.6 Hz, 1H), 7.57 (d, J = 7.8 Hz, 1H), 7.48 (d, J = 7.9 Hz, 2H), 7.35 (p, J = 7.2 Hz, 2H), 7.26 - 7.23 (m, 2H), 6.84 (d, J = 8.3 Hz, 2H), 6.27 (s, 1H), 3.78 (s, 3H), 3.61 (s, 2H), 3.50 (t, J = 5.1 Hz, 4H), 2.49 (t, J = 4.9 Hz, 4H).

**Example 43: 4-(4-(benzoLdlthiazol-2-ylcarbamoyl)benzyl)-N-(2-(trifluoromethyl)phenyl)piperazine-1-carboxamide (compound 1-43)**

2-(Trifluoromethyl)phenyl isocyanate (47 mg, 0.25 mmol) was reacted in the same manner as in Example 37 to obtain 89 mg of the title compound.

$^1$H NMR (300 MHz, $CDCl_3$): δ 10.90 (s, 1H), 8.10 (d, J = 8.3 Hz, 1H), 7.97 (d, J = 8.2 Hz, 2H), 7.91 - 7.70 (m, 1H), 7.64 - 7.42 (m, 5H), 7.39 - 7.27 (m, 2H), 7.14 (t, J = 7.7 Hz, 1H), 6.79 (s, 1H), 3.61 (s, 2H), 3.52 (t, J = 5.1 Hz, 4H), 2.50 (t, J = 5.1 Hz, 4H).

**Example 44: 4-(4-(benzoLdlthiazol-2-ylcarbamoyl)benzyl)-N-(4-fluorophenyl) piperazine-1-carboxamide (compound 1-44)**

4-Fluorophenyl isocyanate (43 mg, 0.31 mmol) was reacted in the same manner as in Example 37 to obtain 106 mg of the title compound.

$^1$H NMR (500 MHz, $CDCl_3$): δ 10.74 (s, 1H), 7.96 (d, J = 8.2 Hz, 2H), 7.91 - 7.81 (m, 1H), 7.54 - 7.49 (m, 1H), 7.47 (d, J = 8.2 Hz, 2H), 7.37 - 7.27 (m, 4H), 6.98 (t, J = 8.7 Hz, 2H), 6.34 (s, 1H), 3.60 (s, 2H), 3.50 (t, J = 5.2 Hz, 4H), 2.48 (t, J = 5.0 Hz, 4H).

**Example 45: 4-(4-(benzo[d]thiazol-2-ylcarbamoyl)benzyl)-N-(2-fluorophenyl) piperazine-1-carboxamide (compound 1-45)**

2-Fluorophenyl isocyanate (43 mg, 0.31 mmol) was reacted in the same manner as in Example 37 to obtain 119 mg of the title compound.

$^1$H NMR (500 MHz, $CDCl_3$): δ 10.78 (s, 1H), 8.09 (td, J = 8.2, 1.6 Hz, 1H), 8.01 - 7.92 (m, 2H), 7.89 - 7.79 (m, 1H), 7.53 - 7.43 (m, 3H), 7.33 (pd, J = 7.2, 1.4 Hz, 2H), 7.17 - 7.01 (m, 2H), 6.96 (tdd, J = 8.2, 6.2, 1.7 Hz, 1H), 6.60 (d, J = 4.0 Hz, 1H), 3.61 (s, 2H), 3.53 (t, J = 5.0 Hz, 4H), 2.50 (t, J = 5.0 Hz, 4H).

**Example 46: 4-(4-(benzo[d]thiazol-2-ylcarbamoyl)benzyl)-N-(2,3-dihydro-1H-inden-5-yl)piperazine-1-carboxamide (compound 1-46)**

5-Indanyl isocyanate (50 mg, 0.31 mmol) was reacted in the same manner as in Example 37 to obtain 115 mg of the title compound.
$^1$H NMR (500 MHz, $CDCl_3$): δ 10.76 (s, 1H), 7.96 (d, J = 8.2 Hz, 2H), 7.88 - 7.79 (m, 1H), 7.52 (dd, J = 7.7, 1.4 Hz, 1H), 7.47 (d, J = 8.1 Hz, 2H), 7.38 - 7.27 (m, 4H), 7.11 (d, J = 8.0 Hz, 1H), 7.00 (dd, J = 8.0, 1.6 Hz, 2H), 6.33 (s, 1H), 3.59 (s, 2H), 3.53 - 3.34 (m, 4H), 2.86 (dt, J = 13.9, 7.4 Hz, 4H), 2.48 (t, J = 5.0 Hz, 4H), 2.05 (p, J = 7.4 Hz, 2H).

**Example 47: 4-(4-(benzo[d]thiazol-2-ylcarbamoyl)benzyl)-N-(3,5-dimethγphenyl)piperazine-1-carboxamide (compound 1-47)**

3,5-Dimethylphenyl isocyanate (46 mg, 0.31 mmol) was reacted in the same manner as in Example 37 to obtain 102 mg of the title compound.
$^1$H NMR (300 MHz, $CDCl_3$): δ 10.83 (s, 1H), 8.03 - 7.93 (m, 2H), 7.90 - 7.80 (m, 1H), 7.53 - 7.42 (m, 3H), 7.39 - 7.28 (m, 2H), 6.98 (s, 2H), 6.69 (s, 1H), 6.30 (s, 1H), 3.59 (s, 2H), 3.49 (t, J = 5.0 Hz, 4H), 2.47 (t, J = 5.0 Hz, 4H), 2.28 (s, 6H).

**Example 48: butyl-(4-(4-(benzo[d]thiazol-2-ylcarbamoyl)benzyl)piperazine-1-carbonyl)glycinate (compound 1-48)**

Butyl isocyanatoacetate (49 mg, 0.31 mmol) was reacted in the same manner as in Example 37 to obtain 80 mg of the title compound.
$^1$H NMR (300 MHz, $CDCl_3$): δ 10.65 (s, 1H), 7.96 (d, J = 7.9 Hz, 2H), 7.87 - 7.84 (m, 1H), 7.57 - 7.49 (m, 1H), 7.45 (d, J = 8.0 Hz, 2H), 7.40 - 7.27 (m, 2H), 5.00 (t, J = 5.3 Hz, 1H), 4.16 (t, J = 6.6 Hz, 2H), 4.03 (d, J = 5.0 Hz, 2H), 3.57 (s, 2H), 3.41 (t, J = 5.0 Hz, 4H), 2.42 (t, J = 5.0 Hz, 4H), 1.63 (dt, J = 14.5, 6.6 Hz, 3H), 1.38 (h, J = 7.3 Hz, 2H), 0.93 (t, J = 7.4 Hz, 3H).

**Example 49: 4-(4-(benzo[d]thiazol-2-ylcarbamoyl)benzyl)-N-(4-chlorophenyl) piperazine-1-carboxamide (compound 1-49)**

4-Chlorophenyl isocyanate (48 mg, 0.31 mmol) was reacted in the same manner as in Example 37 to obtain 59 mg of the title compound.
$^1$H NMR (300 MHz, $CDCl_3$): δ 7.96 (d, J = 8.2 Hz, 2H), 7.89 - 7.84 (m, 1H), 7.61 - 7.57 (m, 1H), 7.49 (d, J = 8.1 Hz, 2H), 7.42 - 7.22 (m, 6H), 6.35 (s, 1H), 3.61 (s, 2H), 3.51 (t, J = 5.0 Hz, 4H), 2.49 (t, J = 5.1 Hz, 4H).

**Example 50: 4-(4-(benzo[d]thiazol-2-ylcarbamoyl)benzyl)-N-(2-ethylphenyl) piperazine-1-carboxamide (compound 1-50)**

2-Ethylphenyl isocyanate (46 mg, 0.31 mmol) was reacted in the same manner as in Example 37 to obtain 107 mg of the title compound.
$^1$H NMR (300 MHz, $CDCl_3$): δ 10.79 (s, 1H), 7.97 (d, J = 7.9 Hz, 2H), 7.90 - 7.78 (m, 1H), 7.61 (d, J = 8.0 Hz, 1H), 7.47 (d, J = 8.3 Hz, 3H), 7.38 - 7.29 (m, 2H), 7.18 (d, J = 7.2 Hz, 2H), 7.09 - 7.04 (m, 1H), 6.23 (s, 1H), 3.60 (s, 2H), 3.50 (t, J = 4.9 Hz, 4H), 2.59 (q, J = 7.6 Hz, 2H), 2.49 (t, J = 5.0 Hz, 4H), 1.24 (t, J = 7.6 Hz, 3H).

**Example 51: 4-(4-(benzo[d]thiazol-2-ylcarbamoyl)benzyl)-N-(p-tolyl) piperazine-1-carboxamide (compound 1-51)**

p-Tolyl isocyanate (42 mg, 0.31 mmol) was reacted in the same manner as in Example 37 to obtain 76 mg of the title compound.
$^1$H NMR (300 MHz, $CDCl_3$): δ 10.59 (s, 1H), 7.96 (d, J = 8.2 Hz, 2H), 7.86 (dd, J = 7.5, 1.7 Hz, 1H), 7.56 (dd, J = 7.7, 1.6 Hz, 1H), 7.48 (d, J = 8.0 Hz, 2H), 7.34 (pd, J = 7.3, 1.5 Hz, 2H), 7.26 - 7.20 (m, 2H), 7.09 (d, J = 8.2 Hz, 2H), 6.31 (s, 1H), 3.60 (s, 2H), 3.49 (t, J = 5.0 Hz, 4H), 2.48 (t, J = 5.0 Hz, 4H), 2.30 (s, 3H).

**Example 52: 4-(4-(benzo[d]thiazol-2-ylcarbamoyl)benzyl)-N-(4-ethylphenyl) piperazine-1-carboxamide (compound 1-52)**

4-Ethylphenyl isocyanate (46 mg, 0.31 mmol) was reacted in the same manner as in Example 37 to obtain 46 mg of the title compound.

$^1$H NMR (300 MHz, $CDCl_3$): δ 10.68 (s, 1H), 7.96 (d, J = 8.1 Hz, 2H), 7.89 - 7.81 (m, 1H), 7.57 - 7.50 (m, 1H), 7.47 (d, J = 8.0 Hz, 2H), 7.41 - 7.28 (m, 2H), 7.25 (d, J = 8.2 Hz, 2H), 7.12 (d, J = 8.3 Hz, 2H), 6.32 (s, 1H), 3.60 (s, 2H), 3.50 (t, J = 5.0 Hz, 4H), 2.60 (q, J = 7.6 Hz, 2H), 2.48 (t, J = 5.0 Hz, 4H), 1.21 (t, J = 7.6 Hz, 3H).

**Example 53: 4-(4-(benzo[d]thiazol-2-ylcarbamoyl)benzyl)-N-cyclohexyl-piperazine-1-carboxamide (compound 1-53)**

Cyclohexyl isocyanate (39 mg, 0.31 mmol) was reacted in the same manner as in Example 37 to obtain 81 mg of the title compound.
$^1$H NMR (300 MHz, $CDCl_3$): δ 10.93 (s, 1H), 7.96 (d, J = 8.0 Hz, 2H), 7.89 - 7.79 (m, 1H), 7.49 - 7.39 (m, 3H), 7.36 - 7.27 (m, 2H), 4.25 (d, J = 7.6 Hz, 1H), 3.71 - 3.60 (m, 1H), 3.56 (s, 1H), 3.35 (t, J = 5.0 Hz, 4H), 2.41 (t, J = 5.0 Hz, 4H), 1.97 - 1.92 (m, 2H), 1.81 - 1.51 (m, 2H), 1.45 - 1.22 (m, 3H), 1.22 - 0.97 (m, 3H).

**Example 54: 4-(4-(benzo[d]thiazol-2-ylcarbamoyl)benzyl)-N-(chloromethyl) piperazine-1-carboxamide (compound 1-54)**

2-Chloroethyl isocyanate (33 mg, 0.31 mmol) was reacted in the same manner as in Example 37 to obtain 67 mg of the title compound.
$^1$H NMR (300 MHz, $CDCl_3$): δ 10.75 (s, 1H), 7.96 (d, J = 7.8 Hz, 2H), 7.91 - 7.77 (m, 1H), 7.51 - 7.41 (m, 3H), 7.40 - 7.26 (m, 2H), 5.02 - 4.65 (m, 1H), 3.69 - 3.63 (m, 1H), 3.61 - 3.58 (m, 2H), 3.39 (t, J = 5.1 Hz, 4H), 2.44 (t, J = 5.1 Hz, 4H).

**Example 55: 4-(4-(benzo[d]thiazol-2-ylcarbamoyl)benzyl)-N-propylpiperazine-1-carboxamide (compound 1-55)**

Propyl isocyanate (27 mg, 0.31 mmol) was reacted in the same manner as in Example 37 to obtain 64 mg of the title compound.
$^1$H NMR (300 MHz, $CDCl_3$): δ 10.89 (s, 1H), 8.00 - 7.90 (m, 2H), 7.90 - 7.80 (m, 1H), 7.51 - 7.41 (m, 3H), 7.37 - 7.28 (m, 2H), 4.43 (t, J = 5.6 Hz, 1H), 3.56 (s, 2H), 3.37 (t, J = 5.0 Hz, 4H), 3.21 (td, J = 7.1, 5.6 Hz, 2H), 2.42 (t, J = 5.0 Hz, 4H), 1.53 (h, J = 7.4 Hz, 2H), 0.92 (t, J = 7.4 Hz, 3H).

**Example 56: 4-(4-(benzo[d]thiazol-2-ylcarbamoyl)benzyl)-N-(2-ethoxyphenyl) piperazine-1-carboxamide (compound 1-56)**

2-Ethoxyphenyl isocyanate (51 mg, 0.31 mmol) was reacted in the same manner as in Example 37 to obtain 77 mg of the title compound.
$^1$H NMR (300 MHz, $CDCl_3$): δ 10.71 (s, 1H), 8.20 - 8.14 (m, 1H), 7.99 (d, J = 8.2 Hz, 2H), 7.93 - 7.84 (m, 1H), 7.60 - 7.46 (m, 3H), 7.43 - 7.30 (m, 2H), 7.22 (s, 1H), 6.98 - 6.93 (m, 2H), 6.88 - 6.84 (m, 1H), 4.12 (q, J = 7.0 Hz, 2H), 3.63 (s, 2H), 3.55 (t, J = 5.0 Hz, 4H), 2.52 (t, J = 5.0 Hz, 4H), 1.45 (t, J = 7.0 Hz, 3H).

**Example 57: 4-(4-(benzo[d]thiazol-2-ylcarbamoyl)benzyl)-N-(2,4,4-trimethyl-pentan-2-yl)piperazine-1-carboxamide (compound 1-57)**

1,1,3,3-Tetramethylbutyl isocyanate (48 mg, 0.31 mmol) was reacted in the same manner as in Example 37 to obtain 107 mg of the title compound.
$^1$H NMR (300 MHz, $CDCl_3$): δ 11.15 (s, 1H), 7.96 (d, J = 8.0 Hz, 2H), 7.89 - 7.82 (m, 1H), 7.43 - 7.37 (m, 3H), 7.34 - 7.27 (m, 2H), 4.32 (s, 1H), 3.55 (s, 2H), 3.31 (t, J = 5.0 Hz, 4H), 2.40 (t, J = 4.9 Hz, 4H), 1.73 (s, 2H), 1.41 (s, 6H), 1.01 (s, 9H).

**Example 58: 4-(4-(benzo[d]thiazol-2-ylcarbamoyl)benzyl)-N-(2-bromophenyl) piperazine-1-carboxamide (compound 1-58)**

2-Bromophenyl isocyanate (62 mg, 0.31 mmol) was reacted in the same manner as in Example 37 to obtain 94 mg of the title compound.
$^1$H NMR (300 MHz, $CDCl_3$): δ 10.66 (s, 1H), 8.19 (dd, J = 8.3, 1.6 Hz, 1H), 8.01 - 7.93 (m, 2H), 7.91 - 7.82 (m, 1H), 7.54 - 7.47 (m, 4H), 7.40 - 7.27 (m, 3H), 7.03 (s, 1H), 6.89 (ddd, J = 8.0, 7.3, 1.6 Hz, 1H), 3.62 (s, 2H), 3.56 (t, J = 5.0 Hz, 4H), 2.52 (t, J = 5.0 Hz, 4H).

**Example 59: N-(benzo[dlthiazol-2-yl)-4-((4-propionylpiperazin-1-yl)methyl) benzamide (compound 1-59)**

Acetyl chloride (22 uL, 0.31 mmol) was reacted in the same manner as in Example 37 to obtain 74 mg of the title compound.

$^1H$ NMR (500 MHz, $CDCl_3$): δ 10.56 (s, 1H), 7.90 (t, J = 8.1 Hz, 4H), 7.88 - 7.82 (m, 1H), 7.56 - 7.50 (m, 1H), 7.39 - 7.27 (m, 2H), 3.56 (s, 2H), 3.45 (qd, J = 7.1, 5.2 Hz, 2H), 3.07 (s, 4H), 2.51 (t, J = 5.0 Hz, 4H), 0.99 (t, J= 7.1 Hz, 3H).

**Example 60: N-(benzo[dlthiazol-2-yl)-4-((4-benzoylpiperazin-1-yl)methyl) benzamide (compound 1-60)**

Benzoyl chloride (36 μL, 0.31 mmol) was reacted in the same manner as in Example 37 to obtain 80 mg of the title compound.

$^1H$ NMR (500 MHz, DMSO): δ 12.80 (s, 1H), 8.50 (s, 1H), 8.14 (d, J = 8.4 Hz, 2H), 8.03 (dd, J = 7.9, 1.1 Hz, 1H), 7.80 (d, J = 8.0 Hz, 1H), 7.53 (d, J = 8.3 Hz, 2H), 7.52 - 7.42 (m, 3H), 7.35 (td, J = 7.6, 1.1 Hz, 1H), 7.26 - 7.18 (m, 2H), 6.93 (tt, J = 7.4, 1.2 Hz, 1H), 3.63 (s, 2H), 3.51 (t, J = 4.9 Hz, 4H), 2.47 (t, J = 5.0 Hz, 4H).

**Example 61: N-(benzo[dlthiazol-2-yl)-4-((4-ethylpiperazin-1-yl)methyl) benzamide (compound 1-61)**

N-(Benzo[d]thiazol-2-yl)-4-((4-(ethylsulfonyl)piperazin-1-yl)methyl)benzamide (100 mg, 0.28 mmol) obtained in Example 31 was dissolved in anhydrous DMF (3 mL), potassium carbonate (79 mg, 0.57 mmol) and bromoethane (23 μL, 0.31 mmol) were added, the mixture was stirred at room temperature for 12 hours. The reaction product was diluted with EtOAc and washed with purified water. The separated organic layer was dehydrated with anhydrous $Na_2SO_4$, concentrated under reduced pressure, and separated by column chromatography to obtain 54 mg of the title compound.

$^1H$ NMR (500 MHz, $CDCl_3$): δ 10.50 (s, 1H), 7.91 (t, J = 8.1 Hz, 4H), 7.82 (m, 1H), 7.50 (m, 1H), 7.37 (m, 2H), 3.56 (s, 2H), 3.45 (qd, J = 7.1, 5.2 Hz, 2H), 3.07 (s, 4H), 2.50 (t, J = 5.0 Hz, 4H), 0.94 (t, J= 7.1 Hz, 3H).

**Example 62: N-(benzo[dlthiazol-2-yl)-4-((4-benzylpiperazin-1-yl)methyl) benzamide (compound 1-62)**

Benzyl bromide (37 μL, 0.31 mmol) was reacted in the same manner as in Example 61 to obtain 79 mg of the title compound.

$^1H$ NMR (500 MHz, DMSO): δ 12.80 (s, 1H), 8.50 (s, 1H), 8.14 (d, J = 8.4 Hz, 2H), 8.03 (dd, J = 7.9, 1.1 Hz, 1H), 7.80 (d, J = 8.0 Hz, 1H), 7.53 (d, J = 8.3 Hz, 2H), 7.52 - 7.42 (m, 3H), 7.35 (td, J = 7.6, 1.1 Hz, 1H), 7.26 - 7.18 (m, 2H), 6.93 (tt, J = 7.4, 1.2 Hz, 1H), 3.66 (s, 2H), 3.63 (s, 2H), 3.50 (t, J = 4.9 Hz, 4H), 2.46 (t, J = 5.0 Hz, 4H).

**Example 63: tert-butyl 4-(4-(benzo[d]thiazol-2-ylcarbamoyl)benzylidene) piperidine-1-carboxylate (compound 1-63)**

Put compound 5 (247 mg, 0.74 mmol), tert-butyl 4-((4,4,5,5-tetramethyl-1,3,2-dioxaborolan-2-yl)methylene)piperidine-1-carboxylate (200 mg, 0.62 mmol), $Pd(PPh_3)_4$ (71.50 mg, 0.06 mmol), potassium carbonate (213.79 mg, 1.55 mmol), $Pd(PPh_3)_4$ (71.50 mg, 0.06 mmol) in a reaction vessel and add 1,4-dioxane (10 mL) and $H_2O$ (2 mL). The reaction was stirred at 95° C. for 12 hours and the reaction product was diluted with EtOAc and washed with purified water. The separated organic layer was dehydrated with anhydrous $Na_2SO_4$, concentrated under reduced pressure, and separated by column chromatography to obtain 172 mg of the title compound.

$^1H$ NMR (400 MHz, $CDCl_3$): δ 11.16 (s, 1H), 7.96 - 7.93 (m, 2H), 7.88 - 7.83 (m, 1H), 7.43 - 7.39 (m, 1H), 7.32 - 7.28 (m, 2H), 7.27 - 7.25 (m, 2H), 6.36 (s, 1H), 3.52 (t, J = 5.9 Hz, 3H), 3.40 (t, J = 5.9 Hz, 3H), 2.41 - 2.34 (m, 4H), 1.49 (s, 9H).

**Example 64: tert-butyl 4-(4-(benzo[d]thiazol-2-ylcarbamoyl)benzyl) piperidine-1-carboxylate (compound 1-64)**

Tert-butyl 4-(4-(benzo[d]thiazol-2-ylcarbamoyl)benzylidene)piperidine-1-carboxylate (160 mg, 0.36 mmol) obtained in Example 63 was dissolved in anhydrous MeOH (3 mL) and anhydrous THF (3 mL), and 10 wt% Pd/C was added thereto. The reaction mixture was reacted for 12 hours at room temperature under hydrogen gas conditions, and palladium was removed through a Celite filter. The filter solution was concentrated under reduced pressure and separated by column chromatography to obtain 147 mg of the title compound.

$^1H$ NMR (500 MHz, $CDCl_3$): δ 11.01 (s, 1H), 7.93 (d, J = 8.0 Hz, 2H), 7.86 - 7.85 (m, 1H), 7.47 - 7.45 (m, 1H), 7.33 - 7.29 (m, 2H), 7.24 (d, J = 7.9 Hz, 2H), 2.64 - 2.58 (m, 4H), 1.70 - 1.66 (m, 3H), 1.57 (d, J = 12.6 Hz, 2H), 1.14 (q, J = 11.5 Hz, 2H).

**Example 65: N-(benzo[d]thiazol-2-yl)-4-(piperidin-4-ylidenemethyl)benzamide (compound 1-65)**

Tert-butyl 4-(4-(benzo[d]thiazol-2-ylcarbamoyl)benzylidene)piperidine-1-carboxylate (110 mg, 0.25 mmol) obtained in Example 63 was dissolved in anhydrous DCM (5 mL), and TFA (2 mL) was added and stirred at room temperature for 2 hours. After the reaction was completed, the solvent was concentrated under reduced pressure and washed twice with ether to obtain 120 mg of the title compound.

$^1$H NMR (500 MHz, DMSO): δ 12.91 (s, 1H), 8.71 (s, 2H), 8.15 (d, J = 8.3 Hz, 2H), 8.04 (d, J = 7.9 Hz, 1H), 7.80 (d, J = 8.1 Hz, 1H), 7.50 - 7.45 (m, 3H), 7.38 - 7.34 (m, 1H), 6.57 (s, 1H), 3.21 - 3.20 (m, 2H), 3.15 - 3.14 (m, 2H), 2.67 (t, J = 6.1 Hz, 2H), 2.57 (t, J = 6.1 Hz, 2H).

**Example 66: N-(benzo[d]thiazol-2-yl)-4-(piperidin-4-ylmethyl)benzamide (compound 1-66)**

Tert-butyl 4-(4-(benzo[d]thiazol-2-ylcarbamoyl)benzyl)piperidine-1-carboxylate (110 mg, 0.25 mmol) obtained in Example 64 was reacted in the same manner as in Example 65 to obtain 112 mg of the title compound.

$^1$H NMR (500 MHz, DMSO): δ 12.85 (s, 1H), 8.12 (d, J = 8.0 Hz, 2H), 8.03 (d, J = 7.9 Hz, 1H), 7.80 (d, J = 8.2 Hz, 1H), 7.48 (t, J = 7.7 Hz, 1H), 7.41 (d, J = 8.0 Hz, 2H), 7.36 (t, J = 7.6 Hz, 1H), 3.26 (d, J = 12.5 Hz, 2H), 2.83 (q, J = 11.8 Hz, 2H), 2.66 (d, J = 7.1 Hz, 2H), 1.92 - 1.86 (m, 1H), 1.73 (d, J = 13.9 Hz, 2H), 1.39 - 1.30 (m, 2H).

**Example 67: 4-(4-(benzo[d]thiazol-2-ylcarbamoyl)benzylidene)-N-ethylpiperidine-1-carboxamide (compound 1-67)**

N-(Benzo[d]thiazol-2-yl)-4-(piperidin-4-ylidenemethyl)benzamide (75 mg, 0.17 mmol) and ethyl isocyanate (16 pL, 0.20 mmol) obtained in Example 65 were reacted in the same manner as in Example 37 to obtain 45 mg of the title compound.

$^1$H NMR (400 MHz, $CDCl_3$): δ 11.20 (s, 1H), 7.97 - 7.95 (m, 2H), 7.88 - 7.83 (m, 1H), 7.43 - 7.39 (m, 1H), 7.32 - 7.28 (m, 2H), 7.27 - 7.25 (m, 2H), 6.37 (s, 1H), 4.44 (t, J = 5.4 Hz, 1H), 3.48 (t, J = 5.8 Hz, 2H), 3.38 (t, J = 5.8 Hz, 2H), 3.30 (qd, J = 7.2, 5.3 Hz, 2H), 2.46 - 2.39 (m, 4H), 1.16 (t, J = 7.2 Hz, 3H).

**Example 68: 4-(4-(benzo[d]thiazol-2-ylcarbamoyl)benzylidene)-N-phenylpiperidine-1-carboxamide (compound 1-68)**

Phenyl isocyanate (22 pL, 0.20 mmol) was reacted in the same manner as in Example 67 to obtain 51 mg of the title compound.

$^1$H NMR (400 MHz, $CDCl_3$): δ 10.91 (s, 1H), 7.98 - 7.96 (m, 2H), 7.87 - 7.85 (m, 1H), 7.53 - 7.41 (m, 1H), 7.38 - 7.27 (m, 8H), 7.05 (tt, J = 7.0, 1.3 Hz, 1H), 6.52 (s, 1H), 6.42 (s, 1H), 3.61 (t, J = 5.9 Hz, 2H), 3.52 (t, J = 5.9 Hz, 2H), 2.56 - 2.53 (m, 2H), 2.51 - 2.47 (m, 2H).

**Example 69: 4-(4-(benzo[d]thiazol-2-ylcarbamoyl)benzyl)-N-ethylpiperidine-1-carboxamide (compound 1-69)**

N-(Benzo[d]thiazol-2-yl)-4-(piperidin-4-ylmethyl)benzamide (75 mg, 0.17 mmol) and ethyl isocyanate (16 μL, 0.20 mmol) obtained in Example 66 were reacted in the same manner as in Example 37 to obtain 40 mg of the title compound.

$^1$H NMR (400 MHz, $CDCl_3$): δ 11.03 (s, 1H), 7.93 (d, J = 8.3 Hz, 2H), 7.87 - 7.83 (m, 1H), 7.48 - 7.44 (m, 1H), 7.33 - 7.28 (m, 2H), 7.23 (d, J = 8.2 Hz, 2H), 4.38 (t, J = 5.4 Hz, 1H), 3.91 (dt, J = 13.0, 2.9 Hz, 2H), 3.27 (qd, J = 7.2, 5.3 Hz, 2H), 2.70 (td, J = 12.9, 2.6 Hz, 2H), 2.59 (d, J = 7.1 Hz, 2H), 1.72 - 1.66 (m, 1H), 1.63 - 1.58 (m, 2H), 1.20 - 1.12 (m, 5H).

**Example 70: 4-(4-(benzo[d]thiazol-2-ylcarbamoyl)benzyl)-N-ethylpiperidine-1-carboxamide (compound 1-70)**

Phenyl isocyanate (22 pL, 0.20 mmol) was reacted in the same manner as in Example 69 to obtain 51 mg of the title compound.

$^1$H NMR (400 MHz, DMSO): δ 12.80 (s, 1H), 8.43 (s, 1H), 8.10 (d, J = 8.0 Hz, 2H), 8.02 (d, J = 7.9 Hz, 1H), 7.79 (d, J = 8.1 Hz, 1H), 7.49 - 7.44 (m, 3H), 7.40 (d, J = 7.9 Hz, 2H), 7.34 (t, J = 7.6 Hz, 1H), 7.21 (t, J = 7.9 Hz, 2H), 6.91 (t, J = 7.3 Hz, 1H), 4.11 (d, J = 13.2 Hz, 2H), 2.77 - 2.70 (m, 2H), 2.65 (d, J = 7.2 Hz, 2H), 1.86 - 1.77 (m, 1H), 1.62 - 1.58 (m, 2H), 1.20 - 1.10 (m, 2H).

**Example 71: 4-(((1S,4S)-2,5-diazabicyclo[2.2.1]heptan-2-yl)methyl)-N-(benzo[d]thiazol-2-yl)benzamide (compound 1-71)**

Tert-butyl (1S,4S)-5-(4-(benzo[d]thiazol-2-ylcarbamoyl)benzyl)-2,5-diaza-bicyclo[2.2.1]heptane-2-carboxylate (300 mg, 0.65 mmol) obtained in Example 25 was reacted in the same manner as in Example 3 to obtain 240 mg of the title compound.

$^1$H NMR (400 MHz, $CDCl_3$): δ 9.49 (s, 1H), 9.27 (s, 1H), 8.23 (d, J = 8.3 Hz, 2H), 8.04 (dd, J = 8.0, 1.2 Hz, 1H), 7.80 (d, J = 8.0 Hz, 1H), 7.72 (d, J = 7.9 Hz, 2H), 7.51 - 7.47 (m, 1H), 7.38 - 7.34 (m, 1H), 4.48 (s, 1H), 4.40 - 4.33 (m, 2H), 3.63 - 3.38 (m, 4H).

**Example 72: (1S,4S)-5-(4-(benzo[d]thiazol-2-ylcarbamoyl)benzyl)-N-ethyl-2,5-diazabicyclo[2.2.1]heptane-2-carboxamide (compound 1-72)**

4-(((1S,4S)-2,5-Diazabicyclo[2.2.1]heptan-2-yl)methyl)-N-(benzo[d]thiazol-2-yl)benzamide (200 mg, 0.43 mmol) and ethyl isocyanate (41 μL, 0.52 mmol) obtained in Example 71 were reacted in the same manner as in Example 37 to obtain 118 mg of the title compound.

$^1$H NMR (400 MHz, $CDCl_3$): δ 10.84 (s, 1H), 7.96 - 7.94 (m, 2H), 7.87 - 7.84 (m, 1H), 7.52 - 7.49 (m, 1H), 7.46 - 7.44 (m, 2H), 7.37 - 7.28 (m, 2H), 4.44 (s, 1H), 4.12 - 4.09 (m, 1H), 3.78 (s, 2H), 3.48 - 3.45 (m, 2H), 3.33 - 3.26 (m, 2H), 3.18 (dt, J = 8.6, 1.9 Hz, 1H), 2.86 (dt, J = 9.6, 1.9 Hz, 1H), 2.69 (d, J = 9.6 Hz, 1H), 1.89 (d, J = 9.7 Hz, 1H), 1.74 - 1.71 (m, 1H), 1.18 - 1.14 (m, 3H).

**Example 73: (1S,4S)-5-(4-(benzo[d]thiazol-2-ylcarbamoyl)benzyl)-N-phenyl-2,5-diazabicyclo[2.2.1]heptane-2-carboxamide (compound 1-73)**

Phenyl isocyanate (56 μL, 0.52 mmol) was reacted in the same manner as in Example 72 to obtain 114 mg of the title compound.

$^1$H NMR (400 MHz, $CDCl_3$): δ 11.03 (s, 1H), 7.96 - 7.94 (m, 2H), 7.86 - 7.83 (m, 1H), 7.47 - 7.40 (m, 5H), 7.34 - 7.27 (m, 4H), 7.03 (tt, J = 7.2, 1.2 Hz, 1H), 6.19 (s, 1H), 4.57 (s, 1H), 3.80 (s, 2H), 3.61 (d, J = 8.4 Hz, 1H), 3.54 (s, 1H), 3.33 (dd, J = 8.5, 2.2 Hz, 1H), 2.88 (dd, J = 9.7, 2.1 Hz, 1H), 2.79 - 2.77 (m, 1H), 1.97 - 1.94 (m, 1H), 1.79 - 1.77 (m, 1H).

**Example 74: tert-butyl 4-(4-(benzo[d]thiazol-2-ylcarbamoyl)phenyl)-3,6-dihydropyridine-1-(2H)-carboxylate (compound 1-74)**

Compound 5 (1.00 g, 3.33 mmol) and (1-(tert-butoxycarbonyl)-1,2,3,6-tetrahydropyridin-4-yl)boronic acid (1.00 g, 4.44 mmol) were reacted in the same manner as in Example 63 to obtain 950 mg of the title compound.

$^1$H NMR (500 MHz, $CDCl_3$): δ 11.12 (s, 1H), 7.95 (d, J = 8.2 Hz, 2H), 7.88 - 7.82 (m, 1H), 7.42 (d, J = 8.0 Hz, 2H), 7.43 - 7.37 (m, 1H), 7.33 - 7.27 (m, 2H), 5.92 (1H, s), 3.83 (2H, m), 3.34 (2H, t, J = 5.6 Hz), 2.15 (2H, s), 1.43 (9H, s).

**Example 75: N-(benzo[d]thiazol-2-yl)-4-(1,2,3,6-tetrahydropyridin-4-yl) benzamide (compound 1-75)**

Tert-butyl 4-(4-(benzo[d]thiazol-2-ylcarbamoyl)phenyl)-3,6-dihydropyridine-1-(2H)-carboxylate (200 mg, 0.46 mmol) obtained in Example 74 was reacted in the same manner as in Example 3 to obtain 189 mg of the title compound.

$^1$H NMR (500 MHz, $CDCl_3$): δ 11.14 (s, 1H), 7.97 (d, J = 8.2 Hz, 2H), 7.82 (m, 1H), 7.42 (d, J = 8.0 Hz, 2H), 7.43 (m, 1H), 7.37 (m, 2H), 5.90 (1H, s), 3.97 (2H, m), 3.34 (2H, t, J = 5.6 Hz), 2.45 (2H, s).

**Example 76: 4-(4-(benzo[d]thiazol-2-ylcarbamoyl)phenyl)-N-(4-trifluoro-methyl)phenyl)-3,6-dihydropyridine-1-(2H)-carboxylate (compound 1-76)**

N-(Benzo[d]thiazo1-2-yl)-4-(1,2,3,6-tetrahydropyridin-4-yl)benzamide (150 mg, 0.45 mmol) obtained in Example 75 was reacted in the same manner as in Example 39 to obtain 189 mg of the title compound.

$^1$H NMR (500 MHz, $CDCl_3$): δ 11.12 (s, 1H), 8.23 (d, J = 7.7 Hz, 2H), 8.03 (d, J= 7.7 Hz, 2H), 7.95 (d, J = 8.2 Hz, 2H), 7.82 (m, 1H), 7.42 (d, J = 8.0 Hz, 2H), 7.37 (m, 1H), 7.27 (m, 2H), 5.92 (1H, s), 3.83 (2H, m), 3.34 (2H, t, J = 5.6 Hz), 2.15 (2H, s).

**Example 77: 4-(4-(benzo[d]thiazol-2-ylcarbamoyl)phenyl)-N-(4-trifluoromethyl)phenyl)piperidine-1-carboxamide (compound 1-77)**

4-(4-(Benzo[d]thiazol-2-ylcarbamoyl)phenyl)-N-(4-trifluoromethyl)phenyl)-3,6-dihydropyridine-1-(2H)-carboxylate (100 mg, 0.19 mmol) obtained in Example 76 was reacted in the same manner as in Example 64 to obtain 69 mg of the title compound.

$^1$H NMR (500 MHz, $CDCl_3$): δ 11.14 (s, 1H), 8.23 (d, J = 7.7 Hz, 2H), 8.03 (d, J= 7.7 Hz, 2H), 7.96 (d, J = 8.2 Hz, 2H), 7.91 - 7.81 (m, 1H), 7.54 - 7.49 (m, 1H), 7.47 (d, J = 8.2 Hz, 2H), 7.37 - 7.27 (m, 4H), 6.98 (t, J = 8.7 Hz, 2H), 6.34 (s, 1H), 2.73 (m, 1H), 1.89 (m, 2H), 1.64 (m, 2H).

**Example 78: tert-butyl 4-(4-(benzo[d]thiazol-2-ylcarbamoyl)-3-(trifluoromethyl)benzylidene)piperidine-1-carboxylate (compound 1-78)**

Compound 6 (744 mg, 1.86 mmol), tert-butyl 4-((4,4,5,5-tetramethyl-1,3,2-dioxaborolan-2-yl)methylene)piperidine-1-carboxylate (500 mg, 1.55 mmol) obtained in Synthesis Example 6 was reacted in the same manner as in Example 63 to obtain 300 mg of the title compound.

$^1$H NMR (300 MHz, DMSO-$d_6$) δ 13.02 (s, 1H), 8.09 - 7.99 (m, 1H), 7.80 (d, J = 8.2 Hz, 3H), 7.67 (d, J = 7.2 Hz, 2H), 7.54 - 7.42 (m, 2H), 7.42 - 7.30 (m, 2H), 6.53 (s, 1H), 3.46 (t, J = 5.9 Hz, 2H), 3.39 (t, J = 5.9 Hz, 3H), 2.44 (t, J = 5.9 Hz, 2H), 2.35 (t, J = 5.9 Hz, 2H), 1.43 (s, 9H).

**Example 79: N-(benzo[d]thiazol-2-yl)-4-(piperidin-4-ylidenemethyl)-2-(trifluoromethyl)benzamide (compound 1-79)**

Tert-butyl 4-(4-(benzo[d]thiazol-2-ylcarbamoyl)-3-(trifluoromethyl) benzylidene)piperidine-1-carboxylate (110 mg, 0.21 mmol) obtained in Example 78 was reacted in the same manner as in Example 3 to obtain 105 mg of the title compound.

$^1$H NMR (300 MHz, DMSO-$d_6$) δ 13.05 (s, 1H), 8.82 (s, 2H), 8.04 (d, J = 7.8 Hz, 1H), 7.81 (t, J = 7.7 Hz, 2H), 7.70 (d, J = 6.4 Hz, 2H), 7.55 - 7.42 (m, 1H), 7.43 - 7.31 (m, 1H), 6.62 (s, 1H), 3.19 (dt, J = 17.0, 5.9 Hz, 4H), 2.61 (dt, J = 16.2, 5.9 Hz, 4H).

**Example 80: 4-(4-(benzo[d]thiazol-2-ylcarbamoyl)-3-(trifluoromethyl) benzylidene)-N-ethylpiperidine-1-carboxamide (compound 1-80)**

N-(Benzo[d]thiazol-2-yl)-4-(piperidin-4-ylidenemethyl)-2-(trifluoromethyl) benzamide (45 mg, 0.09 mmol) and ethyl isocyanate (6.83 mg, 0.10 mmol) obtained in Example 79 were reacted in the same manner as in Example 37 to obtain 27 mg of the title compound.

$^1$H NMR (300 MHz, DMSO-$d_6$) δ 13.00 (s, 1H), 8.09 - 7.99 (m, 1H), 7.80 (d, J = 8.1 Hz, 2H), 7.67 (d, J = 7.1 Hz, 2H), 7.53 - 7.43 (m, 1H), 7.42 - 7.32 (m, 1H), 6.60 - 6.47 (m, 2H), 3.46 - 3.34 (m, 3H), 3.13 - 3.01 (m, 2H), 2.37 (dt, J = 24.5, 5.7 Hz, 4H), 1.03 (t, J = 7.1 Hz, 3H).

**Example 81: 4-(4-(benzo[d]thiazol-2-ylcarbamoyl)-3-(trifluoromethyl) benzylidene)-N-phenylpiperidine-1-carboxamide (compound 1-81)**

N-(Benzo[d]thiazol-2-yl)-4-(piperidin-4-ylidenemethyl)-2-(trifluoromethyl) benzamide (45 mg, 0.09 mmol) and phenyl isocyanate (11.18 mg, 0.10 mmol) obtained in Example 79 were reacted in the same manner as in Example 37 to obtain 33 mg of the title compound.

$^1$H NMR (300 MHz, DMSO-$d_6$) δ 12.99 (s, 1H), 8.59 (s, 1H), 8.04 (d, J = 7.9 Hz, 1H), 7.81 (dd, J = 8.1, 4.3 Hz, 2H), 7.69 (d, J = 7.2 Hz, 2H), 7.54 - 7.42 (m, 3H), 7.42 - 7.30 (m, 1H), 7.31 - 7.18 (m, 2H), 7.00 - 6.88 (m, 1H), 6.56 (s, 1H), 3.56 (dt, J = 22.4, 5.8 Hz, 4H), 2.43 (t, J = 5.7 Hz, 2H).

**Example 82: tert-butyl 4-(4-(benzo[d]thiazol-2-ylcarbamoyl)-3-(trifluoromethyl)benzyl)piperidine-1-carboxylate (compound 1-82)**

Tert-butyl 4-(4-(benzo[d]thiazol-2-ylcarbamoyl)-3-(trifluoromethyl) benzylidene)piperidine-1-carboxylate (150 mg, 0.29 mmol) obtained in Example 78 was reacted in the same manner as in Example 64 to obtain 87 mg of the title compound.

$^1$H NMR (300 MHz, Chloroform-d) δ 11.79 (s, 1H), 7.84 - 7.77 (m, 1H), 7.56 (d, J = 7.8 Hz, 1H), 7.49 (s, 1H), 7.31 - 7.27 (m, 1H), 7.26 - 7.23 (m, 1H), 7.19 (td, J = 7.7, 7.2, 1.4 Hz, 1H), 7.08 (d, 1H), 4.07 (s, 2H), 2.69 - 2.51 (m, 4H), 1.70 - 1.43 (m, 1311), 1.20 - 1.02 (m, 2H).

**Example 83: N-(benzo[d]thiazol-2-yl)-4-(piperidin-4-ylmethyl)-2-(trifluoromethyl)benzamide (compound 1-83)**

Tert-butyl 4-(4-(benzo[d]thiazol-2-ylcarbamoyl)-3-(trifluoromethyl) benzyl)piperidine-1-carboxylate (75 mg, 0.14 mmol) obtained in Example 82 was reacted in the same manner as in Example 79 to obtain 83 mg of the title compound.

$^1$H NMR (300 MHz, DMSO-$d_6$) $\delta$ 13.02 (s, 1H), 8.58 (d, J = 11.6 Hz, 1H), 8.27 (d, J = 11.9 Hz, 1H), 8.04 (d, J = 7.9, 1.2 Hz, 1H), 7.84 - 7.70 (m, 3H), 7.71 - 7.59 (m, 1H), 7.53 - 7.42 (m, 1H), 7.42 - 7.31 (m, 1H), 3.27 (d, J = 12.3 Hz, 2H), 2.93 - 2.69 (m, 4H), 1.97 - 1.81 (m, 1H), 1.72 (d, J = 13.8 Hz, 2H), 1.45 - 1.26 (m, 2H).

**Example 84: 4-(4-(benzo[d]thiazol-2-ylcarbamoyl)-3-(trifluoromethyl)benzyl) -N-ethylpiperidine-1-carboxamide (compound 1-84)**

N-(Benzo[cl]thiazol-2-yl)-4-(piperidin-4-ylmethyl)-2-(trifluoromethyl)benzamide (41 mg, 0.10 mmol) and ethyl isocyanate (7.6 mg, 0.11 mmol) obtained in Example 83 were reacted in the same manner as in Example 37 to obtain 21 mg of the title compound.

$^1$H NMR (300 MHz, DMSO-$d_6$) $\delta$ 12.98 (s, 1H), 8.04 (d, 1H), 7.84 - 7.67 (m, 3H), 7.62 (d, J = 7.5 Hz, 1H), 7.52 - 7.42 (m, 1H), 7.41 - 7.31 (m, 1H), 6.40 (t, J = 5.4 Hz, 1H), 3.93 (d, J = 13.0 Hz, 2H), 3.10 - 2.95 (m, 2H), 2.69 (d, J = 7.0 Hz, 2H), 2.58 (t, J = 12.2 Hz, 2H), 1.82 - 1.65 (m, 1H), 1.51 (d, J = 12.7 Hz, 2H), 1.15 - 0.94 (m, 5H).

**Example 85: 4-(4-(benzo[d]thiazol-2-ylcarbamoyl)-3-(trifluoromethyl)benzyl) -N-phenylpiperidine-1-carboxamide (compound 1-85)**

N-(Benzo[d]thiazol-2-yl)-4-(piperidin-4-ylmethyl)-2-(trifluoromethyl) benzamide (41 mg, 0.10 mmol) and phenyl isocyanate (13 mg, 0.11 mmol) obtained in Example 83 were reacted in the same manner as in Example 37 to obtain 25 mg of the title compound.

$^1$H NMR (300 MHz, DMSO-$d_6$) $\delta$ 12.98 (s, 1H), 8.45 (s, 1H), 8.03 (d, J = 8.0, 1.2 Hz, 1H), 7.83 - 7.69 (m, 3H), 7.68 - 7.59 (m, 1H), 7.53 - 7.40 (m, 3H), 7.35 (td, J = 7.6, 1.2 Hz, 1H), 7.27 - 7.16 (m, 2H), 6.97 - 6.86 (m, 1H), 4.12 (d, J = 13.3 Hz, 2H), 2.86 - 2.64 (m, 4H), 1.89 - 1.72 (m, 1H), 1.59 (d, J = 12.6 Hz, 2H), 1.26 - 1.07 (m, 2H).

**Example 86: tert-butyl 4-(4-(benzo[d]thiazol-2-ylcarbamoyl)-3-fluorobenzylidene)piperidine-1-carboxylate (compound 1-86)**

Compound 7 (2.99 g, 8.51 mmol) and tert-butyl 4-((4,4,5,5-tetramethyl-1,3,2-dioxaborolan-2-yl)methylene)piperidine-1-carboxylate (2.5 g, 7.73 mmol) were reacted in the same manner as in Example 63 to obtain 1.18 g of the title compound.

$^1$H NMR (300 MHz, DMSO-$d_6$) $\delta$ 12.75 (s, 1H), 8.09 - 7.99 (m, 1H), 7.85 - 7.72 (m, 2H), 7.54 - 7.42 (m, 1H), 7.42 - 7.30 (m, 1H), 7.30 - 7.18 (m, 2H), 6.44 (s, 1H), 3.42 (dt, J = 17.0, 6.0 Hz, 4H), 2.46 (t, J = 5.7 Hz, 4H), 2.33 (t, J = 5.5 Hz, 2H), 1.43 (s, 9H).

**Example 87: N-(benzo[d]thiazol-2-yl)-2-fluoro-4-(piperidin-4-ylidenemethyl) benzamide (compound 1-87)**

Tert-butyl 4-(4-(benzo[d]thiazol-2-ylcarbamoyl)-3-fluorobenzylidene) piperidine-1-carboxylate (230 mg, 0.49 mmol) obtained in Example 86 was reacted in the same manner as in Example 79 to obtain 321 mg of the title compound.

$^1$H NMR (300 MHz, DMSO-$d_6$) $\delta$ 8.81 (s, 2H), 8.09 - 7.99 (m, 1H), 7.87 - 7.74 (m, 2H), 7.54 - 7.42 (m, 1H), 7.42 - 7.20 (m, 3H), 6.53 (s, 1H), 3.30 - 3.10 (m, 4H), 2.66 (t, J = 6.0 Hz, 2H), 2.56 (t, J = 5.9 Hz, 3H).

**Example 88: 4-(4-(benzo[d]thiazol-2-ylcarbamoyl)-3-fluorobenzylidene)-N-ethylpiperidine-1-carboxamide (compound 1-88)**

N-(Benzo[d]thiazol-2-yl)-2-fluoro-4-(piperidin-4-ylidenemethyl)benzamide (74 mg, 0.40 mmol) and ethyl isocyanate (31 mg, 0.44 mmol) obtained in Example 87 were reacted in the same manner as in Example 63 to obtain 71 mg of the title compound.

$^1$H NMR (300 MHz, DMSO-$d_6$) $\delta$ 12.74 (s, 1H), 8.09 - 7.99 (m, 1H), 7.84 - 7.72 (m, 2H), 7.54 - 7.42 (m, 1H), 7.42 - 7.30 (m, 1H), 7.30 - 7.18 (m, 2H), 6.53 (t, J = 5.4 Hz, 1H), 6.42 (s, 1H), 3.47 - 3.34 (m, 4H), 3.15 - 2.99 (m, 2H), 2.43 (t, J = 5.8 Hz, 2H), 2.31 (t, J = 5.7 Hz, 2H), 1.03 (t, J = 7.1 Hz, 3H).

**Example 89: 4-(4-(benzo[d]thiazol-2-ylcarbamoyl)-3-fluorobenzylidene)-N-phenylpiperidine-1-carboxamide (compound 1-89)**

N-CBenzo[d]thiazol-2-yl)-2-fluoro-4-(piperidin-4-ylidenemethyl)benzamide (146 mg, 0.40 mmol) and phenyl isocyanate (52 mg, 0.44 mmol) obtained in Example 87 were reacted in the same manner as in Example 63 to obtain 71 mg of the title compound.

$^1$H NMR (300 MHz, DMSO-$d_6$) δ 12.76 (s, 1H), 8.58 (s, 1H), 8.10 - 7.99 (m, 1H), 7.87 - 7.73 (m, 2H), 7.57 - 7.42 (m, 3H), 7.42 - 7.30 (m, 1H), 7.33 - 7.17 (m, 4H), 7.01 - 6.88 (m, 1H), 6.47 (s, 1H), 3.56 (dt, J = 19.5, 5.6 Hz, 4H), 3.18 (d, J = 5.2 Hz, 1H), 2.55 - 2.52 (m, 2H), 2.42 (t, J = 5.7 Hz, 2H).

**Example 90: N-(benzo[d]thiazol-2-yl)-2-fluoro-4-((1-(phenylsulfonyl) piperidin-4-ylidene)methyl)benzamide (compound 1-90)**

N-(Benzo[d]thiazol-2-yl)-2-fluoro-4-(piperidin-4-ylidenemethyl)benzamide (120 mg, 0.33 mmol) and benzenesulfonyl chloride (63 mg, 0.36 mmol) obtained in Example 87 were reacted in the same manner as in Example 63 to obtain 37 mg of the title compound.

$^1$H NMR (300 MHz, DMSO-$d_6$) δ 12.76 (s, 1H), 8.09 - 7.98 (m, 1H), 7.86 - 7.59 (m, 7H), 7.54 - 7.41 (m, 1H), 7.41 - 7.29 (m, 1H), 7.25 - 7.10 (m, 2H), 6.40 (s, 1H), 3.04 (dt, J = 16.4, 5.8 Hz, 4H), 2.59 - 2.52 (m, 2H), 2.49 - 2.38 (m, 2H).

**Example 91: N-(benzo[d]thiazol-2-yl)-4-((1-(ethylsulfonyl)piperidin-4-ylidene)methyl)-2-fluorobenzamide (compound 1-91)**

N-(Benzo[d]thiazol-2-yl)-2-fluoro-4-(piperidin-4-ylidenemethyl)benzamide (120 mg, 0.33 mmol) and ethylsulfonyl chloride (46 mg, 0.36 mmol) obtained in Example 87 were reacted in the same manner as in Example 63 to obtain 56 mg of the title compound.

$^1$H NMR (300 MHz, DMSO-$d_6$) δ 12.77 (s, 1H), 8.09 - 7.99 (m, 1H), 7.79 (t, J = 7.6 Hz, 2H), 7.54 - 7.42 (m, 1H), 7.42 - 7.30 (m, 1H), 7.31 - 7.18 (m, 2H), 6.49 (s, 1H), 3.35 - 3.30 (m, 2H), 3.26 (t, J = 5.8 Hz, 2H), 3.08 (q, J = 7.4 Hz, 2H), 2.57 - 2.52 (m, 2H), 2.44 (t, J = 5.7 Hz, 2H), 1.22 (t, J = 7.4 Hz, 3H).

**Example 92: N-(benzo[d]thiazol-2-yl)-4-((1-benzoylpiperidin-4-ylidene) methyl)-2-fluorobenzamide (compound 1-92)**

N-(Benzo[d]thiazol-2-yl)-2-fluoro-4-(piperidin-4-ylidenemethyl)benzamide (120 mg, 0.33 mmol) and benzoyl chloride (55 mg, 0.39 mmol) obtained in Example 87 were reacted in the same manner as in Example 63 to obtain 55 mg of the title compound.

$^1$H NMR (300 MHz, DMSO-$d_6$) δ 12.77 (s, 1H), 8.04 (d, J = 7.7 Hz, 1H), 7.79 (d, J = 8.1 Hz, 2H), 7.54 - 7.39 (m, 6H), 7.42 - 7.29 (m, 1H), 7.31 - 7.19 (m, 2H), 6.48 (s, 1H), 3.68 (s, 2H), 3.43 (s, 2H), 2.71 - 2.51 (m, 3H), 2.40 (s, 2H).

**Example 93: 4-(4-(benzo[d]thiazol-2-ylcarbamoyl)-3-fluorobenzylidene)-N-(4-(trifluoromethyl)phenyl)piperidine-1-carboxamide (compound 1-93)**

N-(Benzo[d]thiazol-2-yl)-2-fluoro-4-(piperidin-4-ylidenemethyl)benzamide (120 mg, 0.33 mmol) and 4-(trifluoromethyl)phenyl Isocyanate (67 mg, 0.36 mmol) obtained in Example 87 were reacted in the same manner as in Example 63 to obtain 75 mg of the title compound.

$^1$H NMR (300 MHz, DMSO-$d_6$) δ 12.77 (s, 1H), 8.99 (s, 1H), 8.04 (d, J = 7.8 Hz, 1H), 7.85 - 7.67 (m, 4H), 7.60 (d, J = 8.7 Hz, 2H), 7.54 - 7.42 (m, 1H), 7.42 - 7.30 (m, 1H), 7.33 - 7.20 (m, 2H), 6.48 (s, 1H), 3.58 (dt, J = 19.2, 5.8 Hz, 4H), 2.60 - 2.53 (m, 2H), 2.48 - 2.37 (m, 2H).

**Example 94: 4-(4-(benzo[d]thiazol-2-ylcarbamoyl)-3-fluorobenzylidene)-N-isopropylpiperidine-1-carboxamide (compound 1-94)**

N-(Benzo[d]thiazol-2-yl)-2-fluoro-4-(piperidin-4-ylidenemethyl)benzamide (120 mg, 0.33 mmol) and isopropyl isocyanate (31 mg, 0.36 mmol) obtained in Example 87 were reacted in the same manner as in Example 63 to obtain 75 mg of the title compound.

$^1$H NMR (300 MHz, DMSO-$d_6$) δ 12.75 (s, 1H), 8.09 - 7.99 (m, 1H), 7.85 - 7.72 (m, 2H), 7.54 - 7.42 (m, 1H), 7.42 - 7.30 (m, 1H), 7.30 - 7.18 (m, 2H), 6.42 (s, 1H), 6.23 (d, J = 7.7 Hz, 1H), 3.87 - 3.70 (m, 1H), 3.39 (dt, J = 18.1, 5.8 Hz, 4H), 2.43 (t, J = 5.8 Hz, 2H), 2.31 (t, J = 5.7 Hz, 2H), 1.07 (d, J = 6.5 Hz, 6H).

**Example 95: tert-butyl 4-(4-(benzo[d]thiazol-2-ylcarbamoyl)-2-chlorobenzylidene)piperidine-1-carboxylate (compound 1-95)**

Compound 8 (2.72 g, 7.40 mmol) and tert-butyl 4-((4,4,5,5-tetramethyl-1,3,2-dioxaborolan-2-yl)methylene)piperidine-1-carboxylate (2.00 g, 6.20 mmol) were reacted in the same manner as in Example 63 to obtain 0.87 g of the title compound.
$^1$H NMR (300 MHz, DMSO-$d_6$) δ 13.00 (s, 1H), 8.28 (d, J = 1.8 Hz, 1H), 8.13 - 7.98 (m, 2H), 7.80 (d, J = 8.1 Hz, 1H), 7.56 - 7.42 (m, 2H), 7.42 - 7.32 (m, 1H), 6.43 (s, 1H), 3.42 (dt, J = 23.6, 6.0 Hz, 6H), 2.34 (dt, J = 18.8, 5.9 Hz, 4H), 1.43 (s, 9H).

**Example 96: N-(benzo[d]thiazol-2-yl)-3-chloro-4-(piperidin-4-ylidenemethyl) benzamide (compound 1-96)**

Tert-butyl 4-(4-(benzo[d]thiazol-2-ylcarbamoyl)-2-chlorobenzylidene) piperidine-1-carboxylate (580 mg, 1.20 mml) obtained in Example 95 were reacted in the same manner as in Example 79 to obtain 480 mg of the title compound.
$^1$H NMR (300 MHz, DMSO-$d_6$) δ 12.71 (s, 1H), 8.02 (d, J = 7.7 Hz, 1H), 7.80 (d, J = 8.1 Hz, 2H), 7.51 - 7.34 (m, 6H), 7.40 - 7.19 (m, 1H), 7.31 - 7.19 (m, 2H), 6.41 (s, 1H), 3.61 (s, 2H), 3.40 (s, 2H), 2.70 - 2.41 (m, 3H), 2.40 (s, 2H).

**Example 97: 4-(4-(benzo[dlthiazol-2-ylcarbamoyl)-2-chlorobenzylidene)-N-ethylpiperidine-1-carboxamide (compound 1-97)**

N-CBenzo[d]thiazol-2-yl)-3-chloro-4-(piperidin-4-ylidenemethyl)benzamide (65 mg, 0.18 mmol) and ethyl isocyanate (13 mg, 0.19 mmol) obtained in Example 96 were reacted in the same manner as in Example 63 to obtain 36 mg of the title compound.
$^1$H NMR (300 MHz, DMSO-$d_6$) δ 12.99 (s, 1H), 8.27 (d, J = 1.8 Hz, 1H), 8.13 - 7.99 (m, 2H), 7.80 (d, J = 8.1 Hz, 1H), 7.56 - 7.43 (m, 2H), 7.42 - 7.30 (m, 1H), 6.56 (t, J = 5.4 Hz, 1H), 6.41 (s, 1H), 3.48 - 3.40 (m, 2H), 3.15 - 2.99 (m, 2H), 2.40 - 2.23 (m, 4H), 1.02 (t, J = 7.1 Hz, 3H).

**Example 98: tert-butyl 4-(4-(benzo[d]thiazol-2-ylcarbamoyl)-3,5-difluorobenzylidene)piperidine-1-carboxylate (compound 1-98)**

Compound 9 (1.37 g, 3.70 mmol) and tert-butyl 4-((4,4,5,5-tetramethyl-1,3,2-dioxaborolan-2-yl)methylene)piperidine-1-carboxylate (1.00g, 3.10 mmol) were reacted in the same manner as in Example 63 to obtain 400 mg of the title compound.
$^1$H NMR (300 MHz, DMSO-$d_6$) δ 13.17 (s, 1H), 8.06 (dd, J = 7.8, 1.2 Hz, 1H), 7.82 (d, J = 8.1 Hz, 1H), 7.56 - 7.43 (m, 2H), 7.44 - 7.32 (m, 1H), 7.16 (d, J = 9.2 Hz, 2H), 6.42 (s, 1H), 3.49 - 3.32 (m, 7H), 2.46 (t, J = 5.8 Hz, 8H), 2.33 (t, J = 5.8 Hz, 2H), 1.43 (s, 10H).

**Example 99: N-(benzo[d]thiazol-2-yl)-2,6-difluoro-4-(piperidin-4-ylidene-methyl)benzamide (compound 1-99)**

Tert-butyl 4-(4-(benzo[d]thiazol-2-ylcarbamoy))-3,5-difluorobenzylidene) piperidine-1-carboxylate (58 mg, 0.12 mmol) obtained in Example 98 were reacted in the same manner as in Example 79 to obtain 47 mg of the title compound.
$^1$H NMR (300 MHz, DMSO-$d_6$) δ 8.82 (s, 2H), 8.10 - 7.97 (m, 1H), 7.85 - 7.74 (m, 2H), 7.54 - 7.42 (m, 1H), 7.41 - 7.20 (m, 3H), 6.51 (s, 1H), 3.32 - 3.08 (m, 4H), 2.64 (t, J = 6.0 Hz, 2H), 2.51 (t, J = 5.9 Hz, 3H).

**Example 100: 4-(4-(benzo[d]thiazol-2-ylcarbamoyl)-3,5-difluorobenzylidene)-N-ethylpiperidine-1-carboxamide (compound 1-100)**

N-(Benzo[d]thiazol-2-yl)-2,6-difluoro-4-(piperidin-4-ylidenemethyl)benzamide (69 mg) and ethyl isocyanate (14 mg, 0.20 mmol) obtained in Example 99 were reacted in the same manner as in Example 63 to obtain 34 mg of the title compound.
$^1$H NMR (300 MHz, DMSO-$d_6$) δ 13.16 (s, 1H), 8.06 (d, J = 7.8 Hz, 1H), 7.82 (d, J = 8.0 Hz, 1H), 7.55 - 7.43 (m, 1H), 7.44 - 7.32 (m, 1H), 7.16 (d, J = 9.2 Hz, 2H), 6.55 (t, J = 5.4 Hz, 1H), 6.40 (s, 1H), 3.46 - 3.33 (m, 4H), 3.15 - 2.99 (m, 2H), 2.43 (t, J = 5.8 Hz, 2H), 2.30 (t, J = 5.8 Hz, 2H), 1.03 (t, J = 7.1 Hz, 3H).

**Example 101: 4-(4-(benzo[d]thiazol-2-ylcarbamoyl)-3,5-difluorobenzylidene)-N-phenylpiperidine-1-carboxamide (compound 1-101)**

N-(Benzo[d]thiazol-2-yl)-2,6-difluoro-4-(piperidin-4-ylidenemethyl)benzamide (69 mg) and phenyl isocyanate

(19 mg, 0.16 mmol) obtained in Example 99 were reacted in the same manner as in Example 63 to obtain 29 mg of the title compound.

$^1H$ NMR (300 MHz, DMSO-$d_6$) δ 13.18 (s, 1H), 8.60 (s, 1H), 8.11 - 8.01 (m, 1H), 7.82 (d, J = 8.0 Hz, 1H), 7.56 - 7.43 (m, 3H), 7.44 - 7.32 (m, 1H), 7.30 - 7.13 (m, 4H), 7.00 - 6.88 (m, 1H), 6.45 (s, 1H), 3.56 (dt, J = 17.1, 5.9 Hz, 4H), 2.41 (t, J = 5.8 Hz, 2H).

**Example 102: 4-(4-(Benzo[d]thiazol-2-ylcarbamoyl)-3,5-difluorobenzylidene)-**

**N-(4-(trifluoromethyl)phenyl)piperidine-1-carboxamide (compound 1-102)**

N-(Benzo[d]thiazol-2-yl)-2,6-difluoro-4-(piperidin-4-ylidenemethyl)benzamide (69 mg) and 4-(Trifluoromethyl)phenyl isocyanate (30 mg, 0.16 mmol) obtained in Example 99 were reacted in the same manner as in Example 63 to obtain 40 mg of the title compound.

$^1H$ NMR (300 MHz, DMSO-$d_6$) δ 13.18 (s, 1H), 9.00 (s, 1H), 8.06 (d, J = 7.9 Hz, 1H), 7.81 (d, J = 8.0 Hz, 1H), 7.72 (d, J = 8.6 Hz, 2H), 7.60 (d, J = 8.7 Hz, 2H), 7.55 - 7.43 (m, 1H), 7.44 - 7.32 (m, 1H), 7.19 (d, J = 9.2 Hz, 2H), 6.45 (s, 1H), 3.58 (dt, J = 16.6, 5.9 Hz, 4H), 2.54 (s, 4H), 2.44 (d, J = 5.8 Hz, 2H).

**Example 103: Tert-butyl 4-(4-(benzo[d]thiazol-2-ylcarbamoyl)-3-chlorobenzylidene)piperidine-1-carboxylate (compound 1-103)**

Compound 10 (525 mg, 1.43 mmol) and tert-butyl 4-((4,4,5,5-tetramethyl-1,3,2-dioxaborolan-2-yl)methylene)piperidine-1-carboxylate (692 mg, 2.14 mmol) were reacted in the same manner as in Example 63 to obtain 330 mg of the title compound.

$^1H$ NMR (300 MHz, DMSO-$d_6$) δ 12.92 (s, 1H), 8.09 - 7.99 (m, 1H), 7.80 (d, J = 8.0 Hz, 1H), 7.68 (d, J = 7.9 Hz, 1H), 7.54 - 7.40 (m, 2H), 7.42 - 7.29 (m, 2H), 6.43 (s, 1H), 3.41 (dt, J = 18.4, 5.9 Hz, 7H), 2.44 (t, J = 6.0 Hz, 2H), 2.33 (t, J = 5.7 Hz, 2H), 1.43 (s, 9H).

**Example 104: N-(Benzo[d]thiazol-2-yl)-2-chloro-4-(piperidin-4-ylidenemethyl) benzamide (compound 1-104)**

Tert-butyl 4-(4-(benzo[d]thiazol-2-ylcarbamoyl)-3-chlorobenzylidene) piperidine-1-carboxylate (320 mg, 0.66 mmol) obtained in Example 103 was reacted in the same manner as in Example 79 to obtain 300 mg of the title compound.

$^1H$ NMR (300 MHz, DMSO-$d_6$) δ 12.72 (s, 1H), 8.07 - 7.87 (m, 1H), 7.79 (d, J = 8.0 Hz, 1H), 7.70 (d, J = 7.9 Hz, 1H), 7.54 - 7.40 (m, 2H), 7.42 - 7.29 (m, 2H), 6.45 (s, 1H), 3.44 (dt, J = 18.4, 5.9 Hz, 7H), 2.48 (t, J = 6.0 Hz, 2H), 2.37 (t, J = 5.7 Hz, 2H).

**Example 105: 4-(4-(Benzo[d]thiazol-2-ylcarbamoyl)-3-chlorobenzylidene)-N-ethylpiperidine-1-carboxamide (compound 1-105)**

N-CBenzo[d]thiazol-2-yl)-2-chloro-4-(piperidin-4-ylidenemethyl)benzamide (100 mg, 0.26) and ethyl isocyanate (20 mg, 0.27 mmol) ) obtained in Example 104 were reacted in the same manner as in Example 63 to obtain 45 mg of the title compound.

$^1H$ NMR (300 MHz, DMSO-$d_6$) δ 12.91 (s, 1H), 8.04 (d, J = 8.0, 1.2 Hz, 1H), 7.80 (d, J = 8.0 Hz, 1H), 7.68 (d, J = 7.9 Hz, 1H), 7.54 - 7.40 (m, 2H), 7.42 - 7.28 (m, 2H), 6.54 (t, J = 5.4 Hz, 1H), 6.41 (s, 1H), 3.42 (t, J = 5.7 Hz, 3H), 3.07 (qd, J = 7.1, 5.2 Hz, 2H), 2.35 (dt, J = 30.4, 5.7 Hz, 4H), 1.03 (t, J = 7.1 Hz, 3H).

**Example 106: 4-(4-(Benzo[d]thiazol-2-ylcarbamoyl)-3-chlorobenzylidene)-N-phenylpiperidine-1-carboxamide (compound 1-106)**

N-(Benzo[d]thiazol-2-yl)-2-chloro-4-(piperidin-4-ylidenemethyl)benzamide (100 mg, 0.26) and phenyl isocyanate (34 mg, 0.29 mmol) ) obtained in Example 104 were reacted in the same manner as in Example 63 to obtain 54 mg of the title compound.

$^1H$ NMR (300 MHz, DMSO-$d_6$) δ 12.93 (s, 1H), 8.59 (s, 1H), 8.09 - 8.00 (m, 1H), 7.80 (d, J = 8.0 Hz, 1H), 7.69 (d, J = 7.9 Hz, 1H), 7.54 - 7.42 (m, 4H), 7.42 - 7.31 (m, 2H), 7.24 (t, J = 7.9 Hz, 2H), 7.00 - 6.89 (m, 1H), 6.46 (s, 1H), 3.56 (dt, J = 21.1, 5.8 Hz, 4H), 2.47 - 2.35 (m, 2H).

**Example 107: 4-(4-(Benzo[d]thiazol-2-ylcarbamoyl)-3-chlorobenzylidene)-N-(4-(trifluoromethyl)phenyl)piperidine-1-carboxamide (compound 1-107)**

N-CBenzo[d]thiazol-2-yl)-2-chloro-4-(piperidin-4-ylidenemethyl)benzamide (100 mg, 0.26) and 4-(trifluoromethyl)phenyl isocyanate (51 mg, 0.29 mmol) ) obtained in Example 104 were reacted in the same manner as in Example 63 to obtain 57 mg of the title compound.

$^1H$ NMR (300 MHz, DMSO-$d_6$) δ 12.92 (s, 1H), 8.99 (s, 1H),8.08 - 8.00 (m, 1H), 7.80 (d, J = 8.0 Hz, 1H), 7.76 - 7.65 (m, 3H), 7.60 (d, J = 8.7 Hz, 2H), 7.52 - 7.42 (m, 2H), 7.41 - 7.32 (m, 2H), 6.47 (s, 1H), 3.58 (dt, J = 20.6, 6.0 Hz, 4H), 2.43 (t, J = 5.7 Hz, 2H).

**Example 108: 4-(4-(Benzo[d]thiazol-2-ylcarbamoyl)-3-chlorobenzylidene)-N-(4-fluorophenyl)piperidine-1-carboxamide (compound 1-108)**

N-CBenzo[d]thiazol-2-yl)-2-chloro-4-(piperidin-4-ylidenemethyl)benzamide (200 mg, 0.52) and 4-fluorophenyl isocyanate (78 mg, 0.57 mmol) ) obtained in Example 104 were reacted in the same manner as in Example 63 to obtain 117 mg of the title compound.

$^1H$ NMR (300 MHz, DMSO-$d_6$) δ 12.92 (s, 1H), 8.63 (s, 1H), 8.04 (dd, J = 8.0, 1.2 Hz, 1H), 7.80 (d, J = 8.0 Hz, 1H), 7.69 (d, J = 7.9 Hz, 1H), 7.54 - 7.43 (m, 4H), 7.41 - 7.31 (m, 2H), 7.14 - 7.02 (m, 2H), 6.46 (s, 1H), 3.55 (dt, J = 20.9, 5.7 Hz, 4H), 2.50 - 2.35 (m, 4H).

**Example 109: Tert-butyl 4-((5-(benzo[d]thiazol-2-ylcarbamoyl)thiophen-2-yl)methylene)piperidine-1-carboxylate (compound 1-109)**

Compound 11 (1.26 g, 3.71 mmol) and tert-butyl 4-((4,4,5,5-tetramethyl-1,3,2-dioxaborolan-2-yl)methylene)piperidine-1-carboxylate (1.00 g, 3.09 mmol) were reacted in the same manner as in Example 63 to obtain 285 mg of the title compound.

$^1H$ NMR (400 MHz, DMSO-$d_6$) δ 13.23 (s, 1H), 8.79 (s, 1H), 8.01 (d, J = 7.9 Hz, 1H), 7.75 (s, 1H), 7.48 (ddd, J = 8.3, 7.3, 1.3 Hz, 1H), 7.34 (td, J = 7.7, 1.1 Hz, 1H), 6.68 (s, 1H), 3.46 (t, J = 5.9 Hz, 4H), 2.96 (t, J = 5.9 Hz, 2H), 2.39 (t, J = 5.9 Hz, 2H), 1.43 (s, 9H).

**Example 110: N-(Benzo[d]thiazol-2-yl)-5-(piperidin-4-ylidenemethyl) thiophene-2-carboxamide (compound 1-110)**

Tert-butyl 4-((5-(benzo[d]thiazol-2-ylcarbamoyl)thiophen-2-yl)methylene) piperidine-1-carboxylate (250 mg, 0.55 mmol) obtained in Example 109 was reacted in the same manner as in Example 79 to obtain 377 mg of the title compound.

MS (ESI) m/z calcd for $C_{18}H_{17}N_3OS_2$ $[M]^+$ ,355.1; found, 356.3 $[M + H]^+$.

**Example 111: 4-((5-(Benzo[dlthiazol-2-ylcarbamoyl)thiophen-2-yl)methylene)-N-ethylpiperidine-1-carboxamide (compound 1-111)**

N-(Benzo[d]thiazol-2-yl)-5-(piperidin-4-ylidenemethyl)thiophene-2-carboxamide (100 mg, 0.28 mmol) and ethyl isocyanate (22 mg, 0.31 mmol) obtained in Example 109 were reacted in the same manner as in Example 63 to obtain 47 mg of the title compound.

$^1H$ NMR (300 MHz, DMSO-$d_6$) δ 12.98 (s, 1H), 8.20 (s, 1H), 8.01 (d, J = 7.7 Hz, 1H), 7.76 (d, J = 8.0 Hz, 1H), 7.47 (ddd, J = 8.3, 7.3, 1.3 Hz, 1H), 7.33 (td, J = 7.6, 1.2 Hz, 1H), 7.15 (d, J = 4.0 Hz, 1H), 6.55 (d, J = 7.0 Hz, 2H), 3.47 - 3.39 (m, 4H), 3.07 (qd, J = 7.1, 5.2 Hz, 2H), 2.63 - 2.54 (m, 2H), 2.34 (t, J = 5.7 Hz, 2H), 1.03 (t, J = 7.1 Hz, 3H).

**Example 112: 4-((5-(Benzo[d]thiazol-2-ylcarbamoyl)thiophen-2-yl)methylene)-N-phenylpiperidine-1-carboxamide (compound 1-112)**

N-(Benzo[d]thiazol-2-yl)-5-(piperidin-4-ylidenemethyl)thiophene-2-carboxamide (100 mg, 0.28 mmol) and phenyl isocyanate (37 mg, 0.31 mmol) obtained in Example 109 were reacted in the same manner as in Example 63 to obtain 54 mg of the title compound.

$^1H$ NMR (300 MHz, DMSO-$d_6$) δ 12.99 (s, 1H), 8.58 (s, 1H), 8.22 (s, 1H), 8.01 (d, J = 7.8 Hz, 1H), 7.76 (d, J = 8.0 Hz, 1H), 7.55 - 7.43 (m, 3H), 7.39 - 7.28 (m, 1H), 7.29 - 7.15 (m, 3H), 7.00 - 6.90 (m, 1H), 6.61 (s, 1H), 3.65 - 3.54 (m, 4H), 2.69 (d, J = 5.7 Hz, 2H), 2.46 (d, J = 5.8 Hz, 2H).

**Example 113: 4-((5-(Benzo[d]thiazol-2-ylcarbamoyl)thiophen-2-yl)methylene)-N-(4-fluorophenyl)piperidine-1-carboxamide (compound 1-113)**

N-(Benzo[dlthiazol-2-yl)-5-(piperidin-4-ylidenemethyl)thiophene-2-carboxamide (100 mg, 0.28 mmol) and 4-(trifluoromethyl)phenyl isocyanate (42 mg, 0.31 mmol) obtained in Example 109 were reacted in the same manner as in Example 63 to obtain 58 mg of the title compound.

$^1$H NMR (300 MHz, DMSO-$d_6$) δ 12.98 (s, 1H), 8.62 (s, 1H), 8.22 (s, 1H), 8.01 (d, J = 7.8 Hz, 1H), 7.76 (d, J = 8.0 Hz, 1H), 7.48 (dddd, J = 8.4, 6.4, 4.3, 2.5 Hz, 3H), 7.34 (td, J = 7.6, 1.2 Hz, 1H), 7.18 (d, J = 4.0 Hz, 1H), 7.15 - 7.00 (m, 2H), 6.61 (s, 1H), 3.58 (q, J = 5.4 Hz, 4H), 2.68 (t, J = 5.8 Hz, 2H), 2.44 (t, J = 5.8 Hz, 2H).

**Example 114: Tert-butyl 4-((5-(benzo[d]thiazol-2-ylcarbamoyl)thiazol-2-yl) methylene)piperidine-1-carboxylate (compound 1-114)**

Compound 12 (1.26 g, 3.71 mmol) and tert-butyl 4-((4,4,5,5-tetramethyl-1,3,2-dioxaborolan-2-yl)methylene)piperidine-1-carboxylate (1.00 g, 3.09 mmol) were reacted in the same manner as in Example 63 to obtain 350 mg of the title compound.

$^1$H NMR (400 MHz, DMSO-$d_6$) δ 13.23 (s, 1H), 8.79 (s, 1H), 8.01 (d, J = 7.9 Hz, 1H), 7.75 (s, 1H), 7.48 (ddd, J = 8.3, 7.3, 1.3 Hz, 1H), 7.34 (td, J = 7.7, 1.1 Hz, 1H), 6.68 (s, 1H), 3.46 (t, J = 5.9 Hz, 4H), 2.96 (t, J = 5.9 Hz, 2H), 2.39 (t, J = 5.9 Hz, 2H), 1.43 (s, 9H).

**Example 115: N-(Benzo[d]thiazol-2-yl)-2-(piperidin-4-ylidenemethyl) thiazole-5-carboxamide (compound 1-115)**

Tert-butyl 4-((5-(benzo[d]thiazol-2-ylcarbamoyl)thiazol-2-yl)methylene) piperidine-1-carboxylate (250 mg, 0.55 mmol) obtained in Example 114 were reacted in the same manner as in Example 79 to obtain 374 mg of the title compound. MS (ESI) m/z calcd for $C_{17}H_{16}N_4OS_2$ $[M]^+$,356.1; found, 357.1 $[M + H]^+$

**Example 116: N-(Benzo[d]thiazol-2-yl)-2-((1-(ethylcarbamoyl)piperidin-4-ylidene)methyl)thiazole-5-carboxamide (compound 1-116)**

N-(Benzo[d]thiazol-2-yl)-2-(piperidin-4-ylidenemethyl)thiazole-5-carboxamide (120 mg, 0.34 mmol) and ethyl isocyanate (26 mg, 0.31 mmol) obtained in Example 115 were reacted in the same manner as in Example 63 to obtain 47 mg of the title compound.

$^1$H NMR (400 MHz, DMSO-$d_6$) δ 13.22 (s, 1H), 8.79 (s, 1H), 8.01 (d, J = 7.9 Hz, 1H), 7.75 (d, J = 7.7 Hz, 1H), 7.54 - 7.42 (m, 1H), 7.41 - 7.30 (m, 1H), 6.67 (s, 1H), 6.55 (t, J = 5.4 Hz, 1H), 3.44 (t, J = 5.7 Hz, 4H), 3.08 (qd, J = 7.1, 5.1 Hz, 2H), 2.91 (t, J = 5.7 Hz, 2H), 2.37 (t, J = 5.7 Hz, 2H), 1.03 (t, J = 7.1 Hz, 3H).

**Example 117: N-(Benzo[d]thiazol-2-yl)-2-((1-(phenylcarbamoyl)piperidin-4-ylidene)methyl)thiazole-5-carboxamide (compound 1-117)**

N-(Benzo[d]thiazol-2-yl)-2-(piperidin-4-ylidenemethyl)thiazole-5-carboxamide (120 mg, 0.34 mmol) and phenyl isocyanate (44 mg, 0.37 mmol) obtained in Example 115 were reacted in the same manner as in Example 63 to obtain 48 mg of the title compound.

$^1$H NMR (400 MHz, DMSO-$d_6$) δ 13.22 (s, 1H), 8.81 (s, 1H), 8.59 (s, 1H), 8.01 (d, J = 7.9 Hz, 1H), 7.76 (s, 1H), 7.53 - 7.44 (m, 3H), 7.39 - 7.31 (m, 1H), 7.28 - 7.20 (m, 2H), 6.98 - 6.91 (m, 1H), 6.72 (s, 1H), 3.61 (dq, J = 6.5, 3.9 Hz, 4H), 3.02 (t, J = 5.8 Hz, 2H), 2.47 (d, J = 6.0 Hz, 2H).

**Example 118: N-(Benzo[d]thiazol-2-yl)-2-((1-((4-fluorophenyl)carbamoyl) piperidin-4-ylidene)methyl)thiazole-5-carboxamide (compound 1-118)**

N-(Benzo[d]thiazol-2-yl)-2-(piperidin-4-ylidenemethyl)thiazole-5-carboxamide (120 mg, 0.34 mmol) and 4-(trifluoromethyl)phenyl isocyanate (51 mg, 0.37 mmol) obtained in Example 115 were reacted in the same manner as in Example 63 to obtain 56 mg of the title compound.

$^1$H NMR (400 MHz, DMSO-$d_6$) δ 13.23 (s, 1H), 8.80 (s, 1H), 8.63 (s, 1H), 8.01 (d, J = 7.9 Hz, 1H), 7.83 - 7.69 (m, 1H), 7.55 - 7.43 (m, 3H), 7.40 - 7.30 (m, 1H), 7.15 - 7.04 (m, 2H), 6.71 (s, 1H), 3.65 - 3.56 (m, 4H), 3.01 (t, J = 5.8 Hz, 2H), 2.47 (d, J = 6.0 Hz, 4H).

**Example 119: tert-butyl 4-(4-(benzo[d]thiazol-2-ylcarbamoyl)-3-methylbenzylidene)piperidine-1-carboxylate (compound 1-119)**

Compound 13 (700 mg, 2.02 mmol) and tert-butyl 4-((4,4,5,5-tetramethyl-1,3,2-dioxaborolan-2-yl)methylene)piperidine-1-carboxylate (977 mg, 3.02 mmol) were reacted in the same manner as in Example 63 to obtain 552mg of the title compound.

$^1$H NMR (300 MHz, DMSO-$d_6$) δ 12.71 (s, 1H), 8.07 - 7.97 (m, 1H), 7.78 (d, J = 7.9 Hz, 1H), 7.61 (d, J = 7.8 Hz, 1H), 7.53 - 7.41 (m, 1H), 7.40 - 7.28 (m, 1H), 7.19 (d, J = 8.3 Hz, 2H), 6.41 (s, 1H), 3.41 (dt, J = 20.9, 5.9 Hz, 7H), 2.45 (s, 5H), 2.32 (t, J = 5.9 Hz, 2H), 1.43 (s, 9H).

**Example 120: N-(Benzo[d]thiazol-2-yl)-2-methyl-4-(piperidin-4-ylidenemethyl) benzamide (compound 1-120)**

Tert-butyl 4-(4-(benzo[cl]thiazol-2-ylcarbamoyl)-3-methylbenzylidene) piperidine-1-carboxylate (420 mg, 0.91 mmol) obtained in Example 119 were reacted in the same manner as in Example 79 to obtain 700 mg of the title compound.

$^1$H NMR (400 MHz, DMSO-$d_6$) δ 8.02 (dd, J = 7.9, 1.2 Hz, 1H), 7.78 (d, J = 8.0 Hz, 1H), 7.64 (d, J = 7.8 Hz, 1H), 7.47 (td, J = 8.2, 7.7, 1.3 Hz, 1H), 7.35 (td, J = 7.6, 1.1 Hz, 1H), 7.22 (d, J = 7.6 Hz, 2H), 6.50 (s, 1H), 3.25 - 3.07 (m, 4H), 2.60 (dt, J = 39.0, 6.0 Hz, 4H), 2.46 (s, 3H).

**Example 121: 4-(4-(Benzo[d]thiazol-2-ylcarbamoyl)-3-methylbenzylidene)-N-ethylpiperidine-1-carboxamide (compound 1-121)**

N-(Benzo[d]thiazol-2-yl)-2-methyl-4-(piperidin-4-ylidenemethyl)benzamide (150 mg, 0.41 mmol) and ethyl isocyanate (32 mg, 0.45 mmol) obtained in Example 120 were reacted in the same manner as in Example 63 to obtain 70 mg of the title compound.

$^1$H NMR (400 MHz, DMSO-$d_6$) δ 12.70 (s, 1H), 8.02 (dd, J = 7.9, 1.1 Hz, 1H), 7.78 (d, J = 7.9 Hz, 1H), 7.61 (d, J = 7.7 Hz, 1H), 7.51 - 7.42 (m, 1H), 7.39 - 7.30 (m, 1H), 7.19 (d, J = 8.1 Hz, 2H), 6.52 (t, J = 5.4 Hz, 1H), 6.39 (s, 1H), 3.45 - 3.37 (m, 2H), 3.35 (s, 4H), 3.12 - 3.03 (m, 2H), 2.45 (s, 3H), 2.41 (t, J = 5.9 Hz, 5H), 2.30 (t, J = 5.7 Hz, 2H), 1.03 (t, J = 7.1 Hz, 3H).

**Example 122: 4-(4-(Benzo[d]thiazol-2-ylcarbamoyl)-3-methylbenzylidene)-N-phenylpiperidine-1-carboxamide (compound 1-122)**

N-(Benzo[d]thiazol-2-yl)-2-methyl-4-(piperidin-4-ylidenemethyl)benzamide (150 mg, 0.41 mmol) and ethyl isocyanate (54 mg, 0.45 mmol) obtained in Example 120 were reacted in the same manner as in Example 63 to obtain 85 mg of the title compound.

$^1$H NMR (400 MHz, DMSO-$d_6$) δ 12.71 (s, 1H), 8.57 (s, 1H), 8.02 (dd, J = 8.0, 1.2 Hz, 1H), 7.78 (d, J = 8.0 Hz, 1H), 7.63 (d, J = 7.7 Hz, 1H), 7.53 - 7.43 (m, 3H), 7.34 (td, J = 7.6, 1.2 Hz, 1H), 7.23 (td, J = 8.3, 6.3 Hz, 4H), 6.94 (tt, J = 7.3, 1.2 Hz, 1H), 6.43 (s, 1H), 3.59 (dd, J = 6.9, 4.5 Hz, 2H), 3.51 (t, J = 5.7 Hz, 2H), 2.52 (d, J = 5.9 Hz, 2H), 2.46 (s, 3H), 2.40 (t, J = 5.7 Hz, 2H).

**Example 123: 4-(4-(Benzo[d]thiazol-2-ylcarbamoyl)-3-methylbenzylidene)-N-(4-fluorophenyl)piperidine-1-carboxamide (compound 1-123)**

N-(Benzo[d]thiazol-2-yl)-2-methyl-4-(piperidin-4-ylidenemethyl)benzamide (150 mg, 0.41 mmol) and 4-(trifluoromethyl)phenyl isocyanate (62 mg, 0.45 mmol) obtained in Example 120 were reacted in the same manner as in Example 63 to obtain 88 mg of the title compound.

$^1$H NMR (400 MHz, DMSO-$d_6$) δ 12.71 (s, 1H), 8.61 (s, 1H), 8.02 (dd, J = 8.1, 1.1 Hz, 1H), 7.78 (d, J = 8.0 Hz, 1H), 7.63 (d, J = 7.7 Hz, 1H), 7.53 - 7.42 (m, 3H), 7.34 (td, J = 7.7, 1.2 Hz, 1H), 7.21 (d, J = 8.0 Hz, 2H), 7.12 - 7.04 (m, 2H), 6.43 (s, 1H), 3.63 - 3.54 (m, 2H), 3.50 (t, J = 5.7 Hz, 2H), 2.54 - 2.51 (m, 2H), 2.46 (s, 3H), 2.40 (t, J = 5.8 Hz, 2H).

**Experimental Example 1: Measurement of inhibitory activity against Sirt7**

Promega's Cat. No.6450 SIRT Glo assay kit was used, and as the target SIRT7 protein, No. S41-30H protein of SignalChem's Cat. was used. Prepare the compound by diluting it in SIRT Glo buffer in advance to a concentration twice as thick as the final concentration. For the positive control group of white 384 well plate (Greiner Bioone, white, small volume, Cat. No. 874075), and for compound group, dilute sirt7 protein in SIRT Glo buffer and dispense by 5 μL to contain 20 ng per well. In the background control group, 10 μL of SIRT Glo buffer is dispensed.

After adding 5 μL of SIRT Glo buffer to the positive control group, and 5 μL of the diluted compound to the

compound group, react for 30 minutes. After making the substrate provided in the SIRT Glo assay, adding 10 μL each, and reacting for 1 hour, the luciferase activity is measured with a microplate reader (Molecular Device, spectramax i3).

Subtract the luciferase level of the background control group in all wells. The % inhibition of the compound was used to obtain the compound's rate of inhibitory activity against sirt7 in the following:

$$\% \text{ inhibition} = 100-(\text{compound group}/\text{positive control group}) \times 100.$$

Table 1

| compound | inhibitory activity against Sirt7 (% @10 μM) |
|---|---|
| 1-1 | 12% |
| 1-2 | 8% |
| 1-3 | 10% |
| 1-4 | 90% |
| 1-5 | 65% |
| 1-6 | 92% |
| 1-7 | 5% |
| 1-8 | 44% |
| 1-9 | 63% |
| 1-10 | 56% |
| 1-11 | 21% |
| 1-12 | 99% |
| 1-13 | 21% |
| 1-14 | 15% |
| 1-15 | 52% |
| 1-16 | 87% |
| 1-17 | 88% |
| 1-18 | 5% |
| 1-19 | 13% |
| 1-20 | 63% |
| 1-21 | 41% |
| 1-22 | 49% |
| 1-23 | 78% |
| 1-24 | 10% |
| 1-25 | 72% |
| 1-26 | 40% |
| 1-27 | 27% |
| 1-28 | 62% |
| 1-29 | 83% |
| 1-30 | 19% |
| 1-31 | 15% |
| 1-32 | 34% |

(continued)

| compound | inhibitory activity against Sirt7 (% @10 μM) |
|---|---|
| 1-33 | 33% |
| 1-34 | 29% |
| 1-35 | 20% |
| 1-36 | 15% |
| 1-37 | 81% |
| 1-38 | 98% |
| 1-39 | 87% |
| 1-40 | 87% |
| 1-41 | 66% |
| 1-42 | 89% |
| 1-43 | 79% |
| 1-44 | 95% |
| 1-45 | 96% |
| 1-46 | 84% |
| 1-47 | 88% |
| 1-48 | 54% |
| 1-49 | 89% |
| 1-50 | 61% |
| 1-51 | 90% |
| 1-52 | 85% |
| 1-53 | 97% |
| 1-54 | 100% |
| 1-55 | 100% |
| 1-56 | 87% |
| 1-57 | 12% |
| 1-58 | 87% |
| 1-59 | 5% |
| 1-60 | 7% |
| 1-61 | 3% |
| 1-62 | 7% |
| 1-63 | 10% |
| 1-64 | 7% |
| 1-65 | 5% |
| 1-66 | 16% |
| 1-67 | 92% |
| 1-68 | 100% |
| 1-69 | 100% |
| 1-70 | 100% |

(continued)

| compound | inhibitory activity against Sirt7 (% @10 μM) |
|---|---|
| 1-71 | 23% |
| 1-72 | 40% |
| 1-73 | 64% |
| 1-74 | 10% |
| 1-75 | 26% |
| 1-76 | 5% |
| 1-77 | 6% |
| 1-78 | 14% |
| 1-79 | 71% |
| 1-80 | 80% |
| 1-81 | 32% |
| 1-82 | 2% |
| 1-83 | 42% |
| 1-84 | 53% |
| 1-85 | 28% |
| 1-86 | 60% |
| 1-87 | 100% |
| 1-88 | 100% |
| 1-89 | 97% |
| 1-90 | 27% |
| 1-91 | 76% |
| 1-92 | 97% |
| 1-93 | 70% |
| 1-94 | 98% |
| 1-95 | 34% |
| 1-96 | 91% |
| 1-97 | 100% |
| 1-98 | 21% |
| 1-99 | 63% |
| 1-100 | 96% |
| 1-101 | 100% |
| 1-102 | 37% |
| 1-103 | 44% |
| 1-104 | 92% |
| 1-105 | 100% |
| 1-106 | 99% |
| 1-107 | 34% |
| 1-108 | 100% |

**Experimental Example 2: Cancer cell proliferation inhibitory effect of inhibitor compound against Sirt7**

KRAS is a part of the RAS/MAPK pathway, which contributes to the carcinogenesis process by promoting cell growth and proliferation. Mutations in KRAS are found in about 20% of solid cancers and are most commonly found in pancreatic, colorectal, and lung cancers. It has been reported that Sirt7 increases colorectal cancer by increasing MAPK activity through upregulation of p-ERK and p-MEK. Therefore, in this experiment, the cancer cell proliferation inhibitory activity of the compounds of the present invention was evaluated in COL0320DM (KRAS wild-type) and SW480 (KRAS mutant) cells, which are human colorectal cancer cells.

For cell proliferation, cytotoxicity was measured using CCK-8 (Cell counting kit: Dojindo Cat. No. CK04), and 450 nm absorbance was measured to determine whether orange color was formed according to the activity of dehydrogenase in living cells. COL0320DM and SW480 cells were seeded at 3,000 per well in a 96 well plate (BD Falcon, Cat. No. 353072), and after 24 hours, each selected inhibitor compound was treated, and were treated with an inhibitor compound while exchanging the medium every 2 days for a total of 4 days. Cell proliferation was measured with a microplate reader (Molecular Device, spectramax i3) at a wavelength of 450 nm after treatment with 10 μL of CCK-8 per well and reaction at 37° C. for 30 minutes.

As a comparative compound, a Sirt7 inhibitor reported in Biochem Biophys Res Commun.2019;508:451-457 was synthesized and used. Hereinafter, 97491 denotes the comparative compound.

As a result, referring to Figure 1a, it was observed that in COL0320DM cell, t LD50 of he comparative compound (97491) showed 22.5 μM, LD50 of compound 1-37 of the present invention was 12.7 μM, and LD50 of compound 1-38 of the present invention was 16.2 μM, and in the SW480 cell, LD50 of the comparative compound (97491) showed 23.3 μM, LD50 of the present invention compound 1-37 showed 3976 μM, and LD50 of the present invention compound 1-38 showed 1137 μM.

Also, referring to Figure 1b, it was observed that in COL0320DM cell, LD50 of the comparative compound (97491) showed 19.2 μM, LD50 of compound 1-37 of the present invention was 23.6 μM, and LD50 of compound 1-38 of the present invention was 24.2 μM, and in the HT29 cell, LD50 of the comparative compound (97491) showed 16.4 μM, LD50 of the present invention compound 1-37 showed 19.7 μM, and LD50 of the present invention compound 1-38 showed 6.7 μM.

Also, referring to Figure 1c, it was observed that in SW480 cell, LD50 of the comparative compound (97491) showed 18 μM, LD50 of compound 1-37 of the present invention showed 68.4 μM, and LD50 of compound 1-38 of the present invention showed 28 μM, and in SW620 cell, LD50 of the comparative compound (97491) showed 18.1 μM, LD50 of compound 1-37 of the present invention was 48 μM, and LD50 of compound 1-38 of the present invention was 24.8 μM.

**Experimental Example 3: Lactate dehydrogenase (LDH) cytotoxicity test**

LDH (Lactose dehydrogenase) is an enzyme present in the cytoplasm and does not usually pass through the cell membrane, but is released to the outside of the cell, that is, into the medium when the cell membrane is damaged. LDH released is measured to determine whether a compound is toxic. COL0320DM and SW480 cells were treated with the comparative compound (97491) and the compound 1-37 of the present invention at high concentrations of 100 μM and 500 μM.

Thermo's Cat. No. 88953 LDH Cytotoxicity assay kit was used. 20,000 cells were seeded in a 96 well plate and treated with 100 μM and 500 μM compounds after 24 hours of culture. After 48 hours, transfer 50 μL of the compound-treated culture supernatant to a new 96-well plate, add 50 μL of substrate mix buffer provided in the kit, block light, and react at room temperature for 30 minutes. After adding 50 μL of Stop solution, the absorbance was measured at 490 nm using a microplate reader (Molecular Device, spectramax i3).

As a result, referring to Fig. 2(a), in COL0320DM cells, compared to the positive control that damaged all cell membranes, comparative compound (97491) 100 μM showed 51.9% of cytotoxicity, comparative compound (97491) 500 μM showed 59.2% of cytotoxicity, compound 1-37 of the present invention 100 μM showed 62.1% of cytotoxicity, compound 1-37 of the present invention 500 μM showed 62.0% of cytotoxicity.

Referring to Fig. 2 (b), WI38 cells were treated with the comparative compound (97491) and the compound 1-37 of the present invention at high concentrations of 100 μM and 500 μM. In WI38 cells, compared to the positive control that damaged all cell membranes, the comparative compound (97491) 100 μM showed 30.2% of cytotoxicity, the comparative compound (97491) 500 μM showed 54.7% of cytotoxicity, the present invention compound 1-37 100 μM showed 29.9% of cytotoxicity, the present invention compound 1-37 500 μM showed 41.6 % of cytotoxicity.

**Experimental Example 4: In vivo test**

The compounds of the present invention were administered to mouse tumor xenograft to measure tumor suppression in vivo test. BALB/C-nu female 5-week-old mice were introduced. COL0320DM and SW480 cells, which are human colorectal cancer cells, were isolated from culture flasks using trypsin and prepared at a concentration of $1\times10^8$ cell/ml. It was mixed with Matrigel (Gibco, Cat. No. A14133-02) at a ratio of 1:1 and injected 100 μL subcutaneously into the dorsal side of 5-week-old mice. After 2 weeks, when the volume of the mouse tumor reached 100 $mm^3$, oral administration of the compound was started 5 times a week. (Day 0), After that, it was conducted for 3 weeks.

Dissolve the compound in 0.1% carboxymethyl cellulose (CMC) and 0.2% tween 80 solution at concentrations of 2 mg/ml, 4 mg/ml, and 8 mg/ml, and 100 ul was orally administered to achieve concentrations of 10 mpk, 20 mpk, and 40 mpk. In the negative control group, 0.1% carboxymethyl cellulose and 0.2% tween 80 solution were administered instead of the compound according to the same administration route and administration frequency. 6 mice were used for each experimental group (Fig. 3). The size of the tumor was measured in the long axis (length, L) and the short axis (width, W) once every 3 days, and the tumor volume ($mm^3$) was calculated as $(L\times W\times W)/2$ thereby the average for each group was calculated. Statistical processing was performed using the GraphPad Prism6 program, and a p-value of less than 0.05 was considered significant.

A 5-week-old nude mouse whose thymus was removed and whose T cells were reduced and whose immune system was suppressed was introduced, and human colorectal cancer cell lines, COL0320DM and SW480, were subcutaneously injected. As a result of oral administration of the compounds of the present invention 1-37 (10 mpk, 20 mpk, 40 mpk), 1-105 (100 mpk), and 1-106 (100 mpk), which are inhibitors against Sirt7, for 3 weeks, referring to Figs. 4 to 11, compared to the control group fed only a solution in which the reagent was dissolved, the tumor size of both cell lines was reduced, and the results of the first and second in vivo tests showed the same results. In the mouse group injected with COL0320DM, oral administration of 40 mpk of the compound 1-37 showed the most significant tumor formation inhibitory effect and a concentration-dependent tumor formation inhibitory effect.

The above description is merely illustrative of the present invention, and those skilled in the art will be able to make various modifications without departing from the essential characteristics of the present invention. Accordingly, the embodiments disclosed in this specification are intended to explain, not limit, the present invention, and the spirit and scope of the present invention are not limited by these embodiments. The protection scope of the present invention should be construed according to the following claims, and all technologies within the equivalent range should be construed as being included in the scope of the present invention.

## Industrial Applicability

Since it has excellent inhibitory activity of SIRTUIN 7 protein, it can be used to prevent or treat diseases related to SIRTUIN 7 protein.

## Claims

1. A compound represented by Formula 1, a stereoisomer, tautomer, derivative, hydrate, solvate or pharmaceutically acceptable salt thereof:

Formula 1

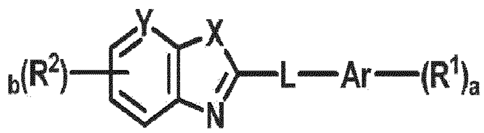

Formula 2-1 Formula 2-2

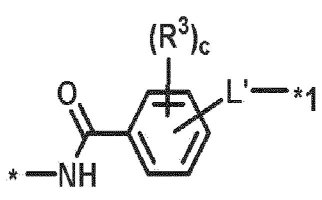

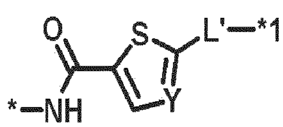

wherein:

1) X is S or $N(R_1)$,
2) wherein $R_1$ is hydrogen; $C_1$~$C_5$ alkyl group; or a $C_7$~$C_{20}$ arylalkyl group substituted with fluorine;
3) Y is independently of each other N or CH,
4) $R^2$ are each the same or different, and each independently hydrogen, or halogen;
5) L is a single bond, $C_1$~$C_6$ alkylene group; $C_2$~$C_6$ alkenylene group; a substituent represented by Formula 2-1; or a substituent represented by Formula 2-2;
6) L' is selected from a group consisting of a single bond; $C_1$~$C_{10}$ alkylene group; $C_2$~$C_{10}$ alkenylene group; and $-(C_nH_{2n})-O-(C_nH_{2n})-$:
7) ∗ represents a position bonded to L, ∗1 represents a position bonded to Ar,
8) $R^3$ are each the same or different, and is each independently selected from the group consisting of hydrogen; halogen; and a $C_1$~$C_{10}$ alkyl group unsubstituted or substituted with fluorine,
9) Ar is a $C_6$~$C_{20}$ arylene group; or a $C_2$~$C_{20}$ heterocyclic group comprising at least one heteroatom of N and O;
10) $R^1$ is selected from the group consisting of hydrogen; halogen; a $C_1$~$C_{10}$ alkyl group unsubstituted or substituted with fluorine; $-SO_2-R^a$; $-CO-NH-R^b$; and $-CO-R^c$;
11) wherein $R^a$ is selected from the group consisting of a $C_1$~$C_{10}$ alkyl group; $C_3$~$C_{20}$ aliphatic ring group; $C_6$~$C_{20}$ aryl group unsubstituted or substituted with $CF_3$; and -N(R')(R");
12) wherein R' and R" are each independently a $C_1$~$C_{10}$ alkyl group;
13) wherein $R^b$ is selected from the group consisting of a $C_6$~$C_{20}$ aryl group; $C_3$~$C_{20}$ aliphatic ring group; fused ring group of $C_3$~$C_{20}$ aliphatic ring and $C_6$~$C_{20}$ aromatic ring; a $C_1$~$C_{20}$ alkyl group unsubstituted or substituted with ha logen; and - $(C_mH_{2m})-CO-O-(C_mH_{2m+1})$ ;
14) Wherein $R^c$ is selected from the group consisting of a $C_1$~$C_{10}$ alkyl group; $C_6$~$C_{20}$ aryl group, and $C_1$~$C_{10}$ alkoxy group:
15) a is 0 or 1, b is an integer from 0 to 3, c is an integer from 0 to 4, n is independently an integer from 0 to 2, m is independently an integer from 1 to 6,
16) wherein, the alkyl group, the alkenyl group, the alkoxy group, the aryl group, the arylalkyl group, the alkylene group, the alkenylene group, the arylene group, the aliphatic ring group, the heterocyclic group, and the fused ring group may be further substituted with one or more substituents selected from the group consisting of halogen; $C_1$~$C_{10}$ alkyl group; $C_2$~$C_{10}$ alkenyl group; $C_2$~$C_{10}$ alkynyl group; $C_1$~$C_{10}$ alkoxy group; $C_1$~$C_{10}$ carbonyl group; and a $C_1$-$C_{10}$ alkyl group substituted with fluorine.

2. The compound of claim 1, a stereoisomer, tautomer, derivative, hydrate, solvate or pharmaceutically acceptable salt thereof, wherein Formula 1 is represented by Formula 3-1 or Formula 3-2:

Formula 3-1

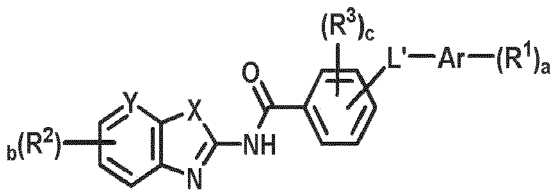

Formula 3-2

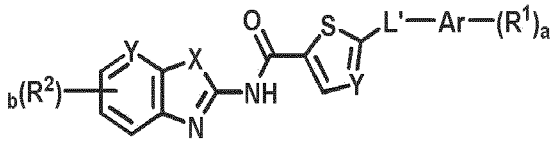

wherein, X, Y, $R^1$, $R^2$, $R^3$, L', Ar, a, b and c are the same as defined in claim 1.

3. The compound of claim 1, a stereoisomer, tautomer, derivative, hydrate, solvate or pharmaceutically acceptable salt thereof, wherein Formula 1 is represented by Formula 4:

Formula 4

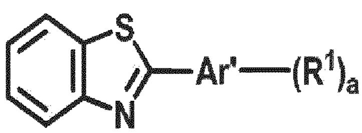

wherein:

1) $R^1$ and a are the same as defined in claim 1,
2) Ar' is a $C_2$-$C_{20}$ heterocyclic group comprising at least one N.

**4.** The compound of claim 1, a stereoisomer, tautomer, derivative, hydrate, solvate or pharmaceutically acceptable salt thereof, wherein Ar is represented by any one of Formula Ar-1 to Formula Ar-11:

Formula Ar-1

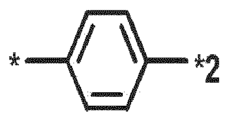

Formula Ar-2

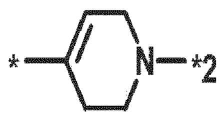

Formula Ar-3

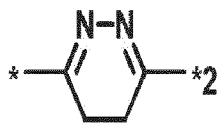

Formula Ar-4

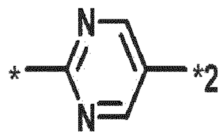

Formula Ar-5

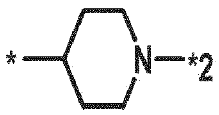

Formula Ar-6

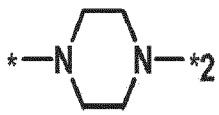

Formula Ar-7

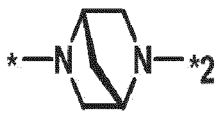

Formula Ar-8

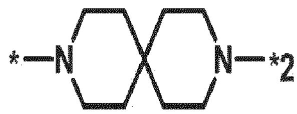

Formula Ar-9

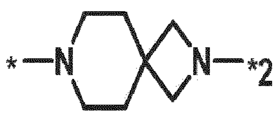

Formula Ar-10

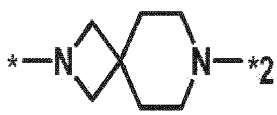

Formula Ar-11

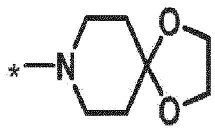

wherein:

1) * represents a position bonded to L,
2) *2 represents the position bonded to $R^1$.

**5.** The compound of claim 1, a stereoisomer, tautomer, derivative, hydrate, solvate or pharmaceutically acceptable salt thereof, wherein Formula 1 is represented by any one of the following compounds:

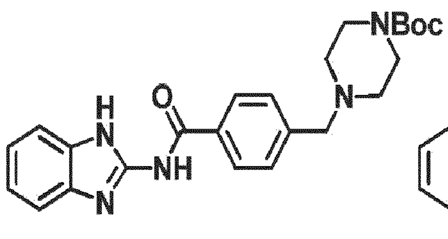

1-1

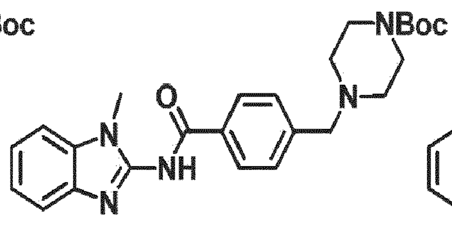

1-2

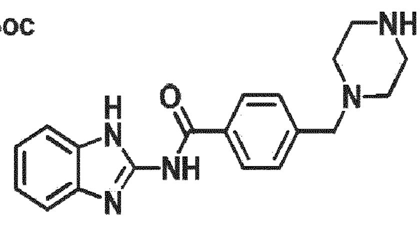

1-3

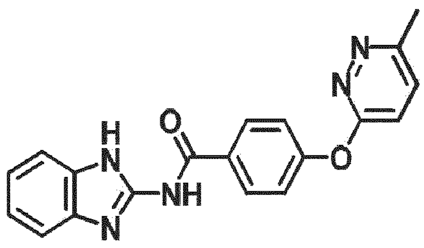

1-4

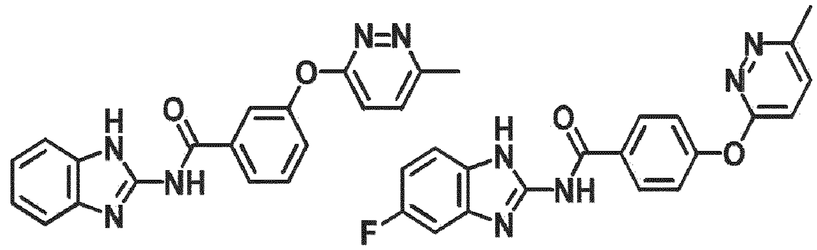

1-5

1-6

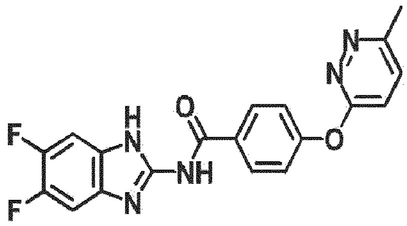

1-7

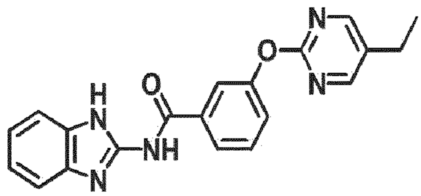

1-8

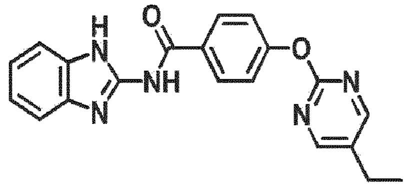

1-9

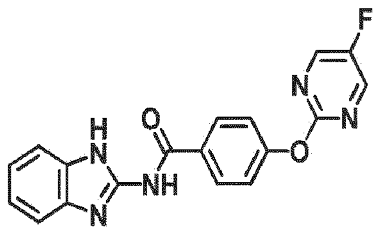

1-10

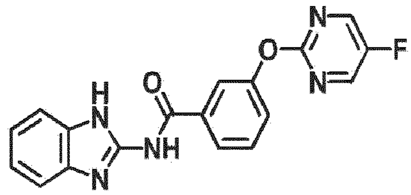

1-11

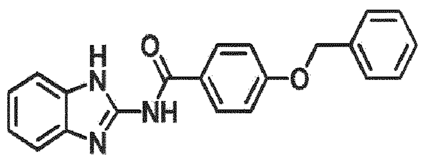

1-12

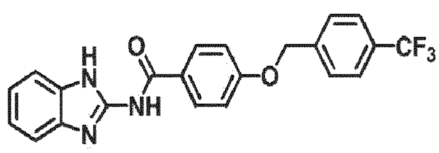

1-13

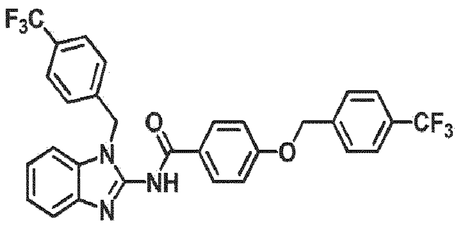

1-14

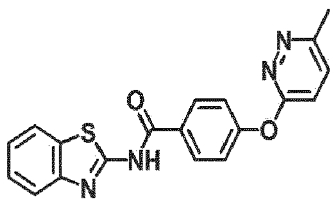

1-15

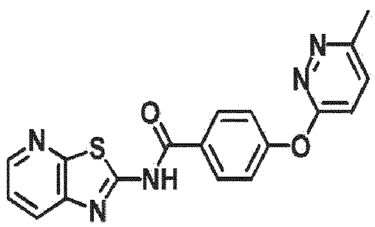

1-16

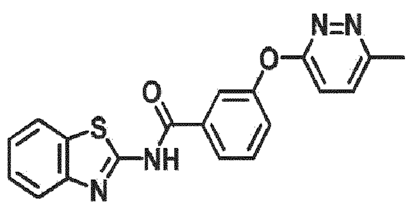

1-17

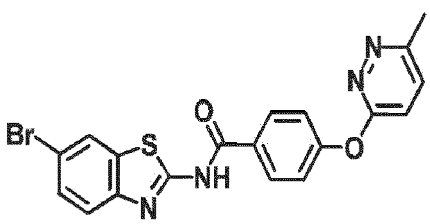

1-18

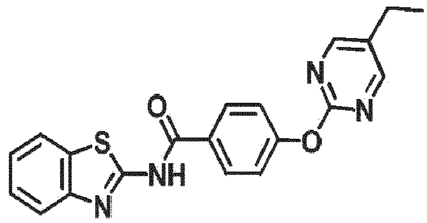
**1-19**

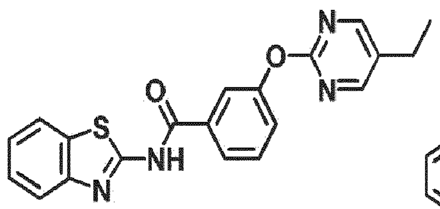
**1-20**

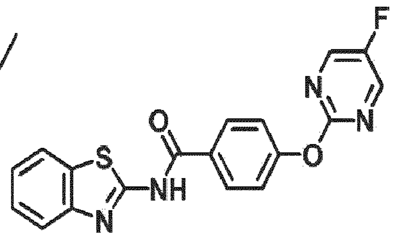
**1-21**

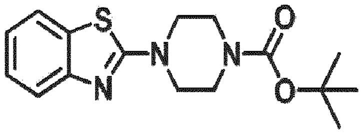
**1-22**

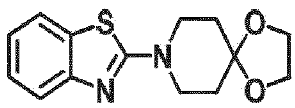
**1-23**

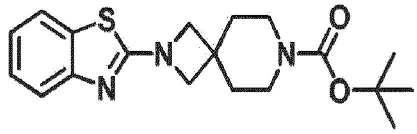
**1-24**

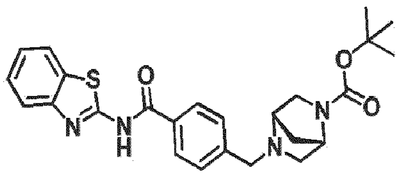
**1-25**

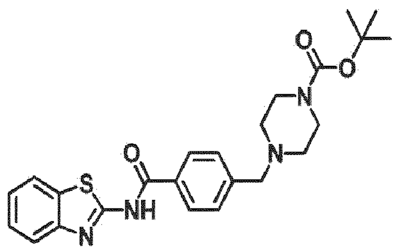
**1-26**

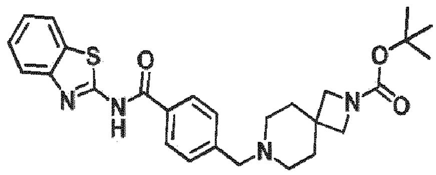
**1-27**

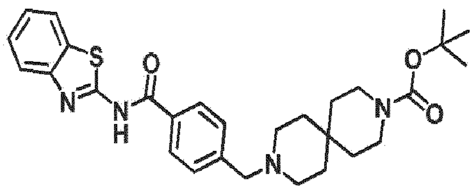
**1-28**

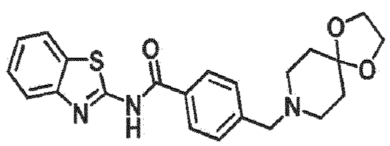
**1-29**

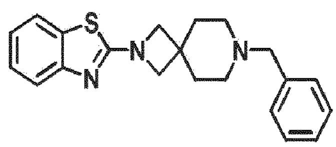
**1-30**

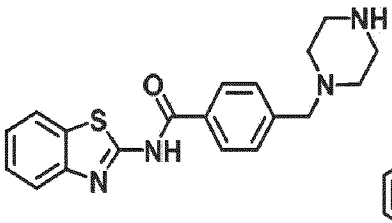
**1-31**

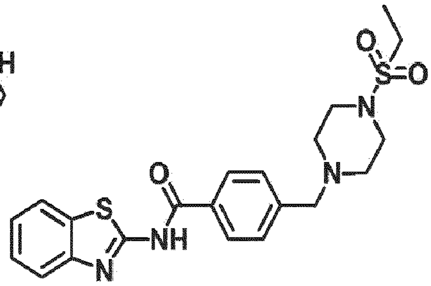
**1-32**

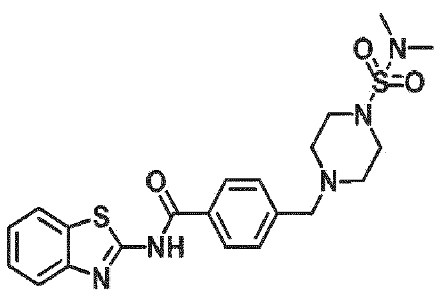
**1-33**

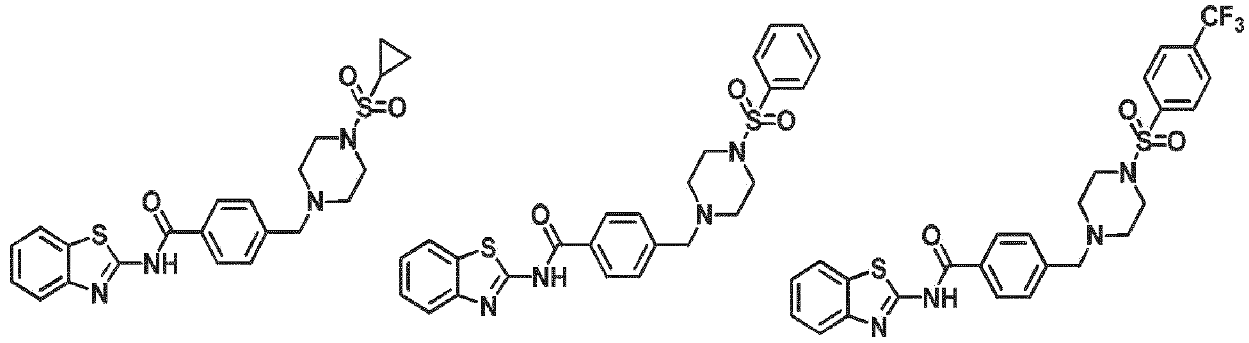

**1-34** **1-35** **1-36**

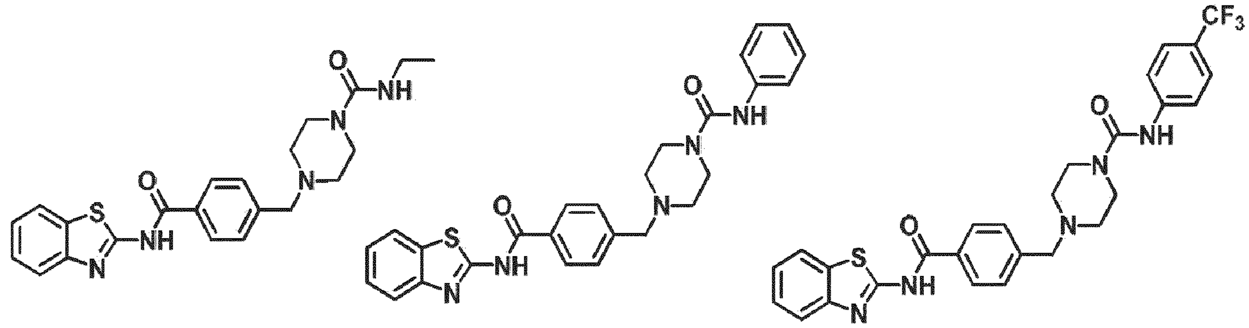

**1-37** **1-38** **1-39**

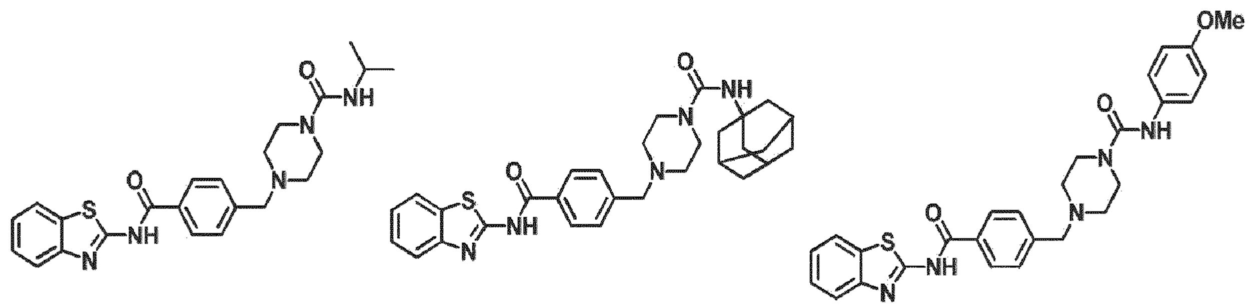

**1-40** **1-41** **1-42**

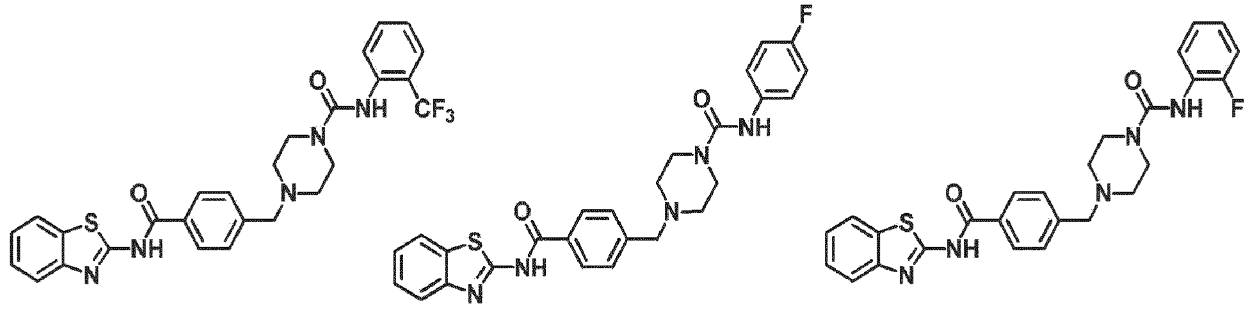

**1-43** **1-44** **1-45**

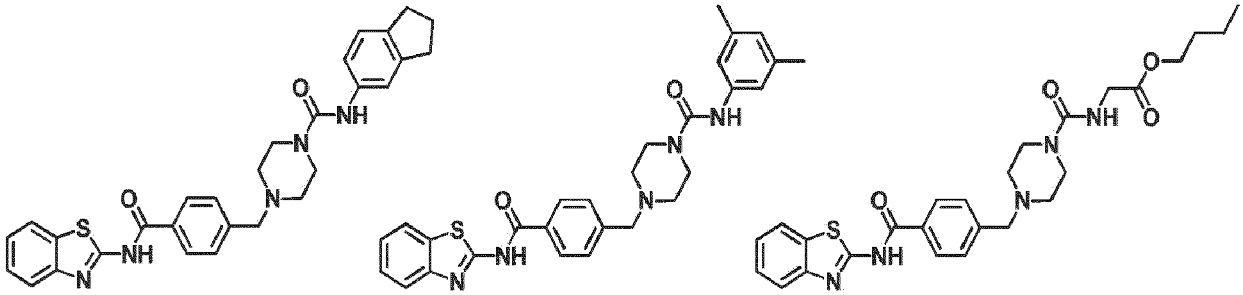

**1-46** **1-47** **1-48**

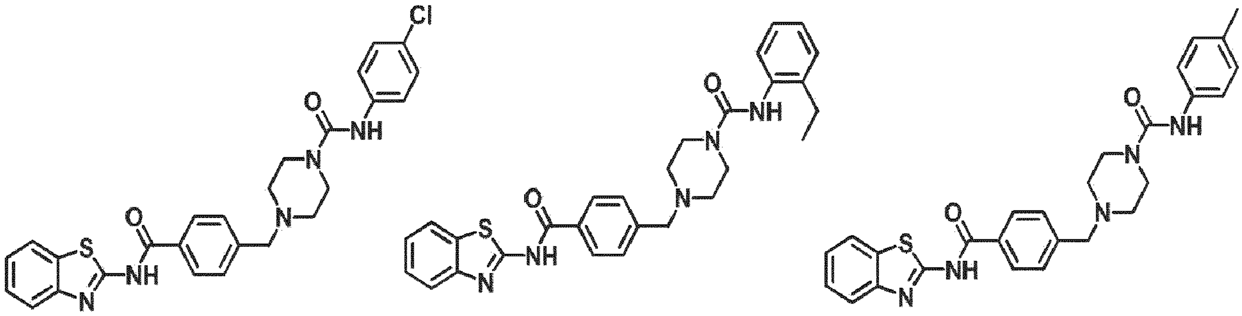

**1-49** **1-50** **1-51**

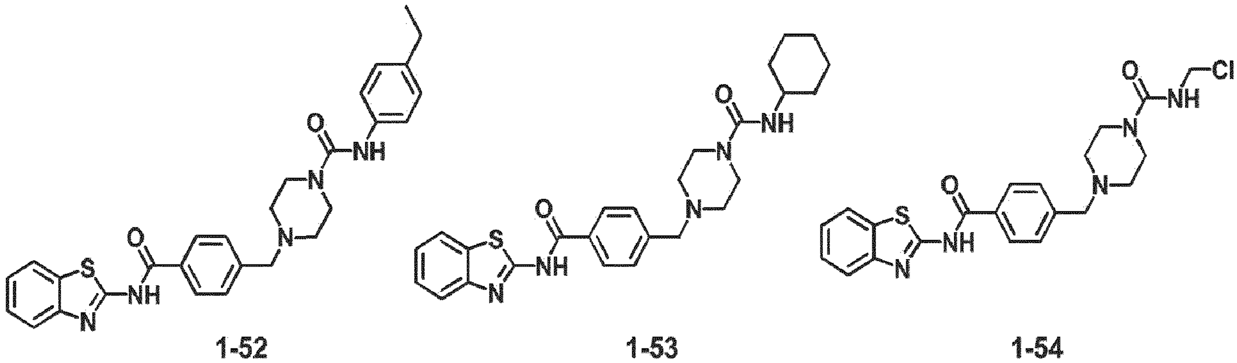

**1-52** **1-53** **1-54**

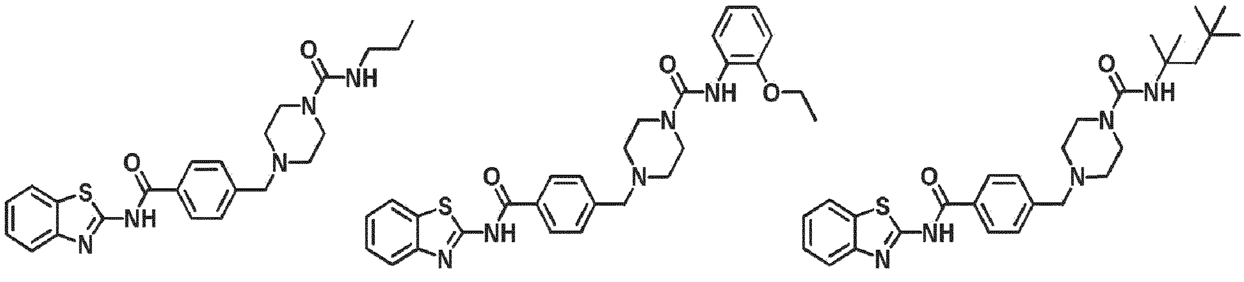

**1-55** **1-56** **1-57**

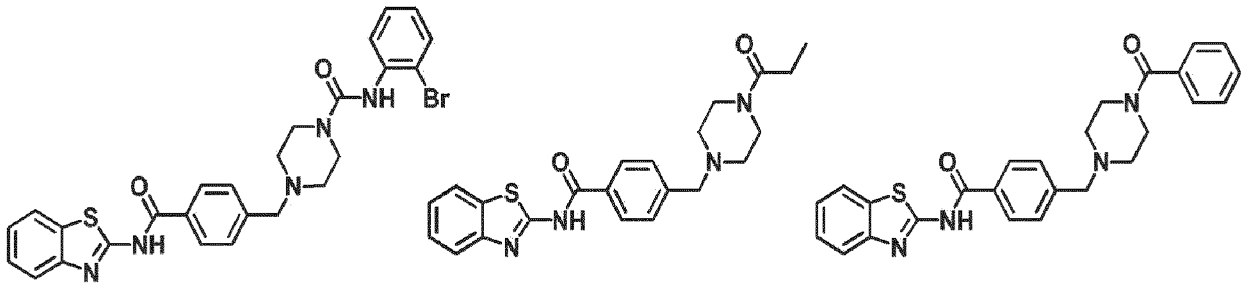

1-58 1-59 1-60

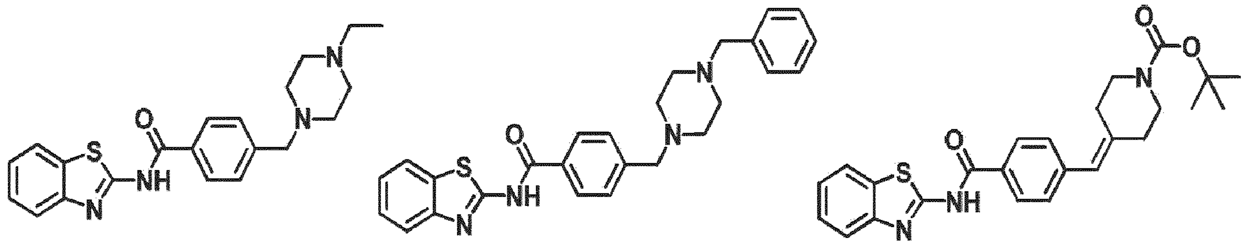

1-61 1-62 1-63

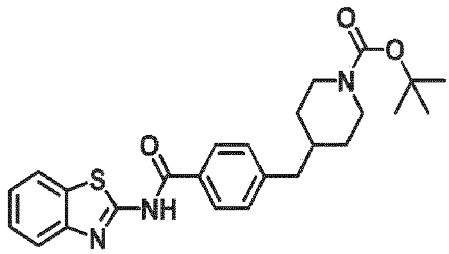

1-64

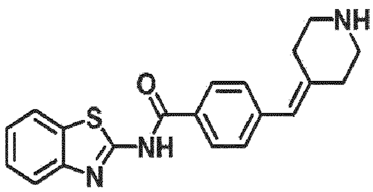

1-65

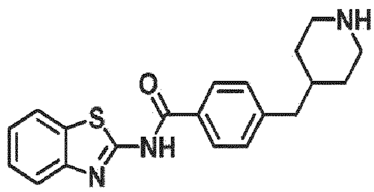

1-66

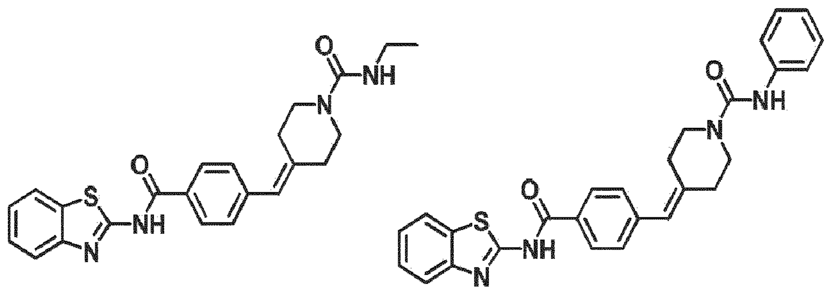

1-67 1-68

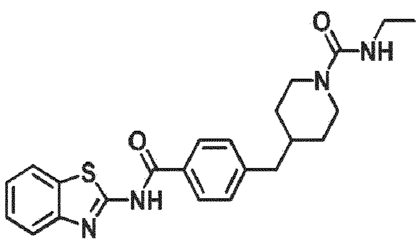

1-69

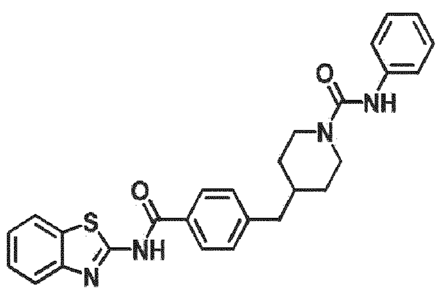

1-70

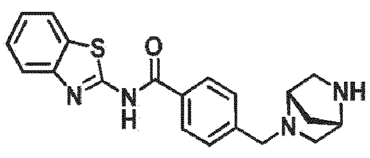

1-71

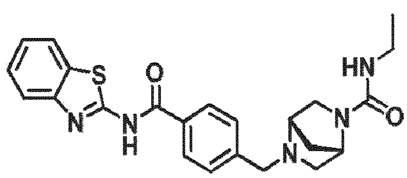

1-72

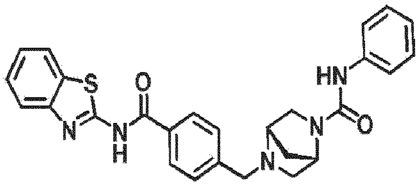

1-73

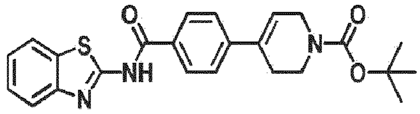

1-74

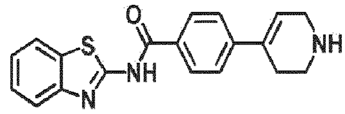

1-75

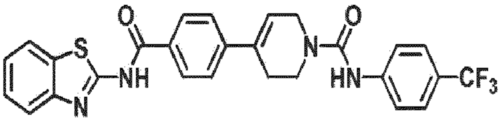

1-76

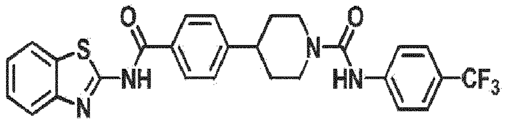

1-77

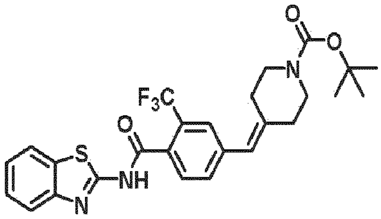

1-78

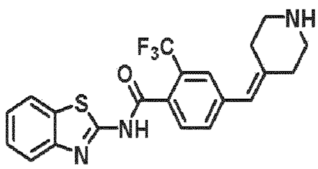

1-79

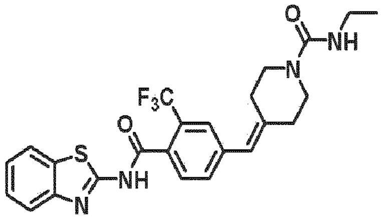

1-80

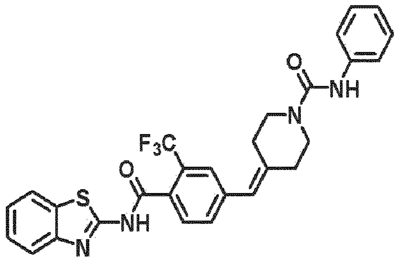

1-81

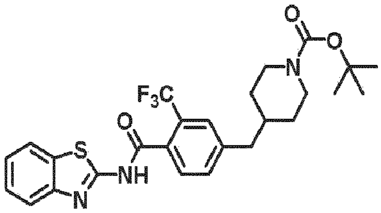

1-82

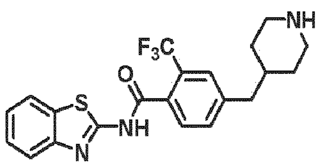

· TFA

1-83

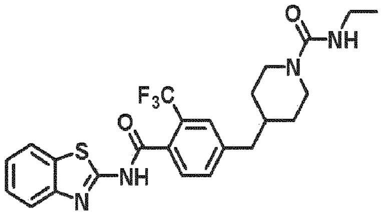

1-84

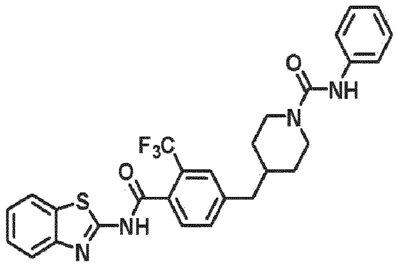

1-85

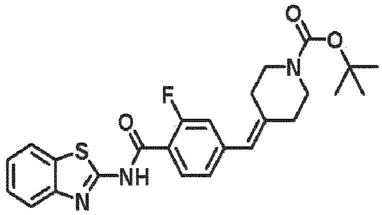

1-86

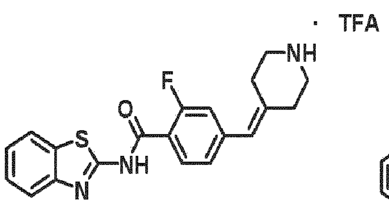

1-87

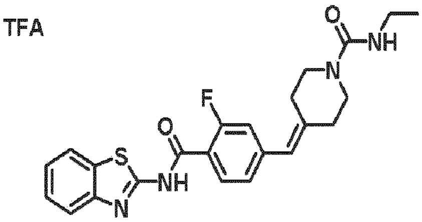

1-88

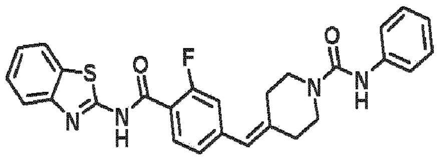

1-89

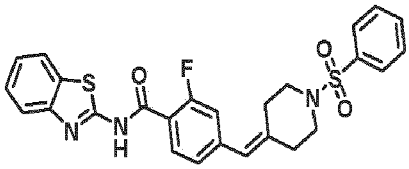

1-90

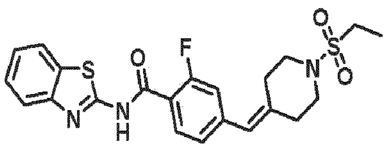

1-91

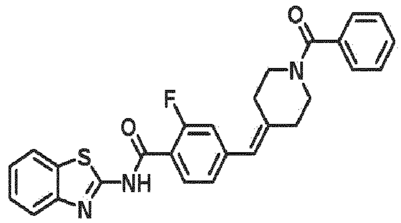

1-92

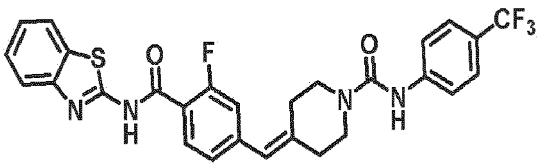

1-93

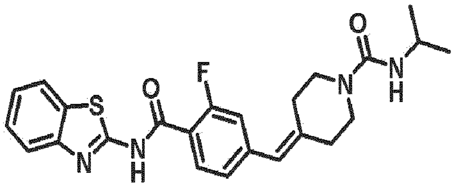

1-94

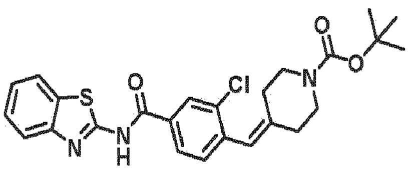

1-95

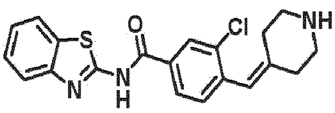

1-96

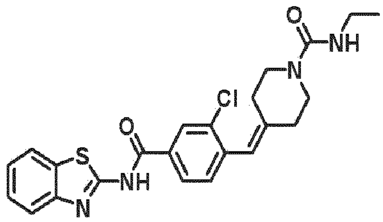

1-97

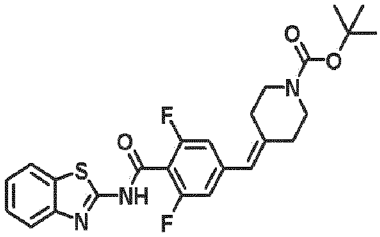

1-98

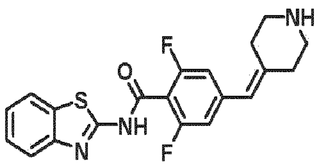

1-99

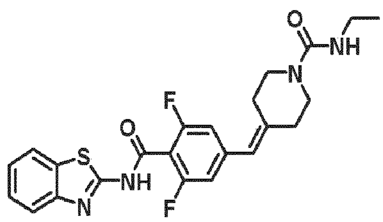

1-100

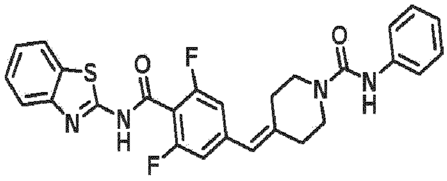

1-101

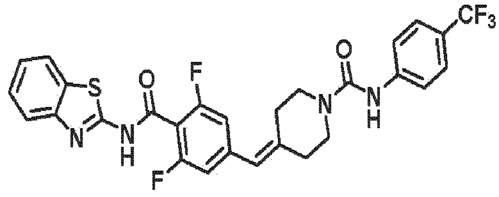

1-102

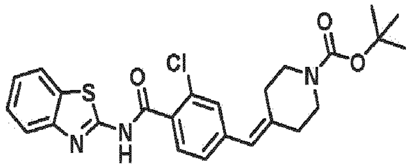

1-103

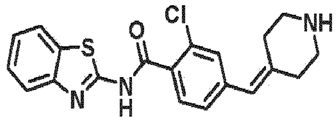

1-104

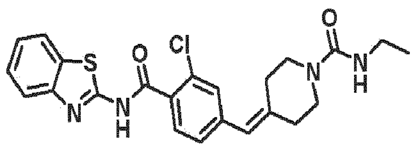

1-105

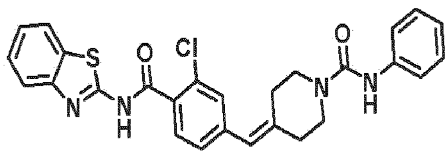

1-106

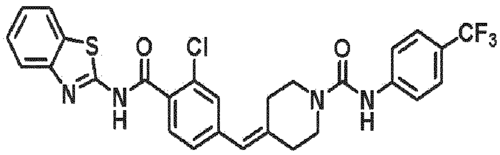

1-107

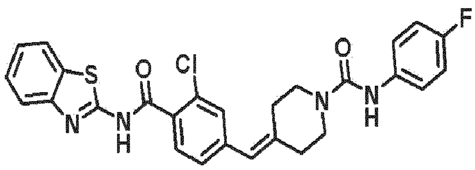

1-108

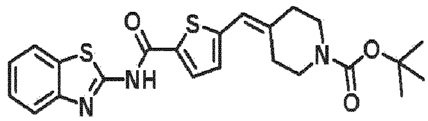

1-109

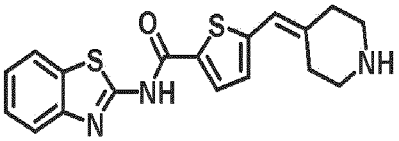

1-110

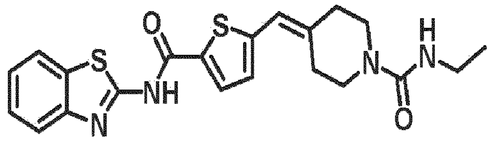

1-111

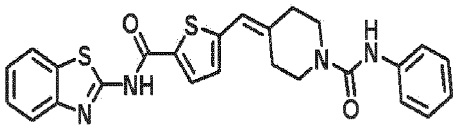

1-112

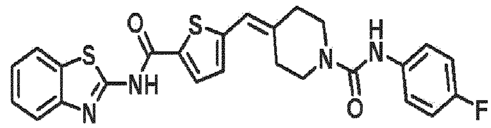

1-113

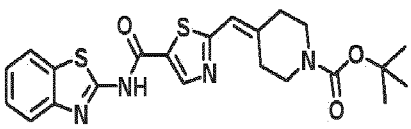

1-114

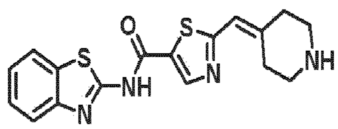

1-115

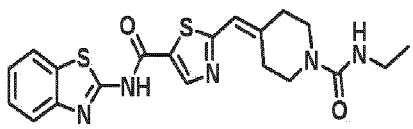

1-116

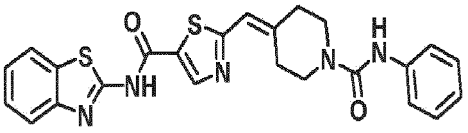

1-117

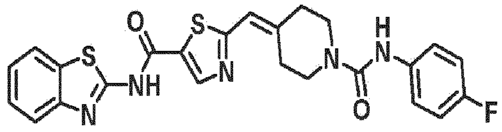

1-118

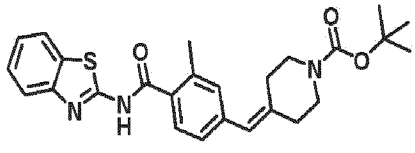

1-119

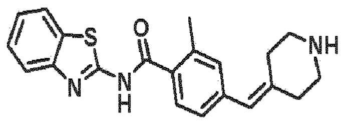

1-120

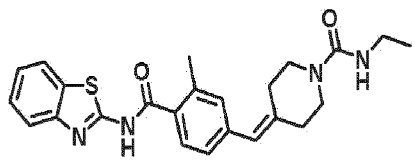

1-121

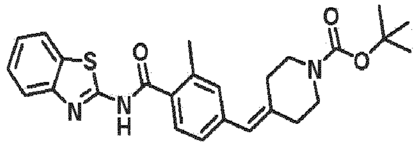

1-119

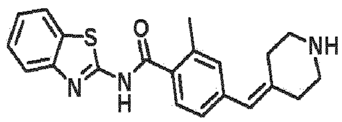

1-120

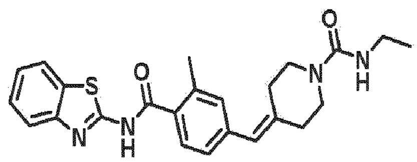

1-121

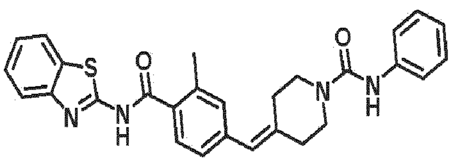

1-122

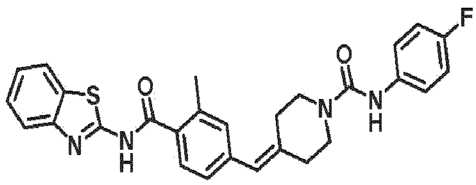

1-123

6. A pharmaceutical composition for preventing or treating of SIRTUIN 7 protein-related diseases as an active ingredient comprising a compound of claim 1, a stereoisomer, tautomer, derivative, hydrate, solvate or pharmaceutically acceptable salt thereof.

7. The pharmaceutical composition of claim 6, wherein the SIRTUIN 7 protein-related diseases are any one selected from the group consisting of obesity, metabolic disorder, glucose resistance, insulin resistance, weight gain, fatty liver, liver fibrosis, hepatitis, liver cirrhosis, mitochondrial myopathy, brain disorder, diabetes, neurodegenerative disease, cardiovascular disease, eye disease, blood clotting disorder, hot flashes, lactic acidosis, MELAS Syndromes (mitochondrial encephalopathy, lactic acidosis, and stroke-like episodes) and cancer.

8. The pharmaceutical composition of claim 7, wherein the cancer comprises gastric cancer, breast cancer, uterine cancer, colon cancer, colorectal cancer, pancreatic cancer, liver cancer, or prostate cancer.

9. The pharmaceutical composition of claim 6, further comprising one or more pharmaceutically acceptable carriers.

10. The pharmaceutical composition of claim 6, wherein the pharmaceutical composition has inhibitory activity against SIRTUIN 7.

Fig. 1a

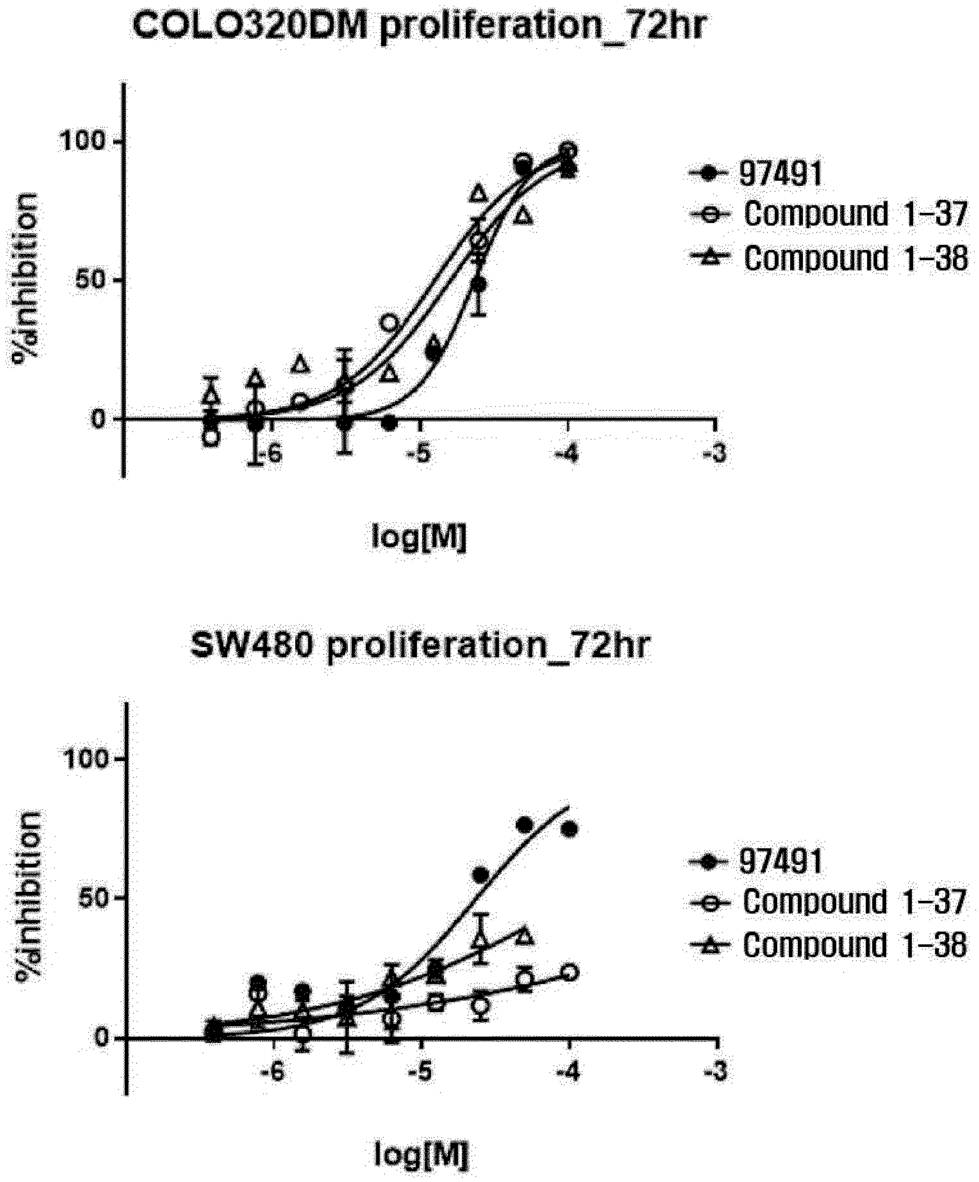
COLO320DM proliferation_72hr
100
50
0
%inhibition
-6
-5
-4
-3
log[M]
97491
Compound 1-37
Compound 1-38
SW480 proliferation_72hr
100
50
0
%inhibition
-6
-5
-4
-3
log[M]
97491
Compound 1-37
Compound 1-38

Fig. 1b

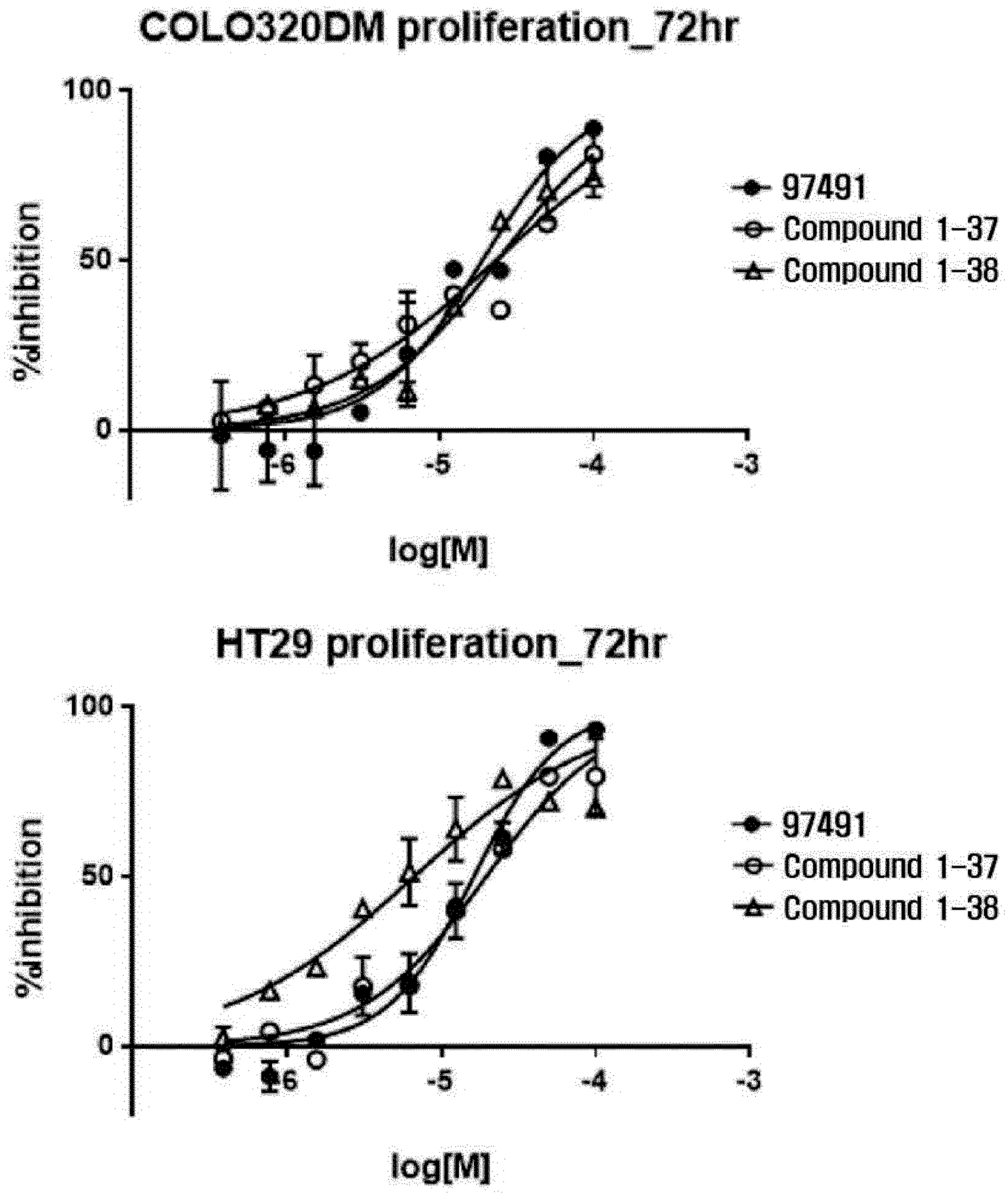
COLO320DM proliferation_72hr
100
50
0
%inhibition
-6
-5
-4
-3
log[M]
97491
Compound 1-37
Compound 1-38
HT29 proliferation_72hr
100
50
0
%inhibition
-6
-5
-4
-3
log[M]
97491
Compound 1-37
Compound 1-38

Fig. 1c

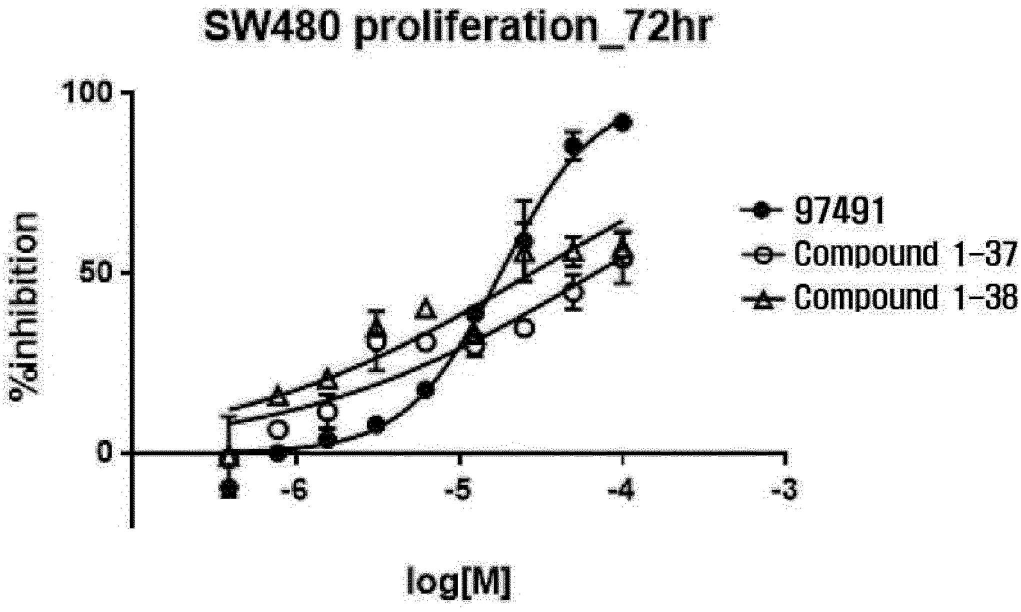
SW480 proliferation_72hr
100
50
0
%inhibition
-6
-5
-4
-3
log[M]
97491
Compound 1-37
Compound 1-38

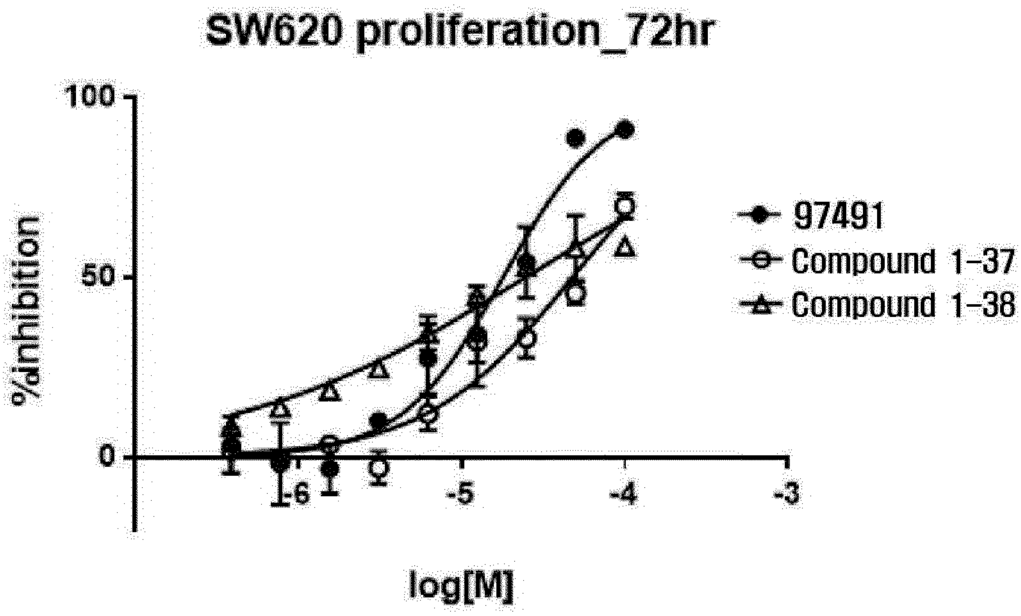
SW620 proliferation_72hr
100
50
0
%inhibition
-6
-5
-4
-3
log[M]
97491
Compound 1-37
Compound 1-38

Fig 2

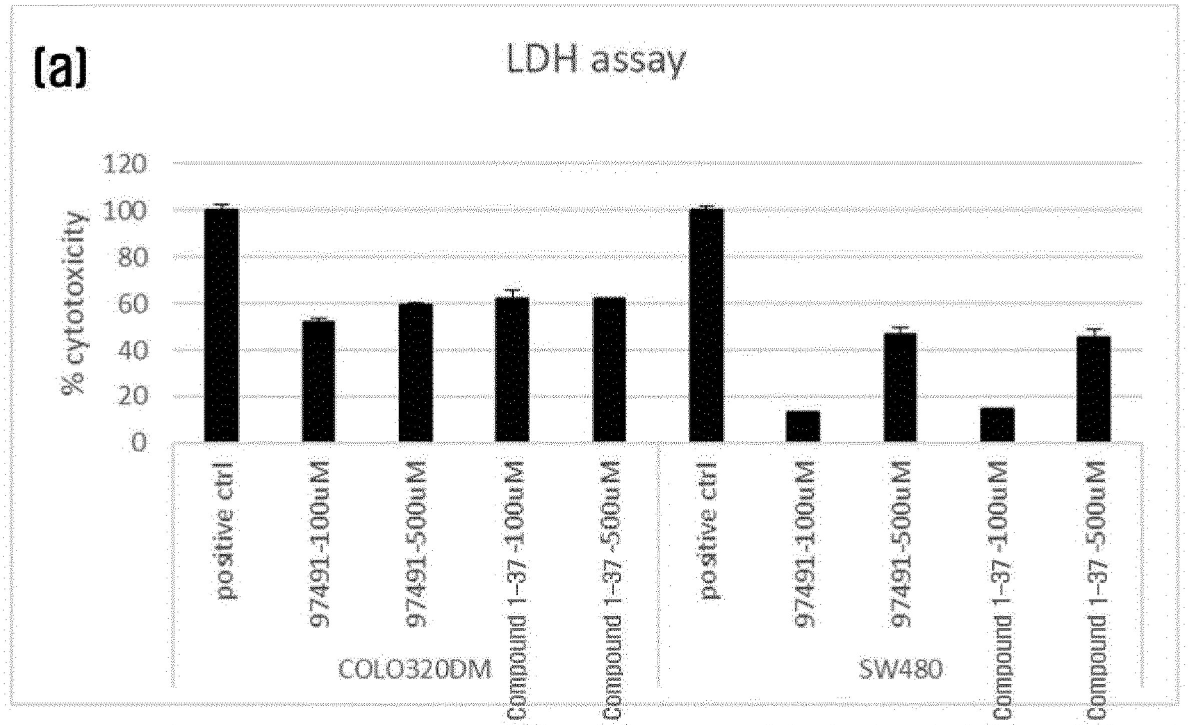
(a)
LDH assay
% cytotoxicity
0
20
40
60
80
100
120
positive ctrl
97491-100uM
97491-500uM
Compound 1-37 -100uM
Compound 1-37 -500uM
COLO320DM
SW480

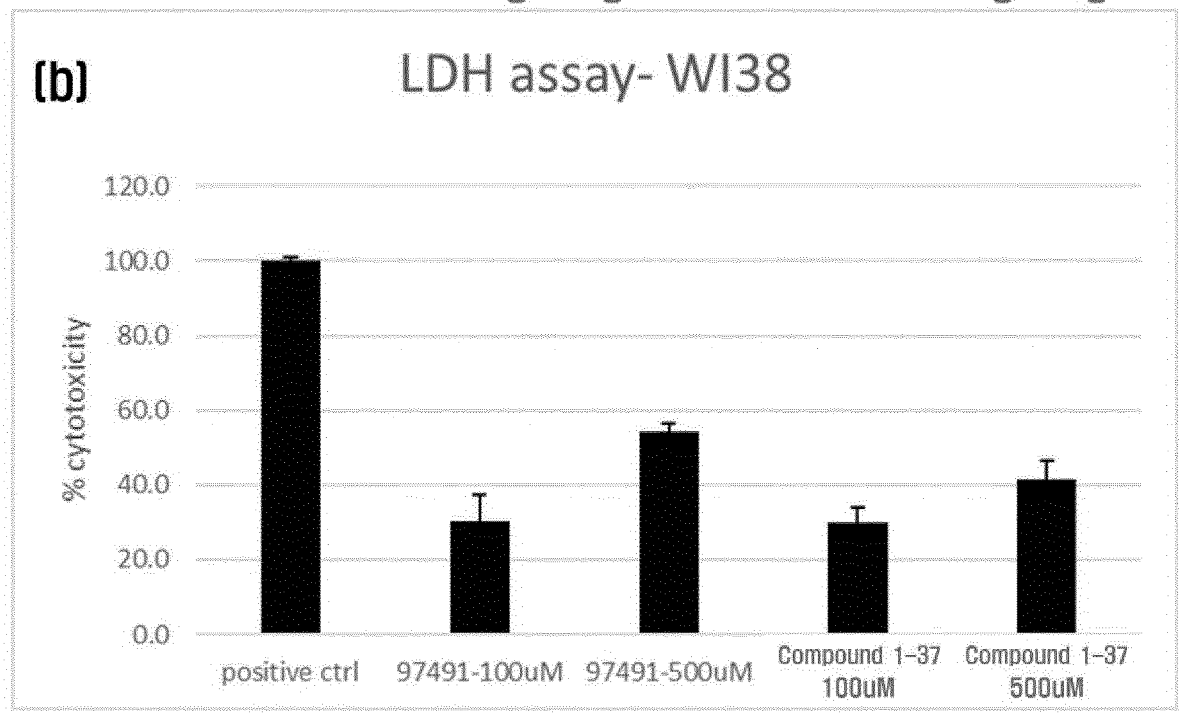
(b)
LDH assay- WI38
% cytotoxicity
0.0
20.0
40.0
60.0
80.0
100.0
120.0
positive ctrl
97491-100uM
97491-500uM
Compound 1-37 100uM
Compound 1-37 500uM

Fig. 3

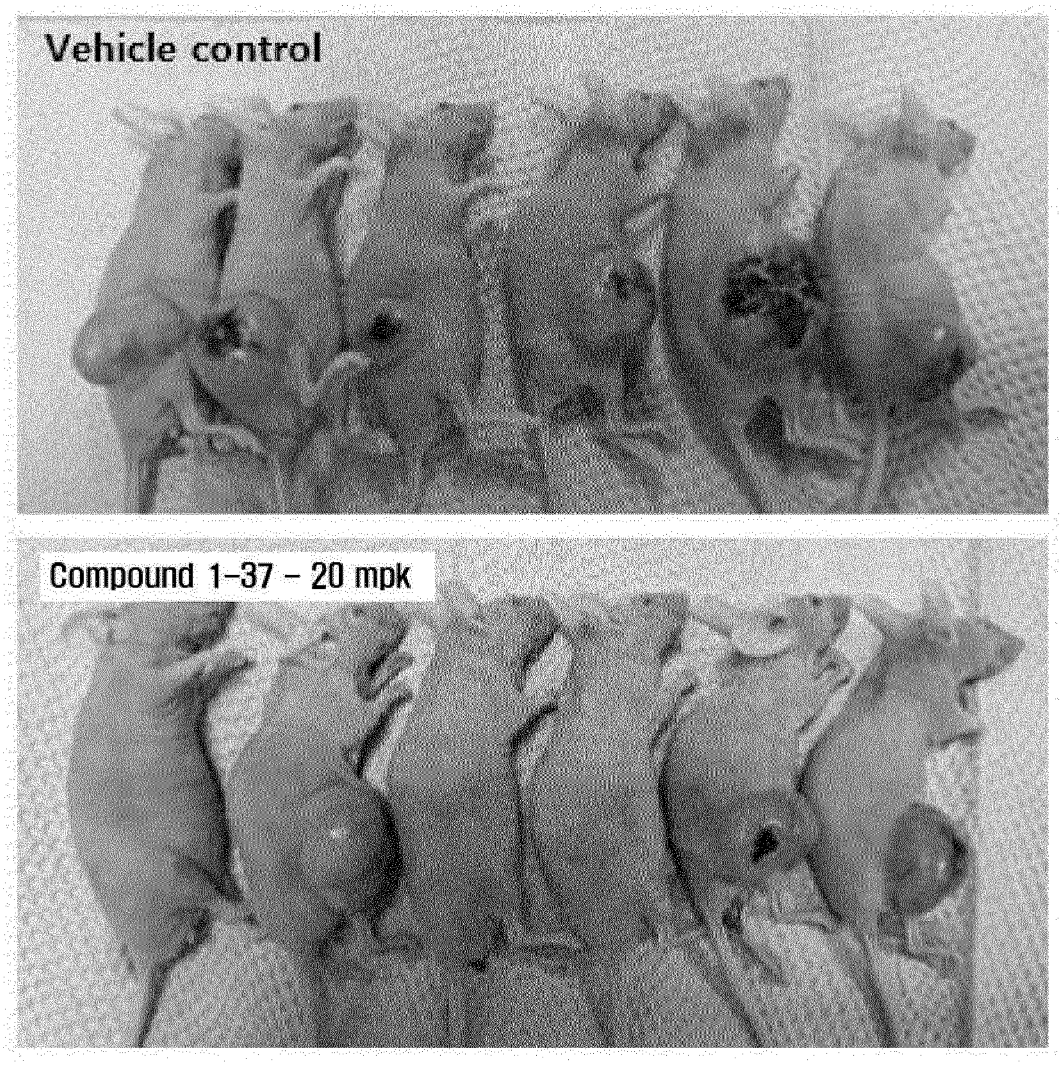

Fig. 4

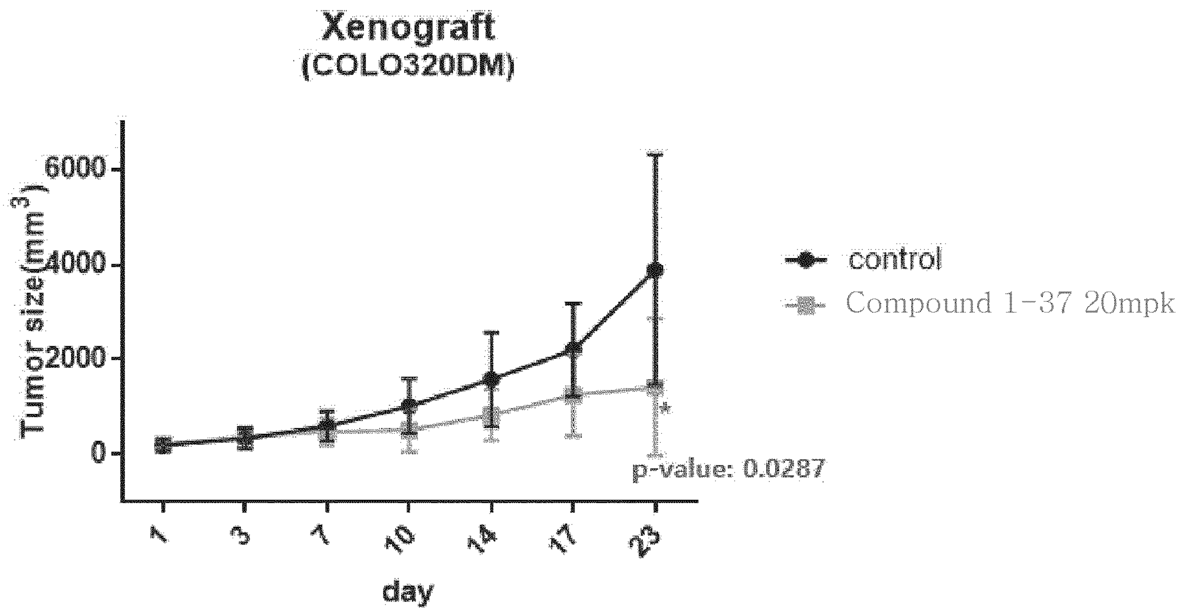
Xenograft
(COLO320DM)
Tumor size(mm$^3$)
6000
4000
2000
0
control
Compound 1-37 20mpk
p-value: 0.0287
1
3
7
10
14
17
23
day

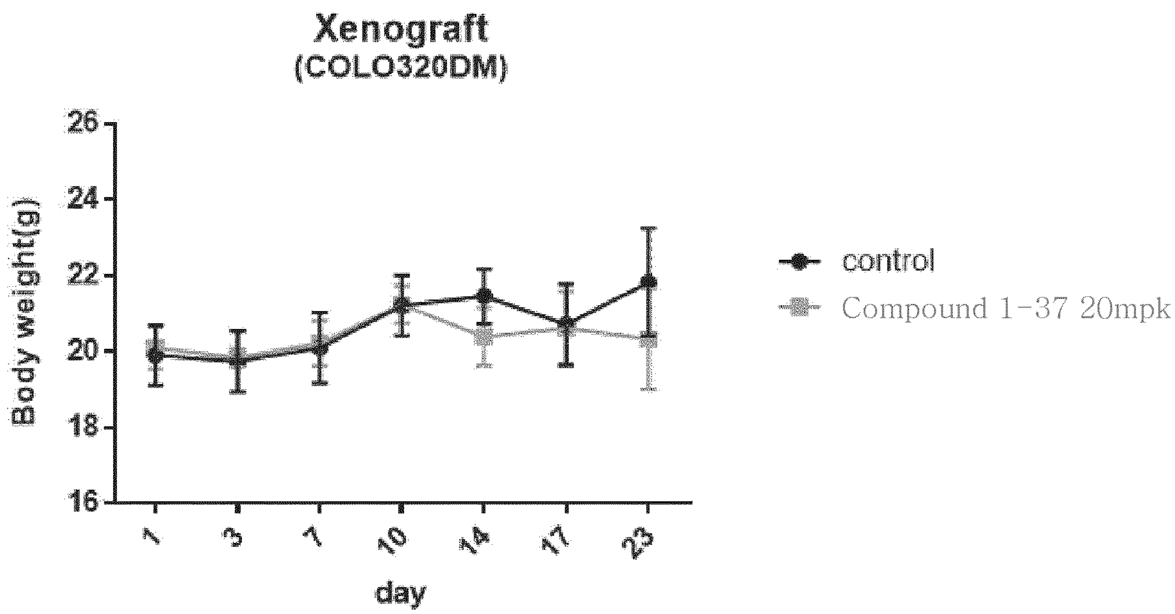
Xenograft
(COLO320DM)
Body weight(g)
26
24
22
20
18
16
control
Compound 1-37 20mpk
1
3
7
10
14
17
23
day

Fig. 5a

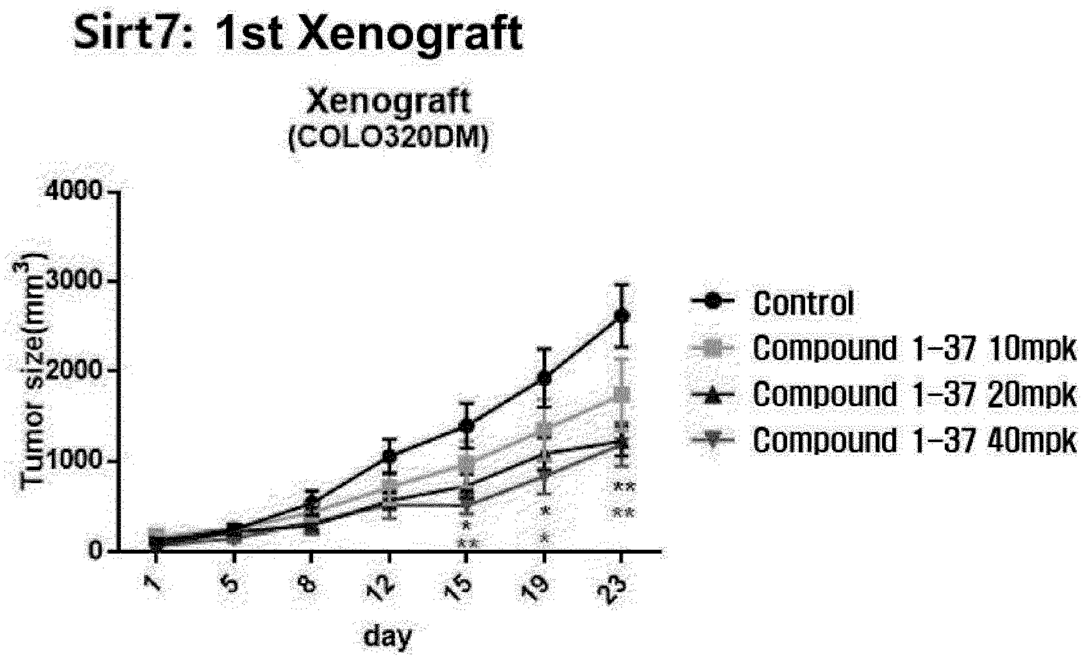


| | | | P-value | Summary |
|---|---|---|---|---|
| Compound 1-37 | 10mpk | 15day | 0.1296 | |
| | 20mpk | | 0.0382 | * |
| | 40mpk | | 0.0077 | ** |
| | 10mpk | 19day | 0.1223 | |
| | 20mpk | | 0.0455 | * |
| | 40mpk | | 0.0158 | * |
| | 10mpk | 22day | 0.0641 | |
| | 20mpk | | 0.0049 | ** |
| | 40mpk | | 0.0073 | ** |

Fig. 5b

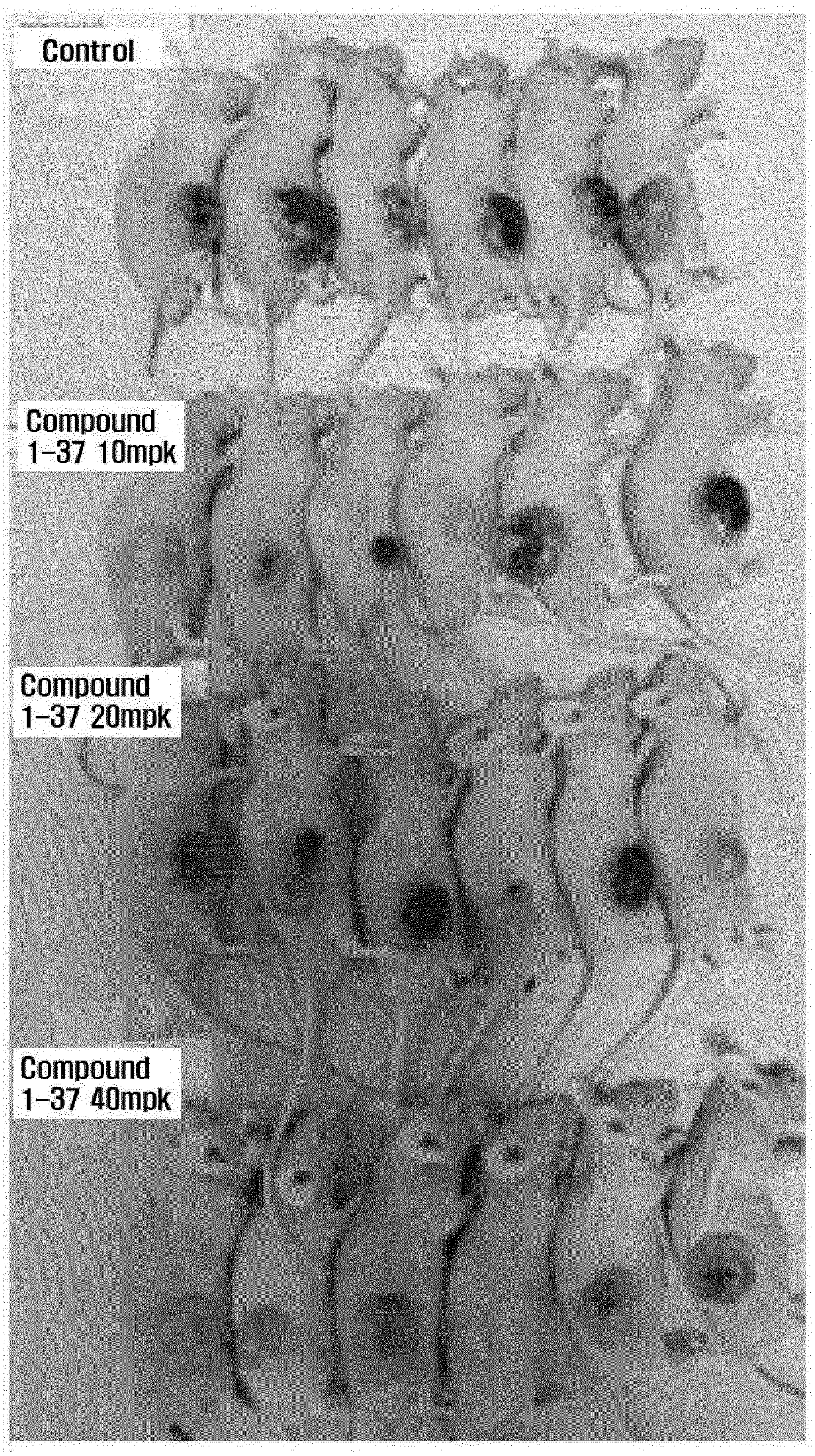
Control
Compound
1-37 10mpk
Compound
1-37 20mpk
Compound
1-37 40mpk

Fig. 6a

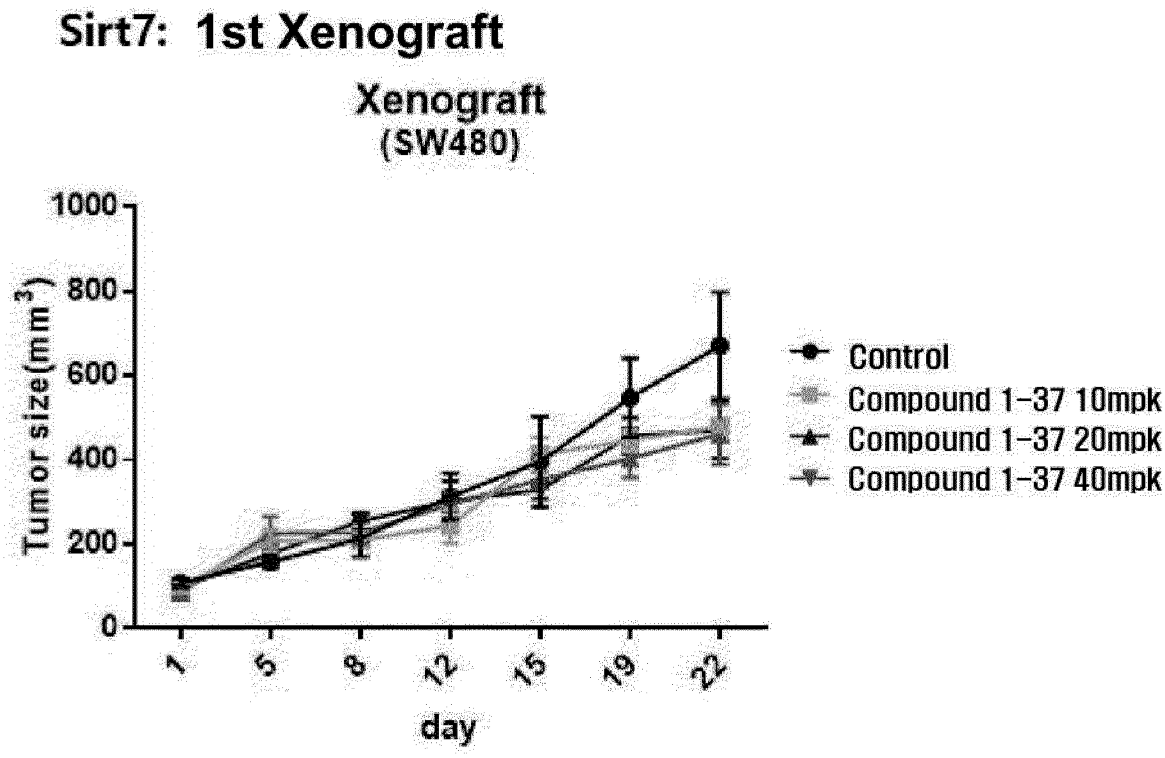


| | | | P-value | Summary |
|---|---|---|---|---|
| Compound 1-37 | 10mpk | 22day | 0.0812 | |
| | 20mpk | | 0.1809 | |
| | 40mpk | | 0.1927 | |

Fig. 6b

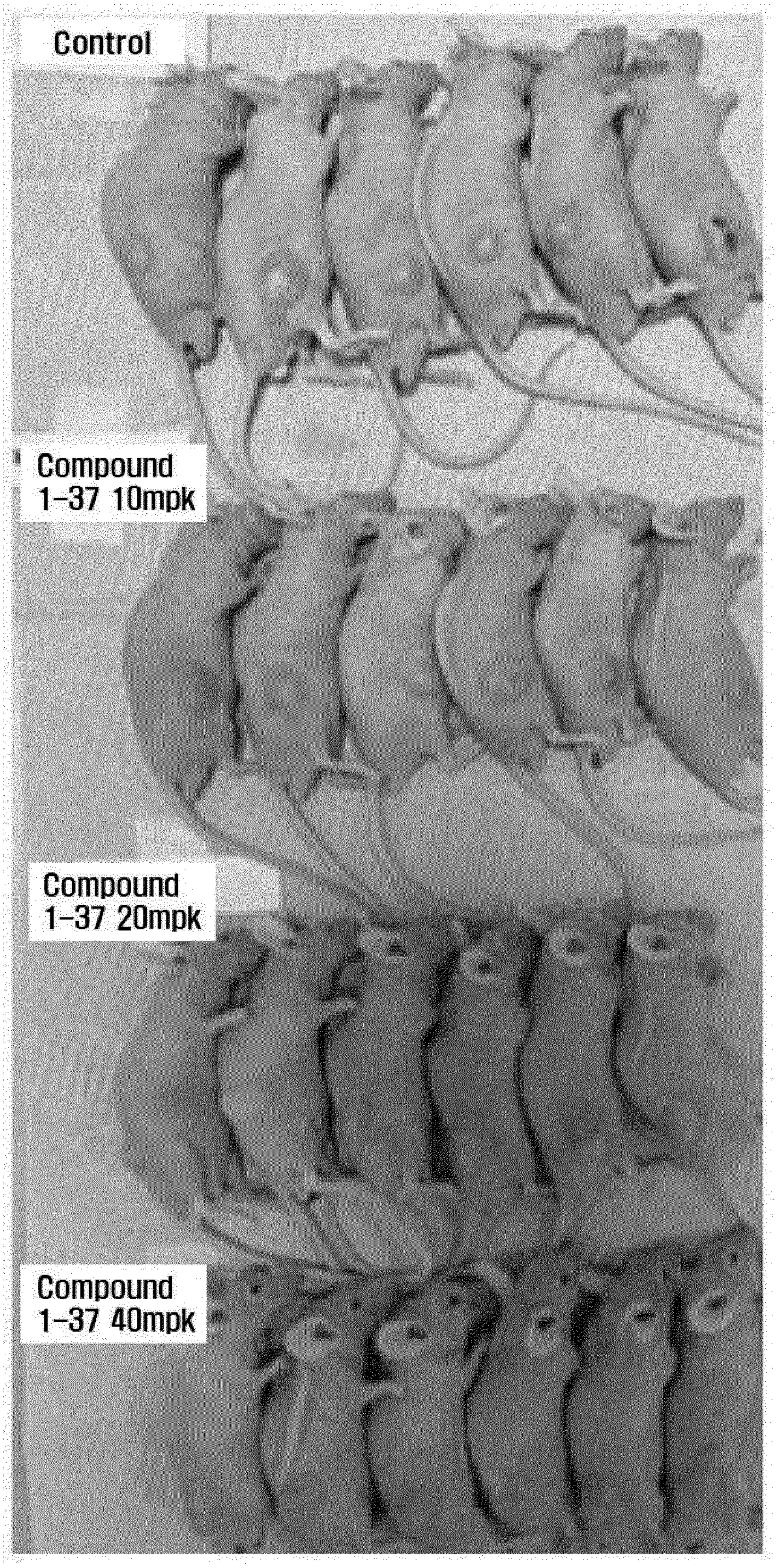
Control
Compound
1-37 10mpk
Compound
1-37 20mpk
Compound
1-37 40mpk

Fig. 7

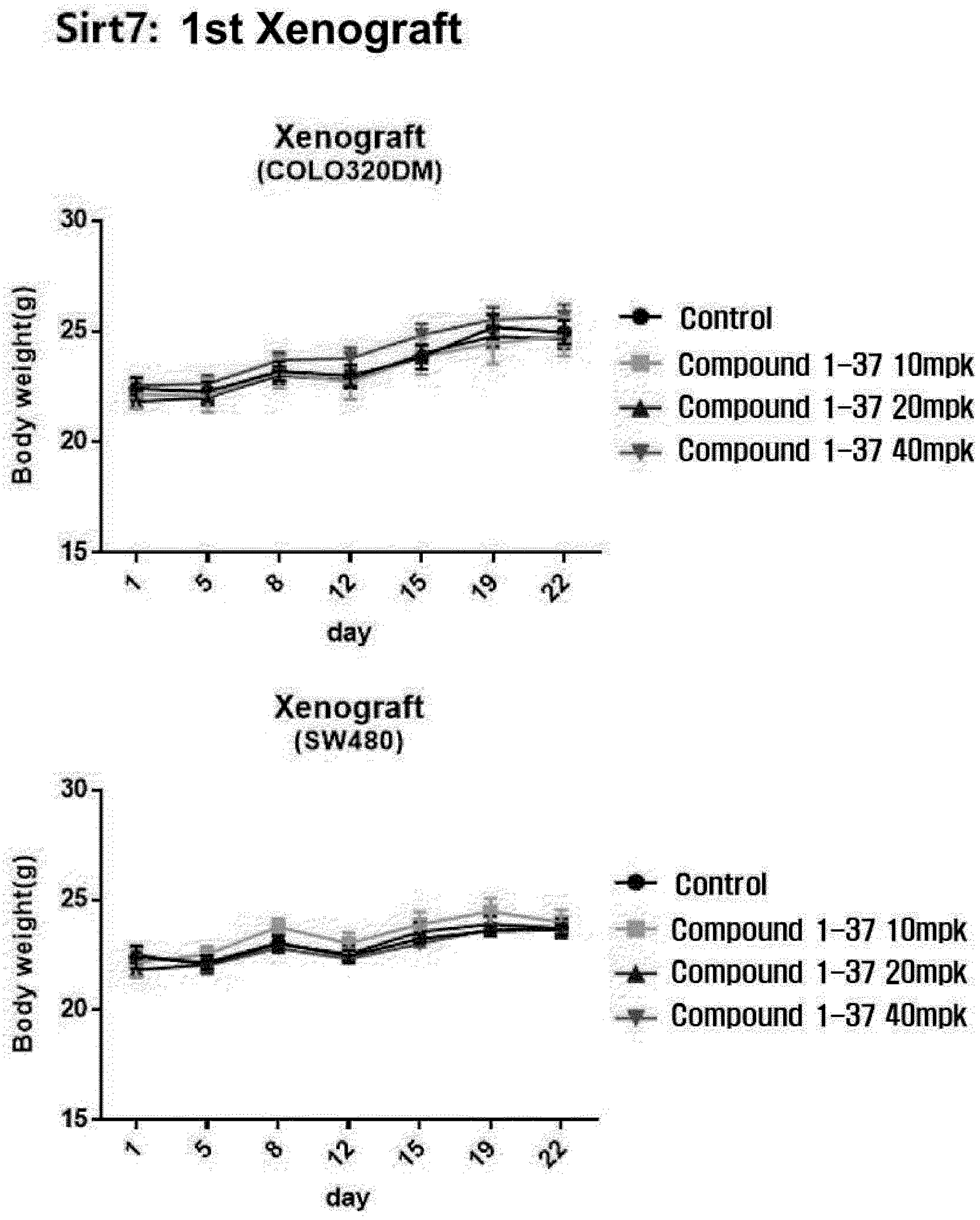
Sirt7: 1st Xenograft
Xenograft
(COLO320DM)
Body weight(g)
30
25
20
15
1
5
8
12
15
19
22
day
Control
Compound 1-37 10mpk
Compound 1-37 20mpk
Compound 1-37 40mpk
Xenograft
(SW480)
Body weight(g)
30
25
20
15
1
5
8
12
15
19
22
day
Control
Compound 1-37 10mpk
Compound 1-37 20mpk
Compound 1-37 40mpk

Fig. 8a

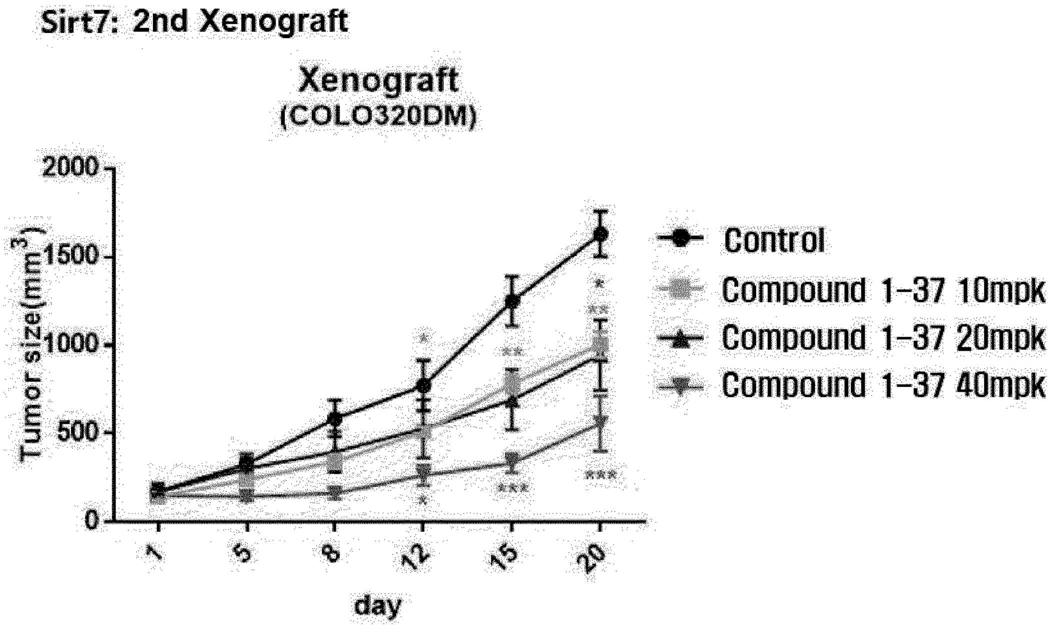


| | | | P-value | Summary |
|---|---|---|---|---|
| Compound 1-37 | 10mpk | 12day | 0.0482 | * |
| | 20mpk | | 0.2928 | |
| | 40mpk | | 0.0112 | * |
| | 10mpk | 15day | 0.0024 | ** |
| | 20mpk | | 0.0871 | |
| | 40mpk | | 0.0003 | *** |
| | 10mpk | 20day | 0.0015 | ** |
| | 20mpk | | 0.0407 | * |
| | 40mpk | | 0.0007 | *** |

Fig. 8b

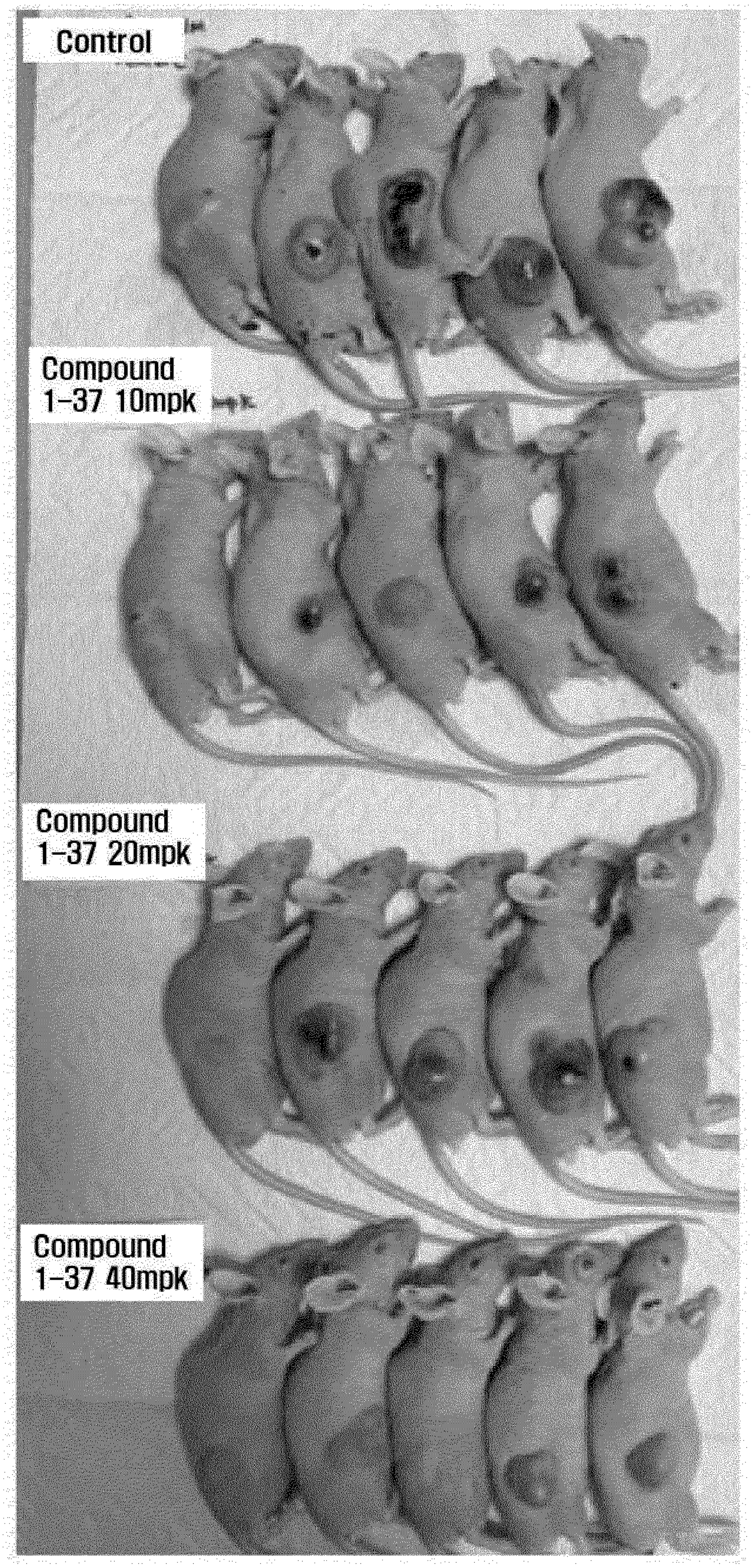
Control
Compound
1-37 10mpk
Compound
1-37 20mpk
Compound
1-37 40mpk

Fig. 9a

Sirt7: 2nd Xenograft

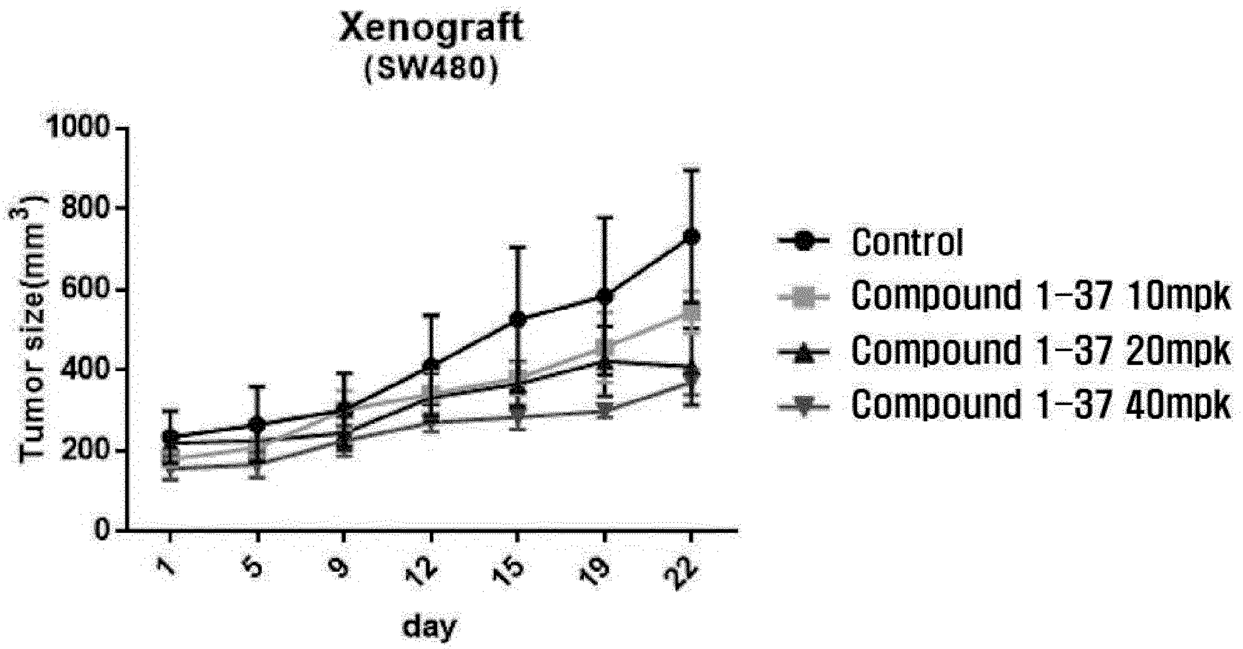


| | | | p-value | Summary |
|---|---|---|---|---|
| Compound 1-37 | 10mpk | 15day | 0.2256 | |
| | 20mpk | | 0.4209 | |
| | 40mpk | | 0.2210 | |
| | 10mpk | 19day | 0.2837 | |
| | 20mpk | | 0.4704 | |
| | 40mpk | | 0.1829 | |
| | 10mpk | 22day | 0.1529 | |
| | 20mpk | | 0.1263 | |
| | 40mpk | | 0.0616 | |

Fig. 9b

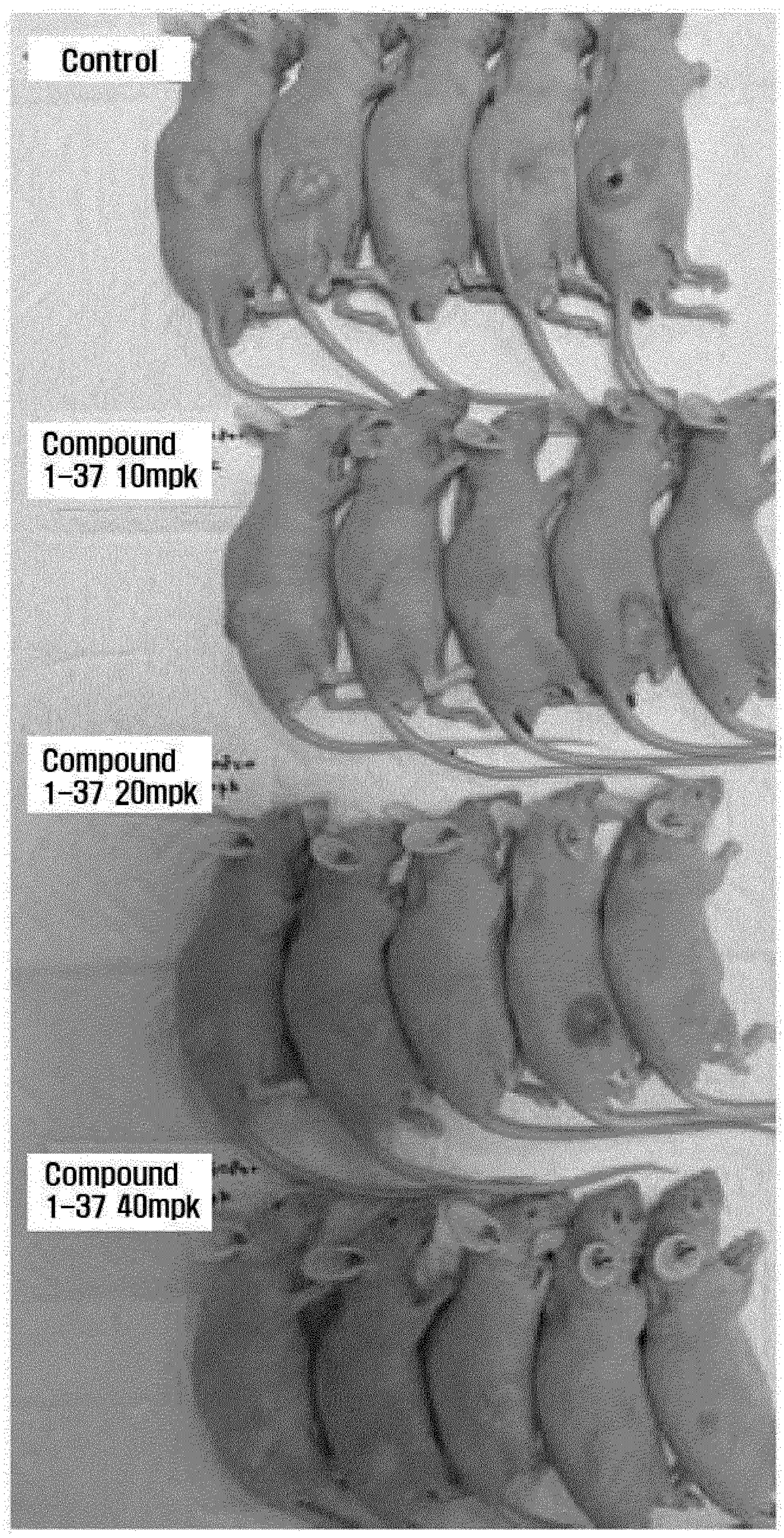
Control
Compound
1-37 10mpk
Compound
1-37 20mpk
Compound
1-37 40mpk

Fig. 10

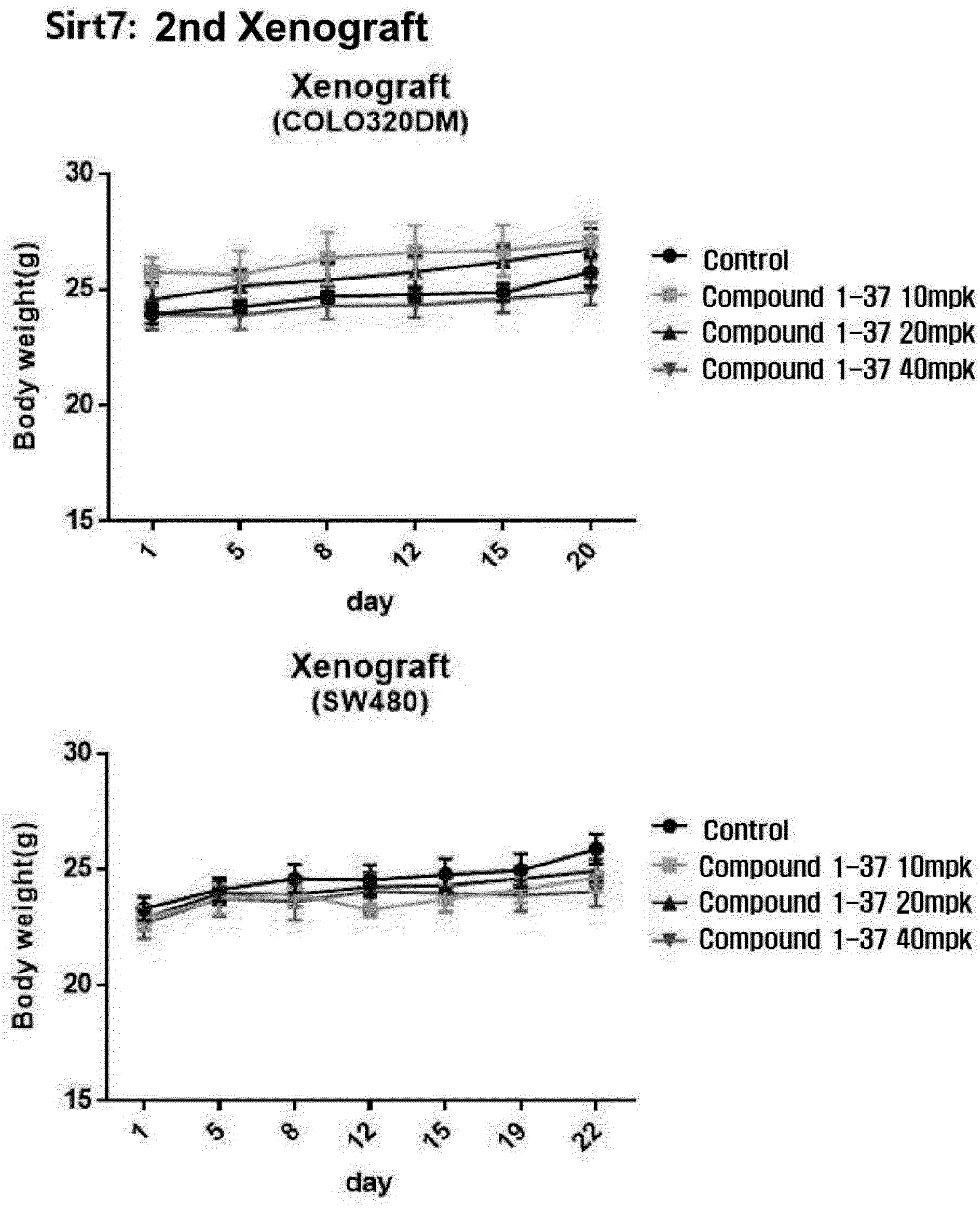
Sirt7: 2nd Xenograft
Xenograft
(COLO320DM)
30
25
20
15
Body weight(g)
1
5
8
12
15
20
day
Control
Compound 1-37 10mpk
Compound 1-37 20mpk
Compound 1-37 40mpk
Xenograft
(SW480)
30
25
20
15
Body weight(g)
1
5
8
12
15
19
22
day
Control
Compound 1-37 10mpk
Compound 1-37 20mpk
Compound 1-37 40mpk

Fig. 11a

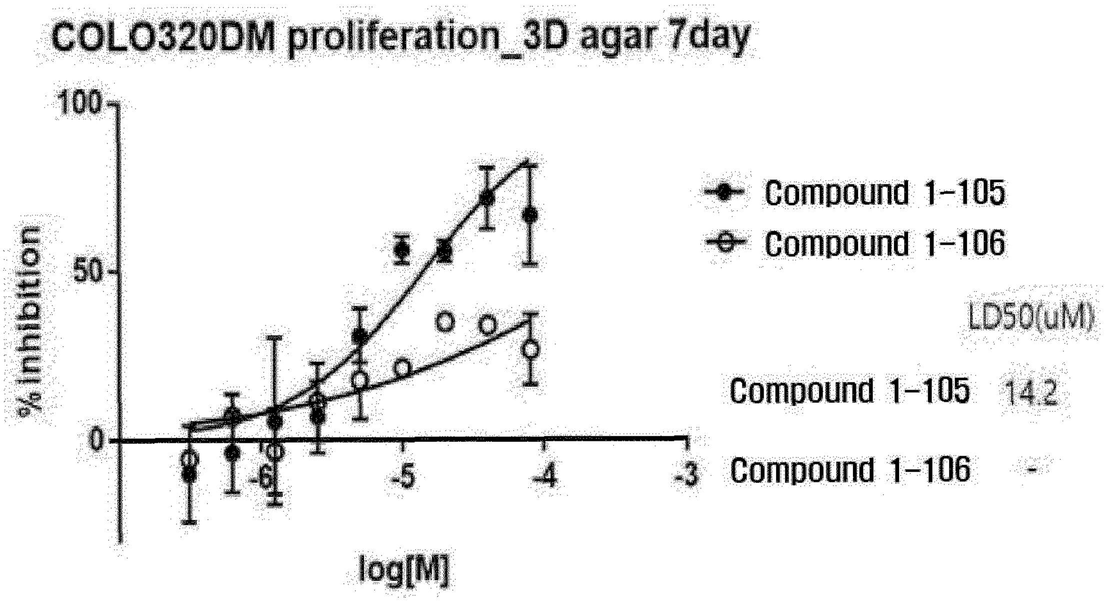
COLO320DM proliferation_3D agar 7day
100
50
0
% inhibition
-6
-5
-4
-3
log[M]
Compound 1-105
Compound 1-106
LD50(uM)
Compound 1-105 14.2
Compound 1-106 -

Fig. 11b

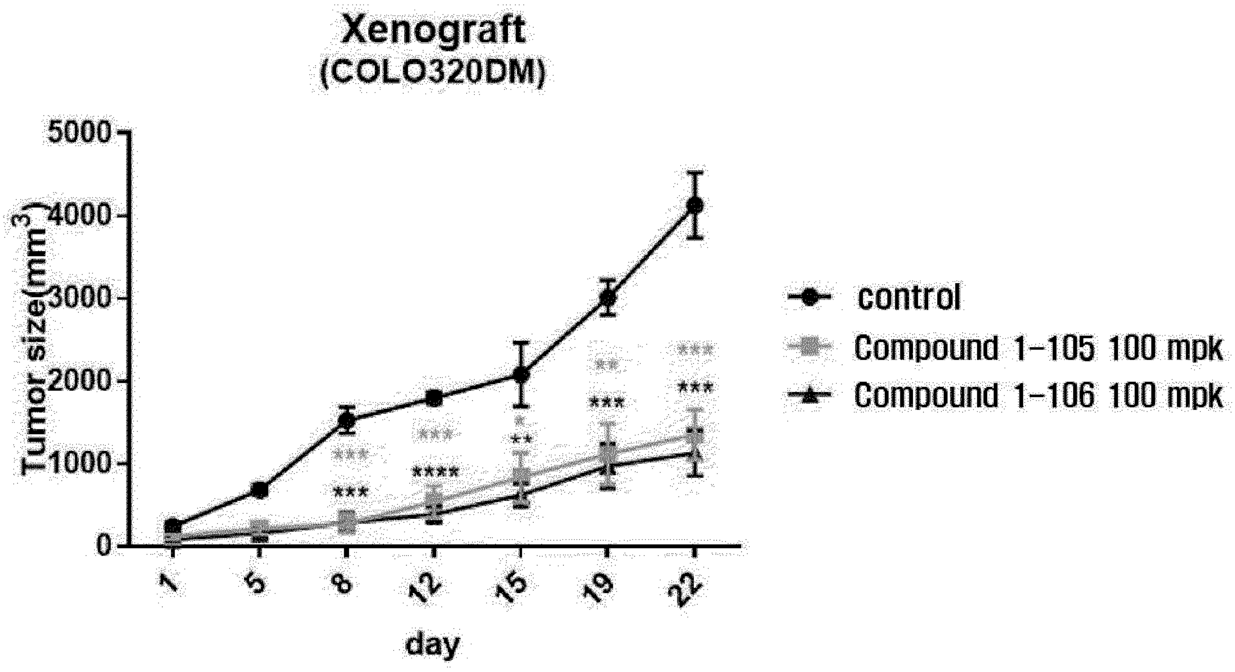
Xenograft
(COLO320DM)
Tumor size(mm³)
5000
4000
3000
2000
1000
0
1
5
8
12
15
19
22
day
control
Compound 1-105 100 mpk
Compound 1-106 100 mpk
***
***
***
****
*
**
**
***
***
***

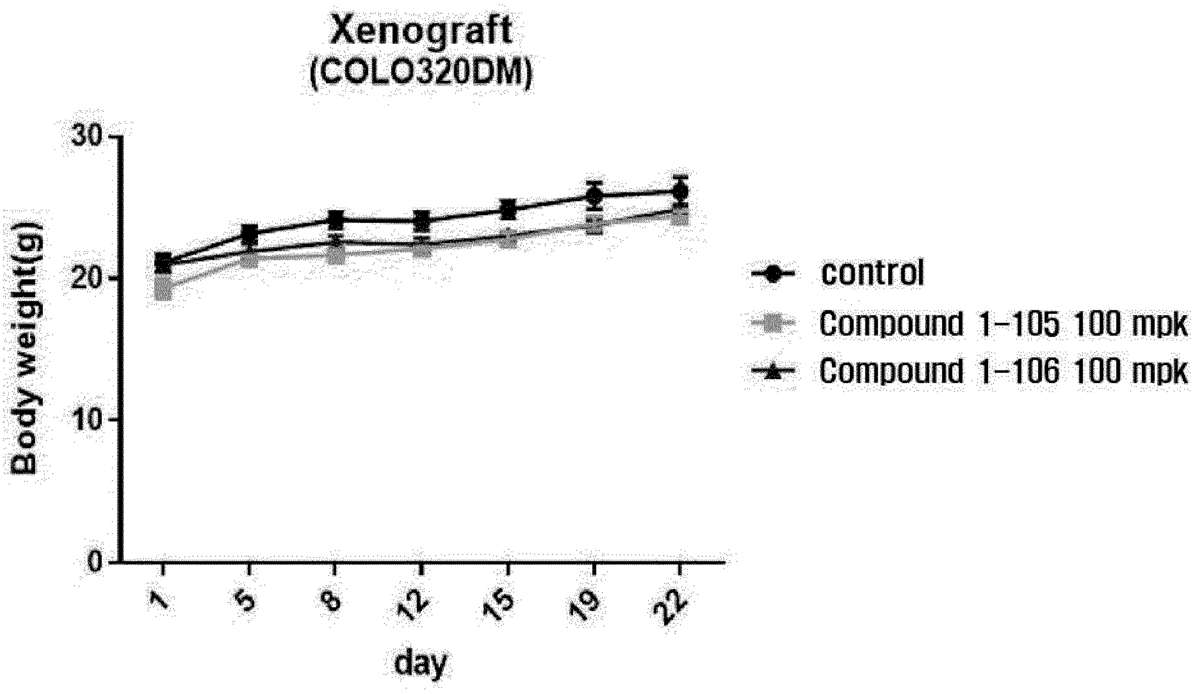
Xenograft
(COLO320DM)
Body weight(g)
30
20
10
0
1
5
8
12
15
19
22
day
control
Compound 1-105 100 mpk
Compound 1-106 100 mpk

Fig. 11c

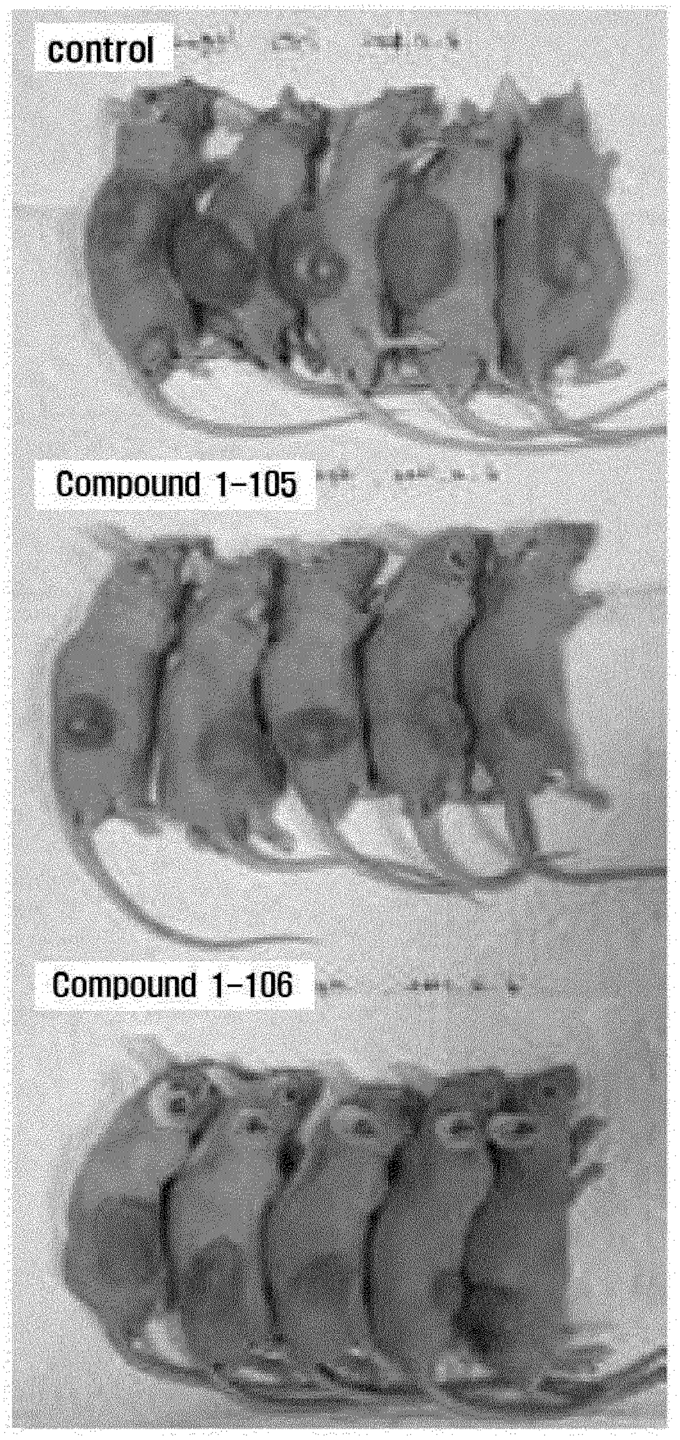

**INTERNATIONAL SEARCH REPORT**

International application No.
**PCT/KR2022/001117**

**A. CLASSIFICATION OF SUBJECT MATTER**

**C07D 235/30**(2006.01)i; **A61K 31/496**(2006.01)i; **A61K 31/501**(2006.01)i; **A61K 31/506**(2006.01)i; **A61K 31/4355**(2006.01)i; **A61P 35/00**(2006.01)i; **C07D 235/32**(2006.01)i; **C07D 403/12**(2006.01)i; **C07D 417/12**(2006.01)i; **C07D 277/82**(2006.01)i

According to International Patent Classification (IPC) or to both national classification and IPC

**B. FIELDS SEARCHED**

Minimum documentation searched (classification system followed by classification symbols)

C07D 235/30(2006.01); A01N 43/78(2006.01); A61K 31/415(2006.01); A61K 31/4172(2006.01); A61K 31/4184(2006.01); A61K 31/425(2006.01); A61K 31/428(2006.01); A61K 31/4439(2006.01); C07D 401/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 벤조티아졸(benzothiazole), 벤즈이미다졸(benzimidazole), SIRTUIN 단백질(SIRTUIN protein), 당뇨병(diabetes), 암(cancer)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2005-0043912 A (F. HOFFMANN-LA ROCHE AG) 11 May 2005 (2005-05-11)<br>See claim 1; paragraphs [0038], [0302], [0627] and [0628]; and example 104. | 1-7,9,10<br>8 |
| Y | KR 10-2020-0043433 A (MERCK PATENT GMBH) 27 April 2020 (2020-04-27)<br>See claim 17; paragraphs [0189], [0191] and [0320]; and table 2. | 8 |
| A | KR 10-2015-0101656 A (KOREA RESEARCH INSTITUTE OF CHEMICAL TECHNOLOGY) 04 September 2015 (2015-09-04)<br>See claims 1, 2 and 4. | 1-10 |
| A | WO 2010-024903 A1 (FENG, Y. et al.) 04 March 2010 (2010-03-04)<br>See entire document. | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 May 2022** | **04 May 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2022/001117**

**C. DOCUMENTS CONSIDERED TO BE RELEVANT**

| Category* | Citation of document, with indication, where appropriate, of the relevant passages | Relevant to claim No. |
|---|---|---|
| A | US 2010-0196368 A1 (AMIRI, P. et al.) 05 August 2010 (2010-08-05)<br>See entire document. | 1-10 |

**INTERNATIONAL SEARCH REPORT**
**Information on patent family members**

International application No.
**PCT/KR2022/001117**

| Patent document cited in search report | Publication date (day/month/year) | Patent family member(s) | Publication date (day/month/year) |
|---|---|---|---|
| KR 10-2005-0043912 A | 11 May 2005 | AR 031244 A1 | 17 September 2003 |
| | | AT 385794 T | 15 March 2008 |
| | | AU 2001-281817 B2 | 24 November 2005 |
| | | AU 8181701 A | 02 January 2002 |
| | | BR 0112395 A | 08 July 2003 |
| | | CA 2413086 A1 | 27 December 2001 |
| | | CA 2413086 C | 28 June 2011 |
| | | CN 1437471 A | 20 August 2003 |
| | | CN 1437471 C | 04 January 2006 |
| | | CY 1107401 T1 | 19 December 2012 |
| | | CZ 2003182 A3 | 18 June 2003 |
| | | DE 60132777 T2 | 12 February 2009 |
| | | DK 1303272 T3 | 26 May 2008 |
| | | EC SP024399 A | 06 February 2003 |
| | | EP 1303272 A2 | 23 April 2003 |
| | | EP 1303272 B1 | 13 February 2008 |
| | | EP 1797878 A2 | 20 June 2007 |
| | | EP 1797878 A3 | 20 January 2010 |
| | | ES 2299504 T3 | 01 June 2008 |
| | | HK 1058148 A1 | 07 May 2004 |
| | | HR P20020962 A2 | 28 February 2005 |
| | | HU 0301315 A2 | 28 August 2003 |
| | | HU 0301315 A3 | 29 October 2007 |
| | | IL 153278 A | 31 December 2012 |
| | | JO 2503 B1 | 05 October 2009 |
| | | JP 2003-535887 A | 02 December 2003 |
| | | JP 3886897 B2 | 28 February 2007 |
| | | KR 10-0571161 B1 | 17 April 2006 |
| | | KR 10-2003-0019446 A | 06 March 2003 |
| | | MX PA02012596 A | 10 April 2003 |
| | | MY 138352 A | 29 May 2009 |
| | | NO 20025978 L | 12 December 2002 |
| | | NO 20073465 L | 12 December 2002 |
| | | NO 324635 B1 | 26 November 2007 |
| | | NZ 522928 A | 27 May 2005 |
| | | PE 20020104 A1 | 16 February 2002 |
| | | PL 207384 B1 | 31 December 2010 |
| | | PL 360697 A1 | 20 September 2004 |
| | | PT 1303272 E | 14 April 2008 |
| | | RU 2251419 C2 | 10 May 2005 |
| | | SI 1303272 T1 | 30 June 2008 |
| | | US 2002-0045615 A1 | 18 April 2002 |
| | | US 2003-0125318 A1 | 03 July 2003 |
| | | US 2003-0176695 A1 | 18 September 2003 |
| | | US 2005-0026906 A1 | 03 February 2005 |
| | | US 2006-0003986 A1 | 05 January 2006 |
| | | US 2008-0108809 A1 | 08 May 2008 |
| | | US 2008-0125419 A1 | 29 May 2008 |
| | | US 2010-0075959 A1 | 25 March 2010 |
| | | US 6521754 B2 | 18 February 2003 |

Form PCT/ISA/210 (patent family annex) (July 2019)

**INTERNATIONAL SEARCH REPORT**
**Information on patent family members**

International application No.
**PCT/KR2022/001117**

| Patent document cited in search report | Publication date (day/month/year) | Patent family member(s) | Publication date (day/month/year) |
|---|---|---|---|
| | | US 6835732 B2 | 28 December 2004 |
| | | US 6963000 B2 | 08 November 2005 |
| | | US 7317007 B2 | 08 January 2008 |
| | | UY 26782 A1 | 28 December 2001 |
| | | WO 01-097786 A3 | 12 December 2002 |
| | | WO 01-97786 A2 | 27 December 2001 |
| | | YU 96502 A | 16 January 2006 |
| | | ZA 200209730 B | 01 March 2004 |
| KR 10-2020-0043433 A | 27 April 2020 | AU 2018-320673 A1 | 02 April 2020 |
| | | BR 112020003594 A2 | 01 September 2020 |
| | | CA 3073333 A1 | 28 February 2019 |
| | | CN 110997662 A | 10 April 2020 |
| | | EP 3672952 A1 | 01 July 2020 |
| | | JP 2020-531531 A | 05 November 2020 |
| | | RU 2020110636 A | 23 September 2021 |
| | | RU 2020110636 A3 | 08 November 2021 |
| | | SG 11202001337 A | 30 March 2020 |
| | | TW 201920119 A | 01 June 2019 |
| | | US 2020-0207720 A1 | 02 July 2020 |
| | | WO 2019-038215 A1 | 28 February 2019 |
| KR 10-2015-0101656 A | 04 September 2015 | KR 10-1596276 B1 | 22 February 2016 |
| WO 2010-024903 A1 | 04 March 2010 | None | |
| US 2010-0196368 A1 | 05 August 2010 | AP 1913 A | 31 October 2008 |
| | | AT 447404 T | 15 November 2009 |
| | | AU 2003-226211 A1 | 13 October 2003 |
| | | AU 2003-226211 B2 | 29 May 2008 |
| | | AU 2004-277405 A1 | 14 April 2005 |
| | | BR 0308854 A | 22 February 2005 |
| | | BR PI0414908 A | 07 November 2006 |
| | | CA 2480638 A1 | 09 October 2003 |
| | | CA 2480638 C | 12 February 2013 |
| | | CA 2539748 A1 | 14 April 2005 |
| | | CN 100515419 C | 22 July 2009 |
| | | CN 1655779 A | 17 August 2005 |
| | | CN 1913884 A | 14 February 2007 |
| | | CO 5611108 A2 | 28 February 2006 |
| | | CY 1109764 T1 | 10 September 2014 |
| | | DK 1499311 T3 | 08 March 2010 |
| | | EA 007987 B1 | 27 February 2007 |
| | | EA 011890 B1 | 30 June 2009 |
| | | EA 200401284 A1 | 28 April 2005 |
| | | EA 200600689 A1 | 25 August 2006 |
| | | EC SP045407 A | 03 January 2005 |
| | | EP 1499311 A1 | 26 January 2005 |
| | | EP 1499311 B1 | 04 November 2009 |
| | | EP 1675584 A1 | 05 July 2006 |
| | | ES 2336094 T3 | 08 April 2010 |
| | | HK 1067945 A1 | 22 April 2005 |
| | | HK 1078779 A1 | 24 March 2006 |
| | | IL 164302 A | 30 December 2010 |

Form PCT/ISA/210 (patent family annex) (July 2019)

**INTERNATIONAL SEARCH REPORT**
**Information on patent family members**

International application No.
**PCT/KR2022/001117**

| Patent document cited in search report | Publication date (day/month/year) | Patent family member(s) | Publication date (day/month/year) |
|---|---|---|---|
| | | JP 2005-529089 A | 29 September 2005 |
| | | JP 2006-193533 A | 27 July 2006 |
| | | JP 2007-507428 A | 29 March 2007 |
| | | JP 2010-275317 A | 09 December 2010 |
| | | JP 4628678 B2 | 09 February 2011 |
| | | JP 5265629 B2 | 14 August 2013 |
| | | KR 10-1071123 B1 | 07 October 2011 |
| | | KR 10-2004-0095355 A | 12 November 2004 |
| | | KR 10-2006-0089232 A | 08 August 2006 |
| | | KR 10-2011-0015702 A | 16 February 2011 |
| | | MX PA04009541 A | 25 January 2005 |
| | | MX PA06003435 A | 20 June 2006 |
| | | NO 20044617 L | 28 December 2004 |
| | | NO 330563 B1 | 16 May 2011 |
| | | NZ 535985 A | 27 April 2007 |
| | | PL 214800 B1 | 30 September 2013 |
| | | PL 372852 A1 | 08 August 2005 |
| | | PL 402389 A1 | 02 April 2013 |
| | | PT 1499311 E | 10 March 2010 |
| | | SI 1499311 T1 | 31 March 2010 |
| | | US 2004-0087626 A1 | 06 May 2004 |
| | | US 2004-0122237 A1 | 24 June 2004 |
| | | US 2007-0299039 A1 | 27 December 2007 |
| | | US 2012-0288501 A1 | 15 November 2012 |
| | | US 7071216 B2 | 04 July 2006 |
| | | US 7728010 B2 | 01 June 2010 |
| | | US 8299108 B2 | 30 October 2012 |
| | | US 8614330 B2 | 24 December 2013 |
| | | WO 03-082272 A1 | 09 October 2003 |
| | | WO 2005-032548 A1 | 14 April 2005 |
| | | ZA 200408386 B | 31 May 2006 |
| | | ZA 200603418 B | 25 July 2007 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KIRAN et al.** *FEBS J,* 2013
- **HUBBI et al.** *J Biol Chem,* 2013
- **CHEN et al.** *MolCell,* 2013
- **SHIN et al.** *Cell Rep,* 2013
- **VAKHRUSHEVA et al.** *Circ Res,* 2008
- **RYU et al.** *Cell Metabolism,* 2014
- **LEE et al.** *Proteomics,* 2014
- **GHIRALDINI et al.** *Mol BiolCell,* 2013
- **VAKHRUSHEVA et al.** *J Physiol Pharmacol,* 2008
- **VAKHRUSHEVA et al.** *Cir Res,* 2008
- **MAO et al.** *Science,* 2011
- **KIRAN et al.** *Exp Cell Res,* 2014
- **BARBER et al.** *Nature,* 2012
- **KIM et al.** *Hepatology,* 2013
- **DE NIGRIS et al.** *Br J Cancer,* 2002
- **FRYE.** *Br J Cancer,* 2002
- **ASHRAF et al.** *Br J Cancer,* 2006
- *Biochem Biophys Res Commun,* 2019, vol. 508, 451-457